(19) Europäisches Patentamt
European Patent Office
Office européen des brevets

(11) **EP 4 451 274 A1**

(12) **EUROPEAN PATENT APPLICATION**

(43) Date of publication:
**23.10.2024 Bulletin 2024/43**

(21) Application number: **23382349.1**

(22) Date of filing: **17.04.2023**

(51) International Patent Classification (IPC):
***G16B 20/00*** (2019.01) ***G16B 10/00*** (2019.01)

(52) Cooperative Patent Classification (CPC):
**G16B 20/00; G16B 10/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **FUJITSU LIMITED**
**Kawasaki-shi, Kanagawa 211-8588 (JP)**

(72) Inventor: **GARCIA-SANTA, Nuria**
**28231 Madrid (ES)**

(74) Representative: **Haseltine Lake Kempner LLP**
**Cheapside House**
**138 Cheapside**
**London EC2V 6BJ (GB)**

Remarks:
Amended claims in accordance with Rule 137(2) EPC.

(54) **RISK AND DIAGNOSIS**

(57) A computer-implemented method comprising: assigning a plurality of family members of a patient each at least one genotype; determining a genetic risk score indicating a likelihood of the patient having the condition using Mendelian and/or Bayesian analysis; determining a family history risk score indicating a likelihood of the patient having the condition based on: how many family members of the patient are known to have or have had the condition and who are not known to have died from the condition and at least one said family member's age when they were diagnosed with the condition, and how many family members of the patient are known to have died from the condition and at least one said family member's age when they died; and determining a genetic family history risk score of the patient having the condition based on the genetic risk score and the family history risk score.

FIGURE 1

**Description**

The present invention relates to risk and diagnosis, and in particular to a computer-implemented method, a computer program, an information processing apparatus, a device, and a system.

One of the most effective measures of a person's risk for a genetic disease is analysis of his or her family medical history. The family history, or pedigree, has long been the backbone of a clinical genetic visit. It contributes to making a diagnosis, determining risk, and assessing the needs for patient education and psychosocial support.

Research on pedigree analysis in unselected patient populations has shown that pedigrees often reveal additional, previously unidentified genetic risk factors. It is likely that family history analysis will become a crucial means of risk assessment in primary care practices (Frezzo, T. M., Rubinstein, W. S., Dunham, D., & Ormond, K. E. (2003), "The genetic family history as a risk assessment tool in internal medicine", Genetics in Medicine, 5(2), 84-91).

Patients' Family History (FH) or family medical history (FMH) is determinant information for assessing the risk factors associated with numerous diseases such as diabetes, coronary heart disease and multiple types of cancers. For example, if a female patient has both her mother and sister having breast cancer, her relative risk of having breast cancer is increased 3.6 times compared with people without such FH (Yang, X., Zhang, H., He, X., Bian, J., & Wu, Y. (2020), "Extracting family history of patients from clinical narratives: exploring an end-to-end solution with deep learning models", JMIR Medical Informatics, 8(12), e22982).

In light of the above, improved methods for risk and diagnosis determination are desirable.

According to an embodiment of a first aspect there is disclosed herein a computer-implemented method comprising: assigning a plurality of family members of a patient each at least one genotype based on rules defining an inheritance mode of a (genetic) disorder/disease/condition and based on information indicating whether or not each of the plurality of family members of the patient is known to have or have had the (genetic) disorder/disease/condition; determining a genetic risk score indicating a likelihood of the patient having the (genetic) disorder/disease/condition using Mendelian and/or Bayesian analysis based on the assigned genotypes of family members of the patient; determining a family history risk score indicating a likelihood of the patient having/being affected by the (genetic) disorder/disease/condition based on: how many family members of the patient are known to have or have had (have been diagnosed with) the (genetic) disorder/disease/condition and who are not known to have died from the (genetic) disorder/disease/condition and (the or each or) at least one said family member's age when they were diagnosed with the condition, and how many family members of the patient are known to have died from the (genetic) disorder/disease/condition and (the or each or) at least one said family member's age when they died; and determining a genetic family history risk score of the patient having the (genetic) disorder/disease/condition based on/using the genetic risk score and the family history risk score.

According to an embodiment of a second aspect there is disclosed herein a computer-implemented method comprising, for each of a plurality of (genetic) disorders/diseases/conditions: assigning a plurality of family members of a patient each at least one genotype based on rules defining an inheritance mode of a (genetic) disorder/disease/condition and based on information indicating whether or not each of the plurality of family members of the patient is known to have or have had the (genetic) disorder/disease/condition; determining a genetic risk score indicating a likelihood of the patient having the (genetic) disorder/disease/condition using Mendelian and/or Bayesian analysis based on the assigned genotypes of family members of the patient; determining a family history risk score indicating a likelihood of the patient having/being affected by the (genetic) disorder/disease/condition based on: how many family members of the patient are known to have or have had (have been diagnosed with) the (genetic) disorder/disease/condition and who are not known to have died from the (genetic) disorder/disease/condition and (the or each or) at least one said family member's age when they were diagnosed with the condition, and how many family members of the patient are known to have died from the (genetic) disorder/disease/condition and (the or each or) at least one said family member's age when they died; and determining a genetic family history risk score of the patient having the (genetic) disorder/disease/condition based on/using the genetic risk score and the family history risk score.

A family member being known to have or have had the condition may mean a family member who has been or was diagnosed with the condition.

The condition may be a (genetic) disease and/or a genetic condition and/or a (genetic) disorder.

Determining the genetic family history risk score of the patient having the condition based on/using the genetic risk score and the family history risk score may comprise summing the genetic risk score (multiplied by a penetrance value) and the family history risk score.

Determining the genetic family history risk score of the patient having the condition based on/using the genetic risk score and the family history risk score may comprise multiplying the genetic risk score (and a penetrance value) and the family history risk score.

The information may further indicate, for at least one or each of the plurality of family members of the patient, a family member role of the family member in relation to the patient.

The information may further indicate, for at least one or each of the plurality of family members of the patient:

an age of the family member (any of: a current age, an age at which they were diagnosed with the condition, and an age at which they died); and a status of the family member (dead or alive).

The information may further indicate, for at least one or each of the plurality of family members of the patient, any of: an identification code of the family member (the method may comprise assigning an identification code to the family member); a gender of the family member; an identification code of the family member's father; and an identification code of the family member's mother.

The computer-implemented method may further comprise transforming information in JSON format to a structured data format.

The transforming may comprise applying a set of transformation rules to the information in the JSON format.

The structured data format may be a matrix/table.

The computer-implemented method may further comprise determining the inheritance mode of the condition by applying rules defining a plurality of inheritance modes to the information indicating whether or not each of the plurality of family members of the patient is known to have or have had the condition.

The plurality of inheritance modes may include any of autosomal dominant, autosomal recessive, X-linked recessive, X-linked dominant, and Y-linked.

Determining the inheritance mode of the condition may comprise applying rules defining a plurality of inheritance modes to the information indicating: whether or not each of the plurality of family members of the patient is known to have or have had the condition; the gender of each of the plurality of family members of the patient; for at least one family member, an identification code of the family member's father; and for at least one family member, an identification code of the family member's mother; and optionally an identification code of each family member.

Assigning each of a plurality of family members of a patient at least one genotype may be based on the information indicating: whether or not each of the plurality of family members of the patient is known to have or have had the condition; the gender of each of the plurality of family members of the patient; for at least one family member, an identification code of the family member's father; and for at least one family member, an identification code of the family member's mother; and optionally an identification code of each family member.

Determining the genetic risk score indicating the likelihood of the patient having the condition using Mendelian and/or Bayesian analysis based on the assigned genotypes of family members of the patient may comprise determining the probability that the patient possesses a genotype causing the condition.

Determining the genetic risk score indicating the likelihood of the patient having the condition using Mendelian analysis based on the assigned genotypes of family members of the patient may comprise determining the probability that the patient possesses a genotype causing the condition based on the assigned genotypes of the patient's mother and father (and using rules defining possible genotypes of a child of two individuals based on genotypes of those individuals).

Determining the probability that the patient possesses a genotype causing the condition based on the assigned genotypes of the patient's mother and father may comprise using a Punnett Squares-based approach.

Determining the genetic risk score indicating the likelihood of the patient having the condition using Bayesian analysis based on the assigned genotypes of family members of the patient may comprise: determining, as prior probabilities, probabilities that the patient possesses each possible genotype based on the assigned genotypes of the patient's mother and father (and using rules defining possible genotypes of a child of two individuals based on genotypes of those individuals); determining, as conditional probabilities, probabilities that the patient's at least one child possesses the genotype assigned to them given that the patient possesses each genotype, and based on the assigned genotype of the at least one child's other parent; and using Bayesian analysis, determining the (Bayesian) probability that the patient possess a genotype causing the condition.

Determining the family history risk score may comprise: determining a first contribution to the family history risk score based on how many family members of the patient are known to have or have had (have been diagnosed with) the condition and who are not known to have died from the condition; determining a second contribution to the family history risk score based on how many family members of the patient are known to have died from the condition; determining a third contribution to the family history risk score based on: the age of (the or each or) at least one family member who is known to have or have had (has been diagnosed with) the condition and who is not known to have died from the condition when they were diagnosed with the condition, and the age of (the or each or) at least one family member who is known to have died from the condition when they died; and summing the first to third contributions with corresponding first to third weighting scores.

The first contribution may be determined such that it increases as the number of family members of the patient who are known to have or have had (have been diagnosed with) the condition and who are not known to have died from the condition increases; and/or the second contribution may be determined such that it increases as the number of family members of the patient who are known to have died from the condition increases; and/or the third contribution may be determined such that it increases as the age or ages of the family member or members who are known to have or have had (have been diagnosed with) the condition and who are not known to have died from the condition decreases and as the age or ages of the family member or members who are known to have died from the condition decreases.

Determining the first contribution may comprise: determining the number of first degree family members of the patient who are known to have or have had (have been diagnosed with) the condition and who are not known to have died from the condition; determining the number of second degree family members of the patient who are known to have or have had (have been diagnosed with) the condition and who are not known to have died from the condition; determining the number of third degree family members of the patient who are known to have or have had (have been diagnosed with) the condition and who are not known to have died from the condition; and summing the determined numbers of family members with corresponding first degree, second degree, and third degree primary weighting factors, wherein first degree family members include any of parents, siblings, and children, wherein second degree family members include any of aunts, uncles, grandparents, grandchildren, nieces, nephews, and half-siblings, and wherein third degree family members include any of first cousins, great-grandparents, great-uncles, great-aunts, great-nieces, great-nephews, great-grandchildren, half-aunts, and half-uncles.

Determining the second contribution may comprise: determining the number of first degree family members of the patient who are known to have died from the condition; determining the number of second degree family members of the patient who are known to have died from the condition; determining the number of third degree family members of the patient who are known to have died from the condition; and summing the determined numbers of family members with corresponding first degree, second degree, and third degree secondary weighting factors, wherein first degree family members include any of parents, siblings, and children, wherein second degree family members include any of aunts, uncles, grandparents, grandchildren, nieces, nephews, and half-siblings, and wherein third degree family members include any of first cousins, great-grandparents, great-uncles, great-aunts, great-nieces, great-nephews, great-grandchildren, half-aunts, and half-uncles.

Determining the third contribution may comprise computing the formula:

$$f3 = wn * cond1 + wm * cond2,$$

wherein:

$$cond1 = w1 \sum_{i=1}^{n1st} \left(1 - \frac{age_i}{100}\right) + w2 \sum_{j=1}^{n2nd} \left(1 - \frac{age_j}{100}\right) + w3 \sum_{k=1}^{n3rd} \left(1 - \frac{age_k}{100}\right)$$

wherein:

$$cond2 = w1 \sum_{i=1}^{m1st} \left(1 - \frac{age_i}{100}\right) + w2 \sum_{j=1}^{m2nd} \left(1 - \frac{age_j}{100}\right) + w3 \sum_{k=1}^{m3rd} \left(1 - \frac{age_k}{100}\right)$$

and wherein n1 st, n2nd, and n3rd are the numbers of first to third degree family members of the patient who are known to have or have had (have been diagnosed with) the condition and who are not known to have died from the condition, respectively; mist, m2nd, and m3rd are the numbers of first to third degree family members of the patient who are known to have died from the condition, respectively; wn and wm are fourth and fifth weighting factors, respectively; w1, w2, and w3 are sixth to eighth weighting factors, respectively; in cond1, age indicates the age of the respective family member who is known to have or have had (have been diagnosed with) the condition and who is not known to have died from the condition when they were diagnosed with the (genetic) disorder/disease/condition; and in cond2, age indicates the age of the respective family member who is known to have died from the condition when they died.

Determining the first contribution may comprise dividing the summed total by the number of family members in the plurality of family members of the patient.

The first to third primary weighting factors may be based on the number of first to third degree family members in the plurality of family members, respectively.

The first to third primary weighting factors may be determined by dividing first to third prior primary weighting factors by the number of first to third degree family members in the plurality of family members, respectively.

Determining the second contribution may comprise dividing the summed total by the number of family members in the plurality of family members of the patient.

The first to third secondary weighting factors may be based on the number of first to third degree family members in the plurality of family members, respectively.

The first to third secondary weighting factors may be determined by dividing first to third prior secondary weighting factors by the number of first to third degree family members in the plurality of family members, respectively.

Determining the third contribution may comprise dividing the quantity f3 by the number of family members in the plurality of family members of the patient or by the number of family members considered in the calculation of the quantities cond1 and cond2.

The fourth and fifth weighting factors may be based on the number of family members of the patient who are known to have or have had (have been diagnosed with) the condition and who are not known to have died from the condition and the number of family members of the patient who are known to have died from the condition, respectively.

The sixth to eighth weighting factors may be determined by dividing sixth to eighth prior secondary weighting factors by the number of first to third degree family members in the plurality of family members, respectively, or by the number of first to third degree family members considered in the calculation of the quantities cond1 and cond2, respectively.

Determining the first contribution may comprise dividing the summed total by the number of family members in the plurality of family members of the patient; and/or determining the second contribution may comprise dividing the summed total by the number of family members in the plurality of family members of the patient; and/or determining the third contribution may comprise dividing the quantity f3 by the number of family members in the plurality of family members of the patient or by the number of family members considered in the calculation of the quantities cond1 and cond2.

Determining the genetic risk score may comprise multiplying the genetic risk score by a penetrance value.

The penetrance value may indicate how likely an individual with a genotype which causes the condition is to have the condition.

The penetrance value may indicate a percentage of individuals in a group who have/suffer from the condition, wherein each individual in the group possesses a genotype which causes the condition.

The group may comprise individuals of a first age range.

A predictive age of the patient calculated by adding a projection age to the patient's current age may fall within the first age range.

The condition may be a first condition and the method may comprise carrying out, for a second condition: the assigning of genotypes, the determining of a genetic risk score, the determining of a family history risk score, and the determining of a genetic family history risk score.

The method may comprise carrying out, for a plurality of conditions: the assigning of genotypes, the determining of a genetic risk score, the determining of a family history risk score, and the determining of a genetic family history risk score.

The computer-implemented method may comprise extracting the information indicating whether or not each of the plurality of family members of the patient is known to have or have had the condition from unstructured medical data.

The computer-implemented method may comprise extracting information relating to at least one of the plurality of family members of the patient from unstructured medical data, the information including: a family member role indicating a relationship of the family member with the patient; and the information indicating whether or not the family member is known to have or have had the condition.

The information may include a status of the family member and/or an age of the family member (any of a current age, an age at which they died, and an age at which they were diagnosed with the condition).

The computer-implemented method may comprise extracting the information using a trained family medical history, FMH, model trained to use Named Entity Recognition, NER, and Relation Extraction, RE, to extract entities and relations between the entities from unstructured data.

The unstructured medical data may comprise the patient's clinical reports and/or a clinical report of at least one of the plurality of family members of the patient.

The computer-implemented method may comprise training a family medical history model to obtain the trained FMH model, the training comprising: receiving unstructured training (medical history) data; dividing the unstructured training (medical history) data into sentences and tokenizing each sentence to extract tokens, (each token comprising (only) a word or a punctuation mark); performing noun detection among the tokens; identifying family member role keywords among the detected nouns (by comparing at least some of the detected nouns with a dictionary of family member role keywords) and storing the family member role keywords as part of an annotation dataset; identifying among the tokens at least one observation associated with a family member role keyword (and including a term related to the condition) and storing the at least one observation in association with the family member role keyword in the annotation dataset; optionally identifying among the tokens at least one status keyword associated with a family member role keyword (by using a dictionary of status keywords) and storing the at least one status keyword in association with the family member role keyword in the annotation dataset; using the (entities and relations in the) annotation dataset to annotate the unstructured training (medical history) data; and using the annotations in the unstructured training (medical history) data as ground truth information (and using at least one trained language model,) training the family medical history model to use NER and RE to extract as entities, from the unstructured training (medical history) data, information (entities and relations) included in the annotations. The information included in the annotations may be considered to correspond to the "extracted information" described above with reference to the definition of the first aspect.

Identifying at least one observation associated with a family member role keyword may include, the family member role keyword being a (first) subject family member role keyword: analyzing tokens occurring in the unstructured training (medical history) data between the appearance of the (first) subject family member role keyword and the next appearance of a family member role keyword and identifying at least one token (among those tokens) that fulfils at least one observation criterion as at least one observation associated with the subject family member role keyword.

The computer-implemented method may further comprise, for another family member role keyword (being a second subject family member role keyword) occurring in the unstructured training (medical history) data before the appearance of the (first) subject family member role keyword and separated from the (first) subject family member role keyword by a conjunctive element (being a comma or the word "and" or a sign indicating the word "and"), identifying the at least one token which was identified as the at least one observation associated with the (first) subject family member role keyword as at least one observation associated with the other (second) subject family member role keyword.

The computer-implemented method may further comprise, before identifying at least one observation associated with a family member role keyword, performing syntax tree dependency analysis to generate syntax tags for tokens, and the at least one observation criterion may comprise a criterion that a token must have (or be inside) a syntax tag of pobj or dobj or conj to be considered an observation.

Identifying at least one observation associated with a family member role keyword may include identifying a modality of the at least one observation. Storing the at least one observation may comprise storing in the annotation dataset the modality in association with the at least one observation.

Identifying at least one status keyword associated with a family member role keyword may comprise, if/when the at least one status keyword is part of a token comprising a family member role keyword, identifying the at least one status keyword as being associated with the family member role keyword.

Identifying at least one status keyword associated with a family member role keyword may comprise implementing a first status search comprising: analyzing tokens occurring in the unstructured training (medical history) data between the appearance of a family member role keyword, being a first family member role keyword, and the next appearance of a family member role keyword to identify at least one status keyword (among those tokens) (not already associated with a family member role keyword), and identifying the at least one status keyword as being associated with the first family member role keyword.

Identifying at least one status keyword associated with a family member role keyword may comprise implementing a second status search comprising: identifying a second (another) family member role keyword occurring in the unstructured training (medical history) data before the appearance of the first family member role keyword and separated from the first family member role keyword by a conjunctive element (being a comma or the word "and"); and identifying the at least one status keyword identified in the first status search as being associated with the second family member role keyword.

The computer-implemented method may further comprise identifying among the tokens a family side keyword (according to a dictionary of family side keywords) associated with a family member role keyword and storing the family side keyword in association with the family member role keyword in the annotation dataset, (wherein the family side keyword is one of a group including maternal and paternal).

Identifying a family side keyword associated with a family member role keyword may comprise, if/when the family side keyword is part of a token comprising a family member role keyword, identifying the family side keyword as being associated with the family member role keyword.

The computer-implemented method may further comprise using part-of-speech, POS, analysis to assign POS tags to tokens/words in the unstructured (medical history) data.

Performing noun detection may comprise detecting tokens/words with a POS tag of noun.

Annotating the unstructured training (medical history) data may comprise annotating the unstructured training (medical history) data using a JavaScript Object Notation, JSON, format.

Training the family medical history model may further comprise, in a step proceeding using the annotations in the unstructured training (medical history) data as ground truth information, transforming the annotated unstructured training (medical history) data to: a Begin-entity, Inside-entity, Other, BIO, format; or a Begin-entity, Inside-entity, Other, End, Single, BIOES, format; or a Begin-entity, Inside-entity, Last, Other, Unique, BILOU, format.

The computer-implemented method may further comprise training a family medical history model to obtain the trained family medical history model, the training comprising: receiving unstructured training (medical history) data; dividing the unstructured training (medical history) data into sentences and tokenizing each sentence to extract tokens, (the tokens comprising family member role keywords and/or status keywords); performing noun detection among the tokens (and part of speech extraction on the sentences); identifying family member role keywords among the detected nouns (by comparing at least some of the detected nouns with a dictionary of family member role keywords); extracting at least one group (of words/tokens/entities) (a plurality of groups each) comprising a family member role keyword and at least one of a status keyword associated with the family member role keyword and an observation associated with the family member role keyword, wherein for any token comprising a family member role keyword and a status keyword (according to a dictionary of status keywords), the group including the family member role keyword includes the status keyword; generating syntax tree tags for tokens (to analyze relationships between detected nouns/entities), and based on the syntax tree tags, identifying at least one observation associated with a family member role keyword (and including a term related to the condition), wherein the group including the family member role keyword includes the at least one observation; using the extracted groups to annotate the unstructured training (medical history) data; and using the annotations in the unstructured training (medical history) data as ground truth information (and using at least one trained language model,) training the family medical history model to use NER and RE to extract as entities, from the unstructured training (medical history) data, the information included in the annotations.

Identifying at least one observation associated with a family member role keyword may include identifying a modality of the at least one observation. The group including the family member role keyword and the at least one observation may include the modality.

The computer-implemented method may further comprise, for any token comprising a family member role keyword and not a status keyword, (or for any token comprising a family member role keyword) the family member role keyword being a first family member role keyword, implementing a first status search comprising: analyzing tokens occurring in the unstructured training (medical history) data between the appearance of the first family member role keyword and the next appearance of a family member role keyword to identify a status keyword (among those tokens), wherein the group including the first family member role keyword includes the identified status keyword.

The computer-implemented method may further comprise, for any token comprising a family member role keyword and not a status keyword which has not been extracted as a group comprising a status keyword after the first status search, implementing a second status search comprising: identifying a second (another) family member role keyword occurring in the unstructured training (medical history) data before the appearance of the first family member role keyword and separated from the first family member role keyword by a conjunctive element (being a comma or the word "and"), wherein the group comprising the second (other) family member role keyword includes the status keyword which is also included in the group comprising the first family member role keyword of the first status search.

For any token comprising a family member role keyword and a family side keyword (according to a dictionary of family side keywords), the group including the family member role keyword may include the family side keyword, (wherein the family side keyword is one of a group including maternal and paternal).

The family member role for a said family member may include information indicating a family side of the family member (the family side being maternal or paternal).

Each observation may include information indicating an observation modality (the observation modality being positive or negative).

The computer-implemented method may further comprise extracting information indicating a gender of at least one family member, optionally based on the family member role the at least one family member (and based on rules dictating correspondences between gender and family member roles).

The computer-implemented method may further comprise extracting the information from (the) unstructured (medical history) data for at least one family member for a plurality of family members of the target patient, and the computer-implemented method may further comprise extracting information of at least one relationship (relationships) between the family members based on the family member role of each family member (and based on rules dictating relationships between family member roles).

The computer-implemented method may further comprise predicting a (genetic family history) diagnosis for the patient based on the genetic family history risk score(s) and outputting the diagnosis.

Predicting a (genetic family history) diagnosis may comprise selecting the condition with the highest genetic family history risk score (if that genetic family history risk score is above a genetic family history risk score threshold).

The computer-implemented method may further comprise outputting at least one (genetic family history) diagnosis for the patient based on the genetic family history risk score(s).

Predicting at least one (genetic family history) diagnosis may comprise selecting at least one/any condition with a genetic family history score above a genetic family history risk score threshold.

The computer-implemented method may further comprise outputting a list of high-risk conditions for the patient based on the determined genetic family history risk scores.

The computer-implemented method may further comprise receiving at least one physiological measurement of the patient and outputting a diagnosis for the patient based on the at least one physiological measurement and the genetic family history risk score(s).

The computer-implemented method may further comprise receiving at least one symptom of the patient and outputting a diagnosis for the patient based on the at least one symptom and the genetic family history risk score(s).

The computer-implemented method may further comprise receiving at least one physiological measurement of the patient and at least one symptom of the patient and outputting a diagnosis for the patient based on the at least one physiological measurement, the at least one symptom, and the genetic family history risk score(s).

The computer-implemented method may further comprise: predicting at least one genetic family history diagnosis, the at least one genetic family history diagnosis being at least one diagnosis of the patient based on the genetic family history risk score(s); predicting at least one physiological diagnosis, the at least one physiological diagnosis being at least one diagnosis of the patient based on (the) at least one symptom of the patient and/or (the) at least one physiological measurement of the patient; and comparing the at least one genetic family history diagnosis with the at least one physiological diagnosis and, when at least one diagnosis among the at least one genetic family history diagnosis is the same as a diagnosis among the at least one physiological diagnosis, outputting the at least one diagnosis as at least one final diagnosis.

The computer-implemented method may further comprise: predicting at least one genetic family history diagnosis, the at least one genetic family history diagnosis being at least one diagnosis of the patient based on the genetic family history risk score(s); predicting at least one symptom diagnosis, the at least one symptom diagnosis being at least one diagnosis of the patient based on (the) at least one symptom of the patient; predicting at least one measurement diagnosis, the at least one measurement diagnosis being at least one diagnosis of the patient based on (the) at least one physiological measurement of the patient, and comparing the at least one genetic family history diagnosis, the at least one symptom diagnosis, and the at least one measurement diagnosis and, when at least one diagnosis among the at least one genetic family history diagnosis is the same as a diagnosis among the at least one symptom diagnosis and at least one diagnosis among the at least one measurement diagnosis, outputting the at least one diagnosis as at least one final diagnosis.

The at least one physiological measurement may comprise at least one of: a heart rate; a temperature; at least one electrocardiogram, ECG, measurement; at least one electromyogram, EMG, measurement; at least one galvanic skin response, GSR, sensor measurement; a measurement of sweat gland activity; at least one optical sensor measurement; a pH measurement; a blood pressure measurement; a pacemaker pulse detection measurement; a measurement of skin anomalies by light intensity; and a blood-glucose level measurement.

The computer-implemented method may further comprise receiving the at least one symptom of the patient through speech input from the user.

The computer-implemented method may further comprise converting the speech input into text data and using Named Entity Recognition, NER, to extract the at least one symptom.

The computer-implemented method may further comprise outputting recommendations to the user based on the at least one (final) diagnosis.

The recommendations may comprise recommended medication or recommended action.

Predicting the at least one symptom diagnosis may comprise: maintaining a symptom diagnosis score for each of a plurality of possible diagnoses; adjusting at least one of the symptom diagnosis scores based on a set of symptom diagnosis rules and based on the at least one symptom of the patient; and determining at least one of the possible diagnoses having the highest symptom diagnosis score as the at least one symptom diagnosis.

Predicting the at least one physiological diagnosis may comprise: maintaining a physiological diagnosis score for each of a plurality of possible diagnoses; adjusting at least one of the physiological diagnosis scores based on a set of physiological diagnosis rules and based on the at least one physiological measurement of the patient; and determining at least one of the possible diagnoses having the highest physiological diagnosis score as the at least one physiological diagnosis.

Determining the at least one genetic family history diagnosis may comprise disregarding a diagnosis if its genetic family history risk score is below a genetic family history risk score threshold; and/or determining the at least one symptom diagnosis may comprise disregarding a diagnosis if its symptom diagnosis score is below a symptom diagnosis score threshold; and/or determining the at least one physiological diagnosis may comprise disregarding a diagnosis if its physiological diagnosis score is below a physiological diagnosis score threshold.

The set of symptom diagnosis rules may comprise adding to a said symptom diagnosis score for a diagnosis of heart failure if the at least one symptom comprises: tiredness; and/or chest pain; and/or bad breath; and/or tachycardia; and/or nausea; and/or an accelerated heartrate; and/or a persistent cough.

The set of symptom diagnosis rules may comprise: if the at least one symptom comprises tiredness, adding to at least one symptom diagnosis score for any of heart failure and congestive heart failure; and/or if the at least one symptom comprises chest pain, adding to at least one symptom diagnosis score for any of heart failure, congestive heart failure, muscle strain, gastroesophageal reflux disease, asthma, costochondritis, and valvular heart disease; and/or if the at least one symptom comprises bad breath, adding to at least one symptom diagnosis score for any of heart failure, congestive heart failure, pneumonia, coronavirus, asthma, emphysema, and valvular heart disease; and/or if the at least one symptom comprises tachycardia, adding to at least one symptom diagnosis score for any of heart failure, congestive heart failure, coronary artery disease, heart attack, congenital heart defects, valvular heart disease, hypertension, and cardiomyopathies; and/or if the at least one symptom comprises nausea, adding to at least one symptom diagnosis score for any of heart failure and congestive heart failure; and/or if the at least one symptom comprises an accelerated heartrate, adding to at least one symptom diagnosis score for any of heart failure, congestive heart failure, coronary artery disease, heart attack, congenital heart defects, valvular heart disease, hypertension, and cardiomyopathies; and/or if the at least one symptom comprises a persistent cough, adding to at least one symptom diagnosis score for any of heart failure, congestive heart failure, chronic obstructive pulmonary disease, gastroesophageal reflux disease, asthma, and coronavirus.

The set of symptom diagnosis rules may comprise: if the at least one symptom comprises tiredness, adding to at least one symptom diagnosis score for any of heart failure and congestive heart failure; and/or if the at least one symptom comprises chest pain, adding to at least one symptom diagnosis score for any of heart failure, congestive heart failure, and valvular heart disease; and/or if the at least one symptom comprises bad breath, adding to at least one symptom diagnosis score for any of heart failure, congestive heart failure, and valvular heart disease; and/or if the at least one symptom comprises tachycardia, adding to at least one symptom diagnosis score for any of heart failure, congestive heart failure, coronary artery disease, heart attack, congenital heart defects, valvular heart disease, hypertension, and cardiomyopathies; and/or if the at least one symptom comprises nausea, adding to at least one symptom diagnosis score for any of heart failure and congestive heart failure; and/or if the at least one symptom comprises an accelerated heartrate, adding to at least one symptom diagnosis score for any of heart failure, congestive heart failure, coronary artery disease, heart attack, congenital heart defects, valvular heart disease, hypertension, and cardiomyopathies; and/or if the at least one symptom comprises a persistent cough, adding to at least one symptom diagnosis score for any of heart failure and congestive heart failure.

The set of physiological diagnosis rules may comprise adding to a said physiological diagnosis score for a diagnosis of heart failure if the at least one symptom comprises: P-wave=='enlarged'; and/or QRS_duration=='prolonged'; and/or left_axis_deviation==True; and/or time_to_ID > 50; and/or CSA_muscle=='reduction'; and/or muscle_strength=='reduction'; and/or muscle_fatigability=='high'; and/or submaximal_contraction=='low'; and/or breathing_rate=='low'; and/or heart rate=='high'; and/or GSR < 15; and/or blood_pressure_fluid=='high'.

The set of physiological diagnosis rules may comprise: if the at least one physiological measurement comprises an enlarged P-wave (detected from an ECG), adding to at least one physiological diagnosis score for any of atrial enlargement, chronic respiratory disease, heart failure, congestive heart failure, cardiomyopathies, congenital heart defects, and valvular heart disease; and/or if the at least one physiological measurement comprises a prolonged QRS duration (detected from an ECG), adding to at least one physiological diagnosis score for any of heart failure, congestive heart failure, atrial fibrillation, coronary disease, arrhythmia, ischemic heart disease, and valvular heart disease; and/or if the at least one physiological measurement comprises a left axis deviation (detected from an ECG), adding to at least one physiological diagnosis score for any of ischemic heart disease, heart failure, congestive heart failure, and congenital heart defects; if the at least one physiological measurement comprises a time to ID of more than 50 seconds (detected from an ECG), adding to at least one physiological diagnosis score for any of heart failure and coronary heart disease; and/or if the at least one physiological measurement comprises a cross-sectional area muscle reduction (detected from an EMG), adding to at least one physiological diagnosis score for any of heart failure, congestive heart failure, and skeletal muscle atrophy; and/or if the at least one physiological measurement comprises a muscle strength reduction (detected from an EMG), adding to at least one physiological diagnosis score for any of heart failure, congestive heart failure, skeletal muscle atrophy, and muscular dystrophy; and/or if the at least one physiological measurement comprises a high muscle fatigability (detected from an EMG), adding to at least one physiological diagnosis score for any of heart failure, congestive heart failure, skeletal muscle atrophy, and muscular dystrophy; and/or if the at least one physiological measurement comprises a low submaximal contraction (detected from an EMG), adding to at least one physiological diagnosis score for any of heart failure, congestive heart failure, skeletal muscle atrophy, muscular dystrophy, and neurological disorders; if the at least one physiological measurement comprises a low breathing rate (detected from a pacemaker pulse detection measurement), adding to at least one physiological diagnosis score for any of heart failure, congestive heart failure, bradypnea, lung disorders, chronic bronchitis, and pneumonia; and/or if the at least one physiological measurement comprises a high heart rate (detected from a pacemaker pulse detection measurement), adding to at least one physiological diagnosis score for any of heart failure, congestive heart failure, valvular heart disease, coronary heart disease, and cardiomyopathies; and/or if the at least one physiological measurement comprises an enlarged heart rate (detected from a pacemaker pulse detection measurement), adding to at least one physiological diagnosis score for any of heart failure, congestive heart failure, stroke, and cardiomyopathies; and/or if the at least one physiological measurement comprises a skin conductivity of less than 15 kohms (detected from a GSR sensor measurement), adding to at least one physiological diagnosis score for any of depression and anxiety; and/or if the at least one physiological measurement comprises a high blood pressure, adding to at least one physiological diagnosis score for any of heart failure, congestive heart failure, heart attack, stroke, arterial disease, and aortic aneurysm.

The set of physiological diagnosis rules may comprise: if the at least one physiological measurement comprises an enlarged P-wave (detected from an ECG), adding to at least one physiological diagnosis score for any of atrial enlargement, heart failure, congestive heart failure, cardiomyopathies, congenital heart defects, and valvular heart disease; and/or if the at least one physiological measurement comprises a prolonged QRS duration (detected from an ECG), adding to at least one physiological diagnosis score for any of heart failure, congestive heart failure, atrial fibrillation, coronary disease, arrhythmia, ischemic heart disease, and valvular heart disease; and/or if the at least one physiological measurement comprises a left axis deviation (detected from an ECG), adding to at least one physiological diagnosis score for any of ischemic heart disease, heart failure, congestive heart failure, and congenital heart defects; if the at least one physiological measurement comprises a time to ID of more than 50 seconds (detected from an ECG), adding to at least one physiological diagnosis score for any of heart failure and coronary heart disease; and/or if the at least one physiological measurement comprises a cross-sectional area muscle reduction (detected from an EMG), adding to at least one physiological diagnosis score for any of heart failure and congestive heart failure; and/or if the at least one physiological measurement comprises a muscle strength reduction (detected from an EMG), adding to at least one physiological diagnosis score for any of heart failure and congestive heart failure; and/or if the at least one physiological measurement comprises a high muscle fatigability (detected from an EMG), adding to at least one physiological diagnosis score for any of heart failure and congestive heart failure; and/or if the at least one physiological measurement comprises a low submaximal contraction (detected from an EMG), adding to at least one physiological diagnosis score for any of heart failure and congestive heart failure; if the at least one physiological measurement comprises a low breathing rate (detected from a pacemaker pulse detection measurement), adding to at least one physiological diagnosis score for any of heart failure and congestive heart failure; and/or if the at least one physiological measurement comprises a high heart rate (detected from a pacemaker pulse detection measurement), adding to at least one physiological diagnosis score for any of heart failure, congestive heart failure, valvular heart disease, coronary heart disease, and cardiomyopathies; and/or if the at least one physiological measurement comprises an enlarged heart rate (detected from a pacemaker pulse detection measurement), adding to at least one physiological diagnosis score for any of heart failure, congestive heart failure, stroke, and cardiomyopathies; and/or if the at least one physiological measurement comprises a high blood pressure, adding to at least one physiological diagnosis score for any of heart failure, congestive heart failure, heart attack, stroke, arterial disease, and aortic aneurysm.

The set of symptom diagnosis rules may comprise adding to a said symptom diagnosis score for a diagnosis of depression if the at least one symptom comprises: tiredness; and/or apathy; and/or lack of enthusiasm; and/or sadness; and/or reduced appetite.

The set of symptom diagnosis rules may comprise: if the at least one symptom comprises tiredness, adding to said symptom diagnosis scores for any of depression and anxiety; and/or if the at least one symptom comprises apathy, adding to said symptom diagnosis scores for any of depression and major depressive disorder; and/or if the at least one symptom comprises lack of enthusiasm, adding to said symptom diagnosis scores for any of depression, major depressive disorder, and suicidal ideation; and/or if the at least one symptom comprises sadness, adding to said symptom diagnosis scores for any of depression, major depressive disorder, and suicidal ideation; and/or if the at least one symptom comprises stress and/or anxiety, adding to said symptom diagnosis scores for anxiety; and/or if the at least one symptom comprises reduced appetite, adding to said symptom diagnosis scores for any of depression, major depressive disorder, anorexia, nutrition disorder.

The set of physiological diagnosis rules may comprise adding to a said physiological diagnosis score for a diagnosis of depression (and anxiety) if the at least one symptom comprises: an irregular heartrate; and/or high GSR fluctuation; and/or high blood pressure; and/or low pH levels; and/or high blood-glucose level fluctuation.

The computer-implemented method may further comprise displaying information indicating the determined genetic family history risk score and the condition on a device.

The computer-implemented method may further comprise displaying information indicating the determined genetic family history diagnosis and optionally the associated genetic family history risk score on a device.

The computer-implemented method may further comprise displaying information indicating the at least one final diagnosis and optionally the associated genetic family history risk score on a device.

The computer-implemented method may further comprise displaying, using a device, information indicating output information, the output information comprising any of: at least one determined genetic family history risk score and the associated condition; and a determined diagnosis and optionally the associated genetic family history risk score.

The device may be a magnetic device and/or an internet of things, IOT, device.

The device may comprise at least one light element and the information may comprise a colour of a said light element to indicate a range in which the determined genetic family history risk score falls.

The computer-implemented method may further comprise repeating the assigning of genotypes, the determining of a genetic risk score, the determining of a family history risk score, and the determining of a genetic family history risk score, (and the determination of a genetic family history or final diagnosis) when new information relating to a family member of the patient is received.

According to an embodiment of a third aspect there is disclosed herein a computer program which, when run on a computer, causes the computer to carry out a method comprising: assigning a plurality of family members of a patient each at least one genotype based on rules defining an inheritance mode of a (genetic) disorder/disease/condition and based on information indicating whether or not each of the plurality of family members of the patient is known to have or have had the (genetic) disorder/disease/condition; determining a genetic risk score indicating a likelihood of the patient having the (genetic) disorder/disease/condition using Mendelian and/or Bayesian analysis based on the assigned genotypes of family members of the patient; determining a family history risk score indicating a likelihood of the patient having/being affected by the (genetic) disorder/disease/condition based on: how many family members of the patient are known to have or have had (have been diagnosed with) the (genetic) disorder/disease/condition and who are not known to have died from the (genetic) disorder/disease/condition and (the or each or) at least one said family member's age when they were diagnosed with the condition, and how many family members of the patient are known to have died from the (genetic) disorder/disease/condition and (the or each or) at least one said family member's age when they died; and determining a genetic family history risk score of the patient having the (genetic) disorder/disease/condition based on/using the genetic risk score and the family history risk score.

According to an embodiment of a fourth aspect there is disclosed herein an information processing apparatus comprising a memory and a processor connected to the memory, wherein the processor is configured to: assign a plurality of family members of a patient each at least one genotype based on rules defining an inheritance mode of a (genetic) disorder/disease/condition and based on information indicating whether or not each of the plurality of family members of the patient is known to have or have had the (genetic) disorder/disease/condition; determine a genetic risk score indicating a likelihood of the patient having the (genetic) disorder/disease/condition using Mendelian and/or Bayesian analysis based on the assigned genotypes of family members of the patient; determine a family history risk score indicating a likelihood of the patient having/being affected by the (genetic) disorder/disease/condition based on: how many family members of the patient are known to have or have had (have been diagnosed with) the (genetic) disorder/disease/condition and who are not known to have died from the (genetic) disorder/disease/condition and (the or each or) at least one said family member's age when they were diagnosed with the condition, and how many family members of the patient are known to have died from the (genetic) disorder/disease/condition and (the or each or) at least one said family member's age when they died; and determine a genetic family history risk score of the patient having the (genetic) disorder/disease/condition based on/using the genetic risk score and the family history risk score.

According to an embodiment of a fifth aspect there is disclosed herein a system comprising a device and a server, wherein the server is configured to transmit output information to the device and the device is configured to display information indicating the output information, the output information comprising any of: a determined genetic family history risk score and a condition; a determined genetic family history diagnosis and optionally an associated genetic family history risk score; and at least one final diagnosis and optionally at least one associated genetic family history risk score.

The server may be or comprise the information processing apparatus of the aforementioned fourth aspect.

The device may be a magnetic device and/or an internet of things, IOT, device.

The device may comprise at least one light element and may be configured to display a colour using a said light element to indicate a range in which the determined genetic family history risk score falls.

The device may comprise a screen and may be configured to display on the screen the information indicating the output information.

According to an embodiment of a sixth aspect there is disclosed herein a device configured to receive output information and to display information indicating the output information, the output information comprising any of: a determined genetic family history risk score and a condition; a determined genetic family history diagnosis and optionally an associated genetic family history risk score; and at least one final diagnosis and optionally at least one associated genetic family history risk score.

The device may be a magnetic device and/or an internet of things, IOT, device. The device may comprise at least one light element and may be configured to display a colour using a said light element to indicate a range in which the determined genetic family history risk score falls. The device may comprise a screen and may be configured to display on the screen the information indicating the output information.

The device may comprise the information processing apparatus of the aforementioned fourth aspect.

According to an embodiment of a seventh aspect there is disclosed herein a computer program which, when run on a computer, (or comprising instructions which, when executed by the computer) causes the computer to carry out the method of the first or second aspect.

According to an embodiment of an eighth aspect there is disclosed herein an information processing apparatus comprising a memory and a processor connected to the memory, wherein the processor is configured to carry out the method of the first or second aspect.

Features relating to any aspect/embodiment may be applied to any other aspect/embodiment. The second aspect may be considered an alternative to the first aspect so that features thereafter may be considered to apply to both the first and second aspects.

Reference will now be made, by way of example, to the accompanying drawings, in which:

Figure 1 is a diagram illustrating a system;

Figure 2 is a diagram illustrating a method;
Figure 3 is a diagram illustrating a method;
Figure 4 is a diagram illustrating a module;
Figure 5 is a diagram illustrating a table;
Figure 6 illustrates example code;
Figure 7 is a diagram illustrating a table;
Figure 8 is a diagram illustrating a device;
Figure 9 is a diagram useful for understanding the invention;
Figure 10 is a diagram illustrating a table;
Figure 11 is a diagram illustrating a table;
Figure 12 is a diagram illustrating an interface;
Figure 13 is a diagram illustrating a table;
Figure 14 is a diagram illustrating a table;
Figure 15 is a diagram illustrating a device;
Figure 16 is a diagram useful for understanding the invention;
Figure 17 is a diagram illustrating a table;
Figure 18 is a diagram illustrating a table;
Figure 19 is a diagram illustrating a device; and
Figure 20 is a diagram illustrating an apparatus.

Exploiting patients' FH in a proper way may allow the development of accurate health risk prediction solutions for monitorization and alert systems of individuals, for example providing mechanisms for Preventive Medicine.

An aspect of the present disclosure is an ad-hoc device to provide a real-time alert system to patients about potential health risks based on family antecedents' information integrated in the Electronic Health Records (EHRs) of the patient. Such a device may follow the paradigm of the Internet of Things (IoT), being connected to the network and to the EHR system of the patient in order to provide updated assessments automatically and directly to the patients for Preventive Medicine support.

Features disclosed herein include the following:

- Magnet device to provide alert information of patient's health risks based on family antecedents in an easy and understandable way, showing such information in the simplest way possible to reach all the patients.
- Module to integrate automated calculation of health risks (and diagnosis) based on family antecedents, statistical models and proprietary measures with the IoT magnet device.
- Interactive interface in the magnet device so that the patient could update information or contact directly to physician.
- New measure, 'Danger Factor', to be included in the calculation of health risks prediction.

The following non-exhaustive and exemplary list describes some technical terms:

- *Family Health History*: is a record of health information about a person and their close relatives. A complete record includes information from three generations of relatives, including children, brothers and sisters, parents, aunts and uncles, nieces and nephews, grandparents, and cousins.

- *Preventive Medicine*: is a medical specialty which focuses on the health of individuals and communities. The goal of preventive medicine is to promote health and well-being and prevent disease, disability and death.

- *Text Mining*: the process of deriving high-quality information from text. The process or practice of examining large collections of written resources in order to generate new information. The goal of text mining is to discover relevant information in text by transforming the text into data that can be used for further analysis.

- *Machine Learning (ML):* the subfield of computer science that "gives computers the ability to learn without being explicitly programmed". It explores the study and construction of algorithms that can learn from and make predictions on data.

- *Deep Learning (DL)*: is a part of a broader family of machine learning methods based on learning data representations, as opposed to task-specific algorithms.

- *Artificial Neural Network/Neural Network (ANN/NN):* is an information processing paradigm that is inspired by the way biological nervous system, such as the brain, process information. They can be used to extract patterns and detect trends that are too complex to be noticed by either humans or other computer techniques.

- *Named Entity Recognition (NER)*: is a subtask of information extraction that seeks to locate and classify named entities mentioned in unstructured text into categories such as family members, conditions, etc.

- *Relation Extraction*: is the task of extracting semantic relationships from a text. Extracted relationships usually occur between two or more entities of a certain type (e.g. Person, Organisation, Location) and fall into a number of semantic categories (e.g. married to, employed by, lives in).

- *Internet of Things (IoT)*: describes physical objects (or groups of such objects) with sensors, processing ability, software and other technologies that connect and exchange data with other devices and systems over the Internet or other communications networks. The field has evolved due to the convergence of multiple technologies, including ubiquitous computing, commodity sensors, increasingly powerful embedded systems, as well as machine learning.

- *Family Pedigree:* A pedigree, as related to genetics, is a chart that may be used to track the inheritance of a trait or health condition through generations of a family. The pedigree particularly shows the relationships among family members and, when the information is available, indicates which individuals have a trait(s) of interest. It can also help determine how a trait or condition might be passed down through the generations and what might accompany it.

- *Mendelian Pattern Disorders*: Mendelian inheritance (also known as Mendelism) is a type of biological inheritance following the principles originally proposed by Gregor Mendel in 1865 and 1866. Mendelian disorders, for example, occur when specific mutations in single genes - called germline mutations - are inherited from either of one's two parents. Well-known examples of Mendelian diseases include cystic fibrosis, sickle cell disease, and Duchenne muscular dystrophy.

- *Genotypes*: refers to the entire set of genes in the cells of an organism. In a narrower sense, however, it can refer to different alleles, or variant forms of a gene, for particular traits, or characteristics (our case of interest).

- *Phenotypes:* refers to the observable characteristics in an individual resulting from the expression of genes; the clinical presentation of an individual with a particular genotype.

- *Bayesian Models:* is a statistical model where probability is used to represent all uncertainty within the model, both the uncertainty regarding the output but also the uncertainty regarding the input (a.k.a. parameters) to the model. The probability expresses a degree of belief in an event. The degree of belief may be based on prior knowledge about the event, such as the results of previous experiments, or on personal beliefs about the event.

- *Punnett Squares*: is a square diagram that is used to predict the genotypes of a particular cross or breeding experiment. It is named after Reginald C. Punnett, who devised the approach in 1905. The diagram is used by biologists to determine the probability of an offspring having a particular genotype. The Punnett square is a tabular summary of possible combinations of maternal alleles with paternal alleles.

- *Penetrance*: is the percentage of individuals in a group with a given genotype who exhibit the phenotype associated with that genotype.

Existing methodologies suffer issues including the following:

- No devices to provide health risks alert systems without sensors and the need of carrying on them all the time
- No devices exploiting only family antecedents to estimate risks
- No technology to automatically obtain family antecedents without need of questionnaires to estimate health risks in IoT device
- No technology combining several statistical models considering age ranges of patients and relatives (with e.g. danger factor) to estimate health risks/diagnosis.

The present disclosure includes the following aspects:

- A magnet device may be positioned in an easy visible place inside the home (e.g., in the fridge) to highlight alert information of patient's health risks/diagnoses based only on family antecedents or based on other measurements as well, showing such information in the simplest way possible to reach patients. No need to carry the device and if only using family antecedents then no sensors are needed nor biological/physical measurements to calculate the risk. The device may stay at home and may only need family antecedents' information to calculate risk.

▪ The magnet device may be interactive. Besides showing patient's health risk alerts for a list of diseases, the device may handle inputs from the patient and process such inputs to connect and make changes/updates in the EHR of the patient, or, to provide direct call with the primary care physician assigned to the patient.

▪ Automatic prediction of the patient's health risks including a measure called 'Danger Factor'. Such measure may be obtained by mathematical formulae of different factors extracted from the family antecedents and may be integrated in the final mathematical model for the calculation of the risks'/diagnoses' probabilities.

▪ A module to integrate health risks/diagnosis results based on family antecedents, statistical models and proprietary measures with an IoT device and patient's EHR in parallel, and e.g. to translate/standardise the scores of risks' probabilities to an Alert Colour System easy understandable by all patients.

▪ This module may include reasons for the results for the doctor in the updates of the patient's EHR, not only the probability scores, which may improve explainability. For example, the workflow of the process to obtain the resultant health risk scores/diagnoses may be described so that a user, e.g. a doctor, understands the causes of such results in a transparent way.

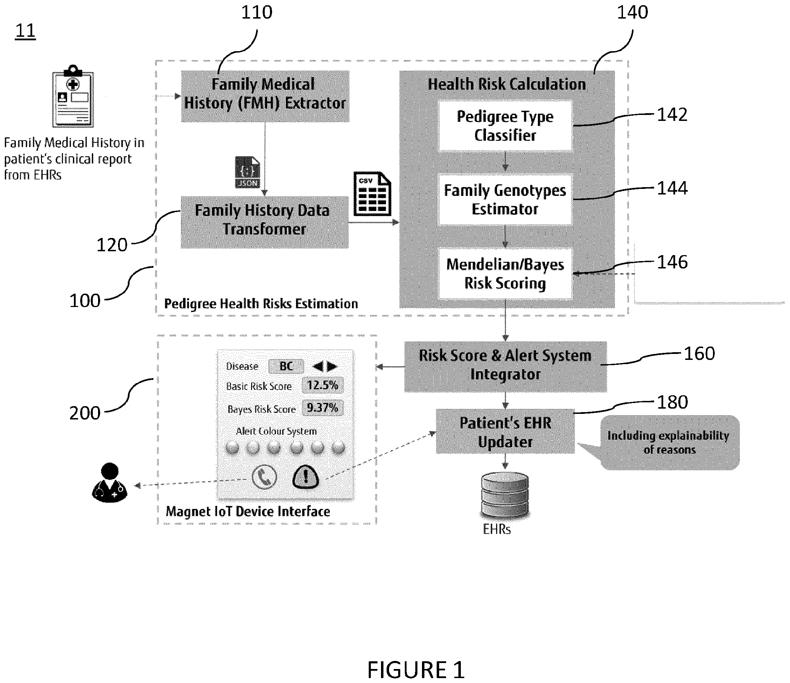

Figure 1 illustrates a system 11 comprising a pedigree health risks estimation module 100 (may be referred to as estimation module 100), a magnetic IoT device interface 200 (may be referred to as a device 200), a risk score and alert system integrator module 160 (may be referred to as an integrator module 160), and a patient's EHR updater 180 (may be referred to as an updater 180).

Pedigree Health Risks Estimation

The estimation module 100 is in charge of the determination of the patient's health risks/diagnoses resulting in probability scores of such risks and/or at least one diagnosis and/or at least one accompanying score.

• Inputs: Text paragraphs of patient's clinical reports with the Family Medical History included (unstructured data)

• Family Medical History (FMH) Extractor 110: This module is in charge of the extraction of relevant named entities from the family information (unstructured data) such as a family member's role, status, side, age and conditions. This information will be provided in JSON format and it will be processed by the next module.

• Family History Data Transformer 120: This module is in charge of analysing the information in JSON format provided by previous module and transforming it into appropriate structured data to be used in the health risks calculation. This data will be included in a CSV file that will be sent to Health Risk Calculation module 140.

• Health Risk Calculation module 140 (may be referred to as calculation module 140): This module is in charge of carrying out the algorithms to predict potential future health risks of patients based on the information of the CSV file provided by the previous component. This module includes three subcomponents. The first two subcomponents (Pedigree Type Classifier 142 (may be referred to as inheritance mode classifier 142) and Family Genotypes Estimator 144) use rule-based approaches with rules known from the literature. The third module (Mendelian/Bayes Risk Scoring module 146 - may be referred to as score module 146) integrates a new scoring factor in the overall mathematical models (this module may make use of two models: one using Mendelian and basic Punnett Squares approach, the other more complex using Mendelian and Bayes algorithm).

• Output of the estimation module 100: The resultant health risk scores for at least one of the two models, for example percentages from 0% to 100%, for a particular condition.

The calculation module 140 may also carry out steps to determine a diagnosis based on the health risk scores as described below. The calculation module 140 may also carry out steps described below of determining other diagnoses based on physiological measurements and/or symptoms, and optionally may determine a final diagnosis based on determined diagnoses.

Risk Score & Alert System Integrator module 160 and updater module 180

The integrator module 160 transmits the determined health risk information (e.g. diagnoses, conditions, scores, etc.) to the device 200, and also to the updater module 180. The updater module 180 is in charge of, integrating and updating health risk information in the EHR of the patient, for example including reasons of why such results were obtained to provide the transparent workflow to the doctor. The integrator module 160 may adapt the information output from the estimation module to be visualized in the device 200, for example based on an alert colour system that translates risk scores to a system of colours. New information from relatives' medical history may be detected by the updater module 180 and will be processed again and the risk scores may be updated if necessary, and the updated information may be forwarded to the patient's EHR and the device 200.

Magnet IoT Device Interface 200

The device 200 may include interactive buttons that a user (i.e. the patient) can use to update or connect with the doctor. A direct call may be established between patient and doctor or warning signals may be sent to the patient's EHR, producing changes/updates in the central system managed by doctor/hospital.

- Inputs: Information received from integrator module 160. This may include, for example, a list of diseases/conditions relevant for the patient depending on the family history; calculated risk scores for the diseases/conditions, and/or the alert colour associated to such risks. The information may include at least one (final) diagnosis and an associated score as described below.

The steps carried out by the elements shown in Figure 1 are described in more detail below.

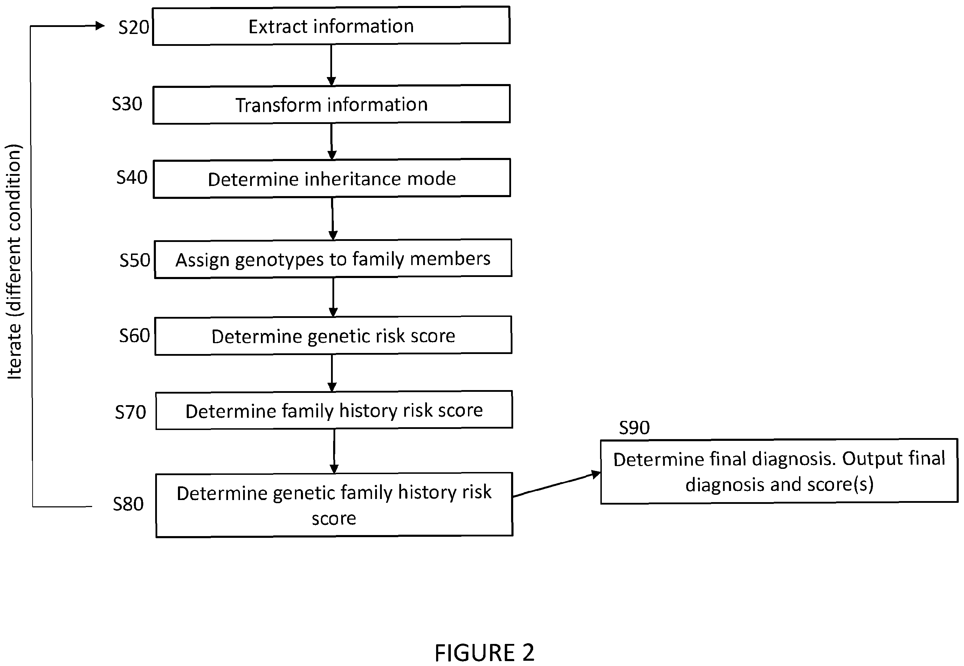

Figure 2 is a diagram illustrating a method. The method may be performed by the system 11, or by the estimation module 100, or by an information processing apparatus 10 described below. The method is for determining a genetic family history risk score for at least one condition. Steps S20-S80 relate to a particular condition and may be repeated for at least one other condition so that genetic family history risk scores may be determined for a plurality of conditions. A condition may be a (genetic) disease and/or a genetic condition and/or a (genetic) disorder. Any of these terms may be used interchangeably herein.

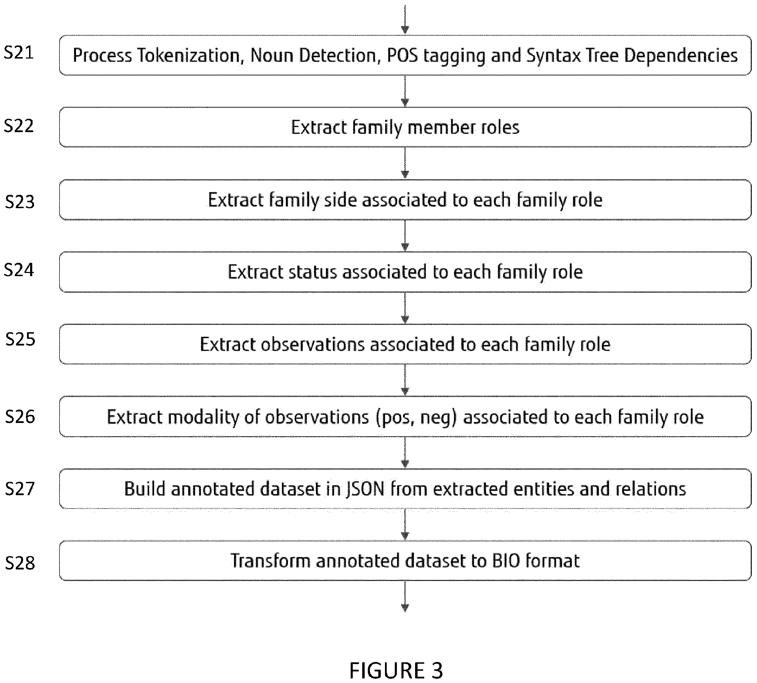

Step S20 comprises extracting information about the patient's family members' medical history from unstructured data and is described below with reference to Figure 3. Step S30 comprises transforming the extracted information. The information may be transformed from a JSON format to a table/matrix format.

Step S40 comprises determining the inheritance mode of a condition. For example, step S40 comprises determining the inheritance mode of the condition by applying rules defining a plurality of inheritance modes to information indicating whether or not each of the plurality of family members of the patient is known to have or have had the condition (e.g. has or has not been diagnosed with the condition).

Step S50 comprises assigning genotypes to the family members of the patient. That is, step S50 comprises assigning each of a plurality of family members of the patient at least one genotype based on rules defining an inheritance mode of the condition and based on information indicating whether or not each of the plurality of family members of the patient is known to have or have had the condition.

Step S60 comprises determining a genetic risk score. The genetic risk score indicates a likelihood of the patient having a condition using Mendelian and/or Bayesian analysis based on the assigned genotypes of family members of the patient.

Step S70 comprises determining a family history risk score. The family history risk score indicates a likelihood of the patient having/being affected by the condition based on how many family members of the patient are known to have or have had the condition. Step S80 comprises determining a genetic family history risk score. Step S90 comprises determining a final diagnosis and outputting the final diagnosis and associated score(s).

The method may comprise just steps S20-S80 and may or may not comprise repeating those steps for different conditions. The method may not comprise step S20 and the information may be received instead of extracted from unstructured data. The method may not comprise steps S20 and S30 and the information may be received in a suitable (e.g. table/matrix) format. Step S40 may comprise determining the inheritance mode based on the known inheritance mode of the condition under consideration - that is, some conditions have only one inheritance mode so that there is no need to apply rules to the information to determine the inheritance mode. The method may not comprise this step if the inheritance mode is known. Steps of the method are described further below.

Family Medical History (FMH) Extractor 110 (Step S20)

This module may be considered to carry out step S20. This module is in charge of processing the input, e.g., the patient's clinical Family Medical report for the extraction of relevant named entities from the family information such as the relatives' role, status, side, age and conditions. Such extraction may be done with known methodologies. The extraction could be done through third-party tools, rule-based approaches, or state-of-the-art techniques for Named Entity Recognition (NER) deep learning-based models.

Step S20 may comprise using an FMH model to extract the information from the unstructured data. The FMH model may be trained as described in the following example and step S20 may comprise training the FMH model in this way.

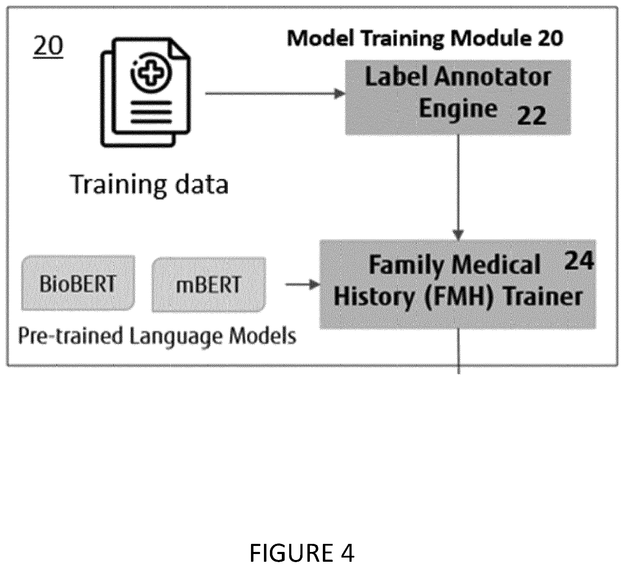

In this example, the FMH model is trained by a model training module 20 illustrated in Figure 4 and comprising a label annotator engine 22 and an FMH trainer 24. The model training module 20 is in charge of the learning process in the creation of a Family Medical History (FMH) model. The FMH model 20 is able to extract relevant entities and their relationships from text fragments of clinical documents.

The model training module 20 receives the following Inputs: unstructured (medical history) data, i.e. text fragments of clinical documents (including family medical information); and pre-trained language models, such as multilingual BERT (Bidirectional Encoder Representations from Transformers), abbreviated as mBERT,, described at https://github.com/google-research/bert/blob/master/multilingual.md, or BioBERT (an implementation of BERT that has been trained on different combinations of biomedical domain corpora; in other words, BioBERT is a domain-specific language representation model pre-trained on large-scale biomedical corpora), described at https://arxiv.org/abs/1901.08746 and https://github.com/naver/biobert-pretrained, for fine-tuning the tasks of NER and Relation Extraction to be performed by the FMH model during a deep learning process (described in relation to the FMH trainer below).

The label annotator engine 22 implements a rule-based algorithm to extract family medical information and create annotated datasets with this information to be used in the learning process (described in relation to the FMH trainer below). The label annotator engine 22 may be a module.

The FMH trainer 24 is in charge of implementing a deep learning process through state-of-the-art techniques and third-party tools, using the annotated datasets created with the label annotator engine 22 to train the FMH model to generate a trained FMH model.

The output of the FMH trainer 24, and of the model training module 20 in general, is the trained FMH model which is capable of extracting family medical information from new unstructured clinical documents, i.e. the FMH trainer 24 trains the FMH model to use Named Entity Recognition (NER) and Relation Extraction (RE) to extract entities and relations between the entities from unstructured (medical history) data.

Label Annotator Engine 22

Figure 3 illustrates a method (according to the running example of training the FMH model) executed by the label annotator engine 22. The label annotator engine 22 receives as an input text fragments of training clinical documents with Family Medical information included (unstructured data). As mentioned above, the label annotator engine 22 implements a rule-based algorithm to extract family medical information from unstructured texts and create annotated datasets to be used in training the FMH model. The rule-based algorithm is illustrated in the method of Figure 3.

Step S21 comprises receiving unstructured (medical history) data; dividing the unstructured (medical history) data into sentences and tokenizing each sentence to extract tokens, the tokens comprising family member role keywords and/or status keywords; and performing noun detection among the tokens (and part of speech (PoS) extraction on the sentences). Step S21 may also comprise generating syntax tree dependencies for each sentence to analyse relationships between detected nouns/entities.

That is, step S21 comprises Process Tokenization, Noun Detection, POS tagging and Syntax Tree Dependencies. As the unstructured (medical history) data, Family History information in discharge summaries is collected from public repositories. The information is filtered by section names of the document(s) to get the appropriate data. Cleaning and text processing tasks may be carried out on the collected information/data. The training data may relate to a target patient. The target patient may be the same as or different from a target patient to which unstructured data (not for training, described later) relates. The training data may relate to a plurality of different families and therefore different target patients.

For the tokenisation, third-party libraries may be used to divide the Family History texts into sentences and tokenize each sentence. Tokenization is a way of separating a piece of text into smaller units called tokens. Here, tokens can be either words, characters, or subwords. Noun detection and Part of Speech (POS) extraction may be carried out over the sentences to get relevant data.

Syntax tree dependencies of sentences are generated to analyse relationships between entities. For the next steps of the method, information processed/extracted in step S21 will aid in the extraction of entities and relations about Family Medical History. For instance, for the sentence, "Mother and grandmother died of diabetes.", the information extracted by tokenization is, following the notation format of

token --> syntax tree tag --> POS tag:

Mother--> nsubj --> NOUN
and --> cc --> CCONJ

```
grandmother--> conj --> NOUN
died --> ROOT --> VERB
of --> prep --> ADP
diabetes --> pobj --> NOUN
. --> punct --> PUNCT
```

The meanings of the POS tags are as follows: NOUN indicates a noun, VERB indicates a verb, ADP indicates an adposition, PUNCT indicates punctuation, and CCONJ indicates a coordinating conjunction. Further POS tags that may be used may be found at https://universaldependencies.org/u/pos/all.html.

The meanings of the syntax tree tags (grammatical tags of the syntax tree dependencies) are as follows: nsubj=subject (noun), ROOT=main verb, prep=preposition, punct=punctuation, cc=coordination, pobj=object of preposition. Further syntax tree tags that may be used may be found at https://downloads.cs.stanford.edu/nlp/software/dependencies_manual.pdf.

Step S21 may be considered to comprise tokenisation optionally followed by noun detection, optionally followed by POS analysis and/or syntax tree tag analysis. Noun detection may be considered to comprise using the POS tags and/or syntax tree tags. Nouns may be detected by filtering tokens to find tokens comprising an element with the POS tag NOUN.

Step S22 comprises identifying family member role keywords among the detected nouns by comparing at least some of the detected nouns with a dictionary of family member role keywords.

That is, step S22 comprises extracting family member roles. An initial dictionary may be defined with 31 basic types (keywords) for family roles. Family roles include entities such as Father, Mother, etc. From such initial set, synsets (i.e. a set of one or more synonyms that share a common meaning) may be searched e.g. in the WordNet (http://compling.hss.ntu.edu.sg/omw/) resource (with multilingual capabilities), to construct an extended dictionary of family roles (keywords) for the desired language (e.g. English). Using this particular method, 25 extended entities are obtained, making a total number of 56 family role types (keywords) in the dictionary.

In order to return a list of family roles (keywords) from the Family History texts the nouns detected in the sentences in step S22 may be filtered using several parameters of those nouns, and then the (filtered) nouns are compared with the dictionary to match potential family roles. The resultant entities matched are outputted to the next step.

Step S23 comprises extracting a family side associated with each family role. The keywords 'maternal' or 'paternal' are searched for. Such keywords are extracted in the tokenisation together with the associated family role keyword as a (unique) token. Therefore, it is checked whether those keywords (maternal or paternal) are present in each (noun) token including a family role (keyword). If so, the family side keyword will be associated with the specific family role keyword in a list. The complete processed list is outputted to the next step.

Step S24 comprises, for any token comprising a family member role keyword and not a status keyword, the family member role keyword being a first family member role keyword, implementing a first status search comprising: analyzing tokens occurring in the unstructured training (medical history) data between the appearance of the first family member role keyword and the next appearance of a family member role keyword to identify a status keyword among those tokens, wherein the group including the first family member role keyword includes the identified status keyword.

Step S24 further comprises, for any token comprising a family member role keyword and not a status keyword which has not been extracted as a group comprising a status keyword after the first status search, implementing a second status search comprising: identifying a second family member role keyword occurring in the unstructured training (medical history) data before the appearance of the first family member role keyword and separated from the first family member role keyword by a conjunctive element (e.g. being a comma or the word "and"), wherein the group comprising the second family member role keyword includes the status keyword which is also included in the group comprising the first family member role keyword of the first status search.

That is, step S24 comprises extracting a status associated with each family role (keyword). A dictionary may be defined with root terms of a status such as died, alive, healthy, live, etc. Such terms may be lemmatized in order to cover all potential verbal forms or variations appearing in the texts. If the status keyword is extracted together with the family role keyword as a (unique) token, the approach in the family side extraction is followed (that is, the status keyword in the token is associated with the family member role keyword). In some implementations, only words with the POS tag of VERB or ADJ (adjective) may be compared with the dictionary of status keywords to identify status keywords.

However, there are cases where this situation does not happen. For such cases, an iterative algorithm capable of analysing text information between appearances of family roles (keywords) in the text may be implemented. This is iterated over all the tokens of the text, and detects two continuous appearances of family roles (keywords). The tokens between such appearances are compared with the dictionary and optionally lemmatizations, looking for status keywords matches. If there is match, the status (keyword) found is associated with the first family role keyword appearing before the status keyword. This part of the iterative algorithm may be referred to as a first status search.

After the first extraction of statuses for the family roles of the list over the whole text (the first status search), another part of the iterative algorithm may be implemented to identify missing statuses due to conjunctive phrases. This may be referred to as a second status search and may be considered a second iteration of the status search. For instance, for the sentence *'His mother, father and brother are alive'*, in the first extraction (first status search) the status (keyword) 'alive' is associated only to brother, but in the second iteration steps may be implemented to associate the status (keyword) 'alive with the family members (role keywords) "mother" and "father" as well. The final list of family roles with their associated status is outputted to the next step.

Step S25 comprises identifying at least one observation associated with a family member role keyword by, the family member role keyword being a (first) subject family member role keyword: analyzing tokens occurring in the unstructured training (medical history) data between the appearance of the (first) subject family member role keyword and the next appearance of a family member role keyword and identifying at least one token among those tokens that fulfils observation criteria as at least one observation associated with the subject family member role keyword. For example, considering the syntax tree dependencies (and POS tags), to identify observations the method needs to find nouns (POS=NOUN) and filter by the syntax tag of 'pobj' (preposition of object) or 'dobj' (direct object) or 'conj' (conjunct). A token may be considered an observation only if the tag of syntax tree dependency is inside one of those values.

Step S25 may further comprise, for another family member role keyword being a second subject family member role keyword occurring in the unstructured training (medical history) data before the appearance of the first subject family member role keyword and separated from the first subject family member role keyword by a conjunctive element (being a comma or the word "and"), identifying the at least one token which was identified as the at least one observation associated with the first subject family member role keyword as at least one observation associated with the second subject family member role keyword.

That is, step S25 comprises extracting observations associated with each family role. The syntax tree parser is used to identify observations based on their dependency with the nouns detected in the sentence. A search for observations is iterated over all the detected nouns of the text. The approach followed is similar to the first status search and the second status search of step S24. The tokens (or nouns) included between two appearances of family roles (keywords) in the text are analysed. If such tokens/nouns fulfil a set of filters and dependency types (observation criteria), they are marked as observations and associated with the first family role (keyword) appearance (i.e. the family role keyword appearing before the observation). For cases of conjunctive phrases, the same methodology used in the status extraction (second status search) is used in the observation extraction. The updated list of family roles (each family role with the related observations included) is outputted to the next step.

Only observations related to the condition under consideration may be extracted, such as observations comprising a keyword relating to the condition - this may be included as an observation criterion. Or, any observation may be extracted. For example, step S20 (and optionally step S30) in Figure 2 may be a step which is not tied to a particular condition, and then the subsequent steps may be iterated for individual conditions. An observation which is extracted may include that the family member died of the condition under consideration or another condition.

Steps S22 to S25 (and optionally step S26) may be considered to comprise extracting at least one group comprising (or a plurality of groups each comprising) a family member role keyword and at least one of a status keyword associated with the family member role keyword and an observation associated with the family member role keyword, wherein for any token comprising a family member role keyword and a status keyword (according to a dictionary of status keywords), the group including the family member role keyword includes the status keyword. That is, each of at least one family role keyword may be extracted together with a status keyword and/or at least one observation. They may be extracted as a token.

Step S26 comprises extracting the modality of an observation. Step S26 may be considered as an extension of step S25, so that identifying at least one observation associated with a family member role keyword includes identifying a modality of the at least one observation, and wherein the group including the family member role keyword and the at least one observation includes the modality.

That is, step S26 comprises extracting the modality of observations (pos, neg). If a family member (role keyword) in the text is described positively with the condition/disease/symptom of the observation, this may be indicated with a positive tag (pos), and if a family member (role keyword) in the text is described negatively, i.e. without, the condition/disease/symptom of the observation this may be indicated with a negative tag (neg). External models may be used to determine if an observation is affirmative (pos tag) or negative (neg tag). The list of observations may be updated with these tags associated with the observations and outputted to the next step.

Steps S21 to S26 may be considered to comprise extracting information from the unstructured training (medical history) data for at least one family member of the target patient, the information comprising: a family member role (keyword) indicating a relationship of the family member with the target patient; a status of the family member (including at least one of a group comprising alive, dead, and healthy); and at least one observation of the family member.

Steps S21 to S26 may be considered to comprise dividing the unstructured training (medical history) data into sentences and tokenizing each sentence to extract tokens; performing noun detection among the tokens; identifying family member role keywords among the detected nouns (by comparing at least some of the detected nouns with a dictionary of family member role keywords) and storing the family member role keywords as part of an annotation dataset; identifying among the tokens at least one status keyword associated with a family member role keyword (by using a dictionary of status keywords) and storing the at least one status keyword in association with the family member role keyword in the annotation dataset; identifying among the tokens at least one observation associated with a family member role keyword and storing the at least one observation in association with the family member role keyword in the annotation dataset; using the (entities and relations in the) annotation dataset to annotate the unstructured training (medical history) data; and using the annotations in the unstructured training (medical history) data as ground truth information (and using at least one trained language model,) training the family medical history model to use NER and RE to extract as entities, from the unstructured training (medical history) data, the information (entities and relations) included in the annotations.

That is, in some implementations, rather than extracting entities as a group (entities being any of family member role keywords, status keywords, observations, family side keywords, etc.) methods may be considered to comprise extracting entities and storing them in the annotation dataset in association with each other.

Step S27 comprises using the extracted groups (annotation dataset) to annotate the unstructured training (medical history) data. That is, step S27 comprises building an annotated dataset in JSON (JavaScript Object Notation) from the extracted entities and relations, i.e. the groups of family member role keywords and statuses/observations etc. Step S27 may comprise, after processing input Family History texts with previous steps, using external libraries for writing the information extracted in JSON format. For instance, in the text example, *"Her father died of leukemia at age 53 and her aunt had leukemia at age 19. Sister died of leukemia, Brother died from HIV/AIDS",* the final output in JSON after previous steps and step S27 is:

"{""info"": [{""family_role"": {""name"": ""father""}, ""family_status"": {""name"": ""died""}, ""family_observation"": [{""name"": ""leukemia"", ""mod"": ""pos""}]}, {""family_role"": {""name"": ""aunt""}, ""family_observation"" [{""name"": ""leukemia"", ""mod"": ""pos""}]}, {""family_role"": {""name"": ""Sister""}, ""family_status"": {""name"": ""died""}, ""family_observation"": [{""name"": ""leukemia"", ""mod"": ""pos""}]}, {""family_role"": {""name"": ""Brother""}, ""family_status"": {""name"": ""died""}, ""family_observation"": [{""name"": ""HIV/AIDS"", ""mod"": ""pos""}]}]}"

The extracted information may also include information related to age, for example any of the current age of the individual, the age at which the individual was diagnosed with the condition under consideration (or a condition), and the age at which the individual died, using techniques the same as or similar to those described herein. For example, once a condition is identified and associated with an individual (family member), a search is performed to identify an age. In this search, for example, numbers present in the text fragment relating to the individual are identified. If a number is identified, the 5-gram tokens before and after the number are analysed - i.e. the words before and after the number, in a window of 5 words, are analysed. If the tokens analysed follow certain reserved patterns (defined in advance, e.g. based on previous training), the number is associated with the individual as their age. Further rules and/or patterns may indicate that the age is the age at which the individual was diagnosed with the condition (for example if the condition appears in a window of words before or after the age). Furthermore, for example, if the text indicates that individual is dead, it may be assumed that the age is related to (i.e. is) the age of death.

To create an annotated dataset, entities (any of the information extracted above, including family member role keywords, status keywords, observations, family side keywords, observation modalities) in the unstructured training data (family history texts) may be tagged with internal tags in the JSON format (e.g. as above). An annotation tag scheme able to represent the Named Entities and their Relationships at once may be used, so that the FMH model extracts everything (all the required entities and relations) at the same time (in one go).

In the annotation tag scheme, representation of the entities (NER) may be as follows: Family member=FH_ROLE, Family side=FH_SIDE, Family status=FH_STATUS, Family age=FH_AGE, Family observation=FH_OBS (also including a modality for each observation: FH_OBS_POS, FH_OBS_NEG). A modality may be extracted for status (FH_STATUS_POS, FH_STATUS_NEG) since for example the terms "not alive" or "not healthy" may be used. Modalities may be extracted for any of the extracted terms (family role, family side). In some implementations only a negative modality may be used and the absence of a negative modality therefore indicates a positive modality.

In the annotation tag scheme, representation of the relations (RE) may be as follows: A numeric indicator (relation) may be included at the end of a generic tag. For instance, the first appearance of a family member role keyword in a sentence (FH_ROLE_1) has a '_1' suffix. A '_1' suffix is then included in the related entities to illustrate the relation (e.g. FH_STATUS_1, FH_OBS_1, etc.). Similar suffixes may then be used for second ('_2'), third ('_3') and so on appearances of family member roles in the same sentence. This tagging scheme may also be used for nested relations, i.e. entity relations shared by various family members (e.g. FH_OBS_1_2, FH_SIDE_2_3, etc.). With this kind of representation, in a particular example of training data there are 132 tag labels, but this number may depend on the training data used. For the text example, *"Mother and grandmother died of diabetes.",* the resultant annotated sentence following the annotation tag scheme is:

<FH_ROLE_1>Mother</FH_ROLE_1> and

<FH_ROLE_2>grandmother</FH_ROLE_2>
<FH_STATUS_1><FH_STATUS_2>died</FH_STATUS_2></FH_STATUS_1> of
<FH_OBS_1><FH_OBS_2>diabetes</FH_OBS_2></FH_OBS_1>.

The annotated dataset following the annotation tag scheme is outputted to the next step.

Step S28 comprises transforming the annotated dataset to the BIO (Begin entity, Inside entity, Other) format. That is, for the training of the FMH model (in particular the fine-tuning of the tasks of NER and Relation Extraction in the creation of the FMH model using the pre-trained language models of mBERT and BioBERT), a specific tag scheme format is required. In the BIO format, every word of an entity is tagged. The previously annotated dataset (in JSON) is transformed to the BIO format through text processing techniques, joining and cleaning terms, and/or combining tags for nested annotations. The result of this transformation on the previous example is:

| | |
|---|---|
| Mother | B-FH_ROLE_1 |
| and | O |
| grandmother | B-FH_ROLE_2 |
| died | B-FH_STATUS_1_2 |
| of | O |
| diabetes. | B-FH_OBS_1_2 |

The annotated dataset transformed to this BIO format will be the final output of the Label Annotator Engine in the running example. The BIO / IOB format (I being short for inside, O being short for outside (other), and B being short for beginning) is a tagging format for tagging tokens in a chunking task in computational linguistics. The B- prefix before a tag indicates that the tag is the beginning of a chunk, and an I- prefix before a tag indicates that the tag is inside a chunk. An O tag indicates that a token belongs to no entity/chunk. There are other formats that could be used, e.g. BIOES (BIO is the same + E- prefix before a tag indicates that the tag is the end of a chunk, and an S-prefix before a tag indicates that the chunk is single (single element=one word). BIOES is also called BILOU (L for last, same as the E, and, U for unique, same as the S).

In some implementations of this running example, some method steps or parts of method steps may be omitted and/or combined and/or executed in a different order. For example steps S22-S26 may be considered as a single step comprising some or all of those steps, i.e. of extracting a group (of words/tokens/entities) comprising a family member role keyword and at least one of a status keyword associated with the family member role keyword and an observation associated with the family member role keyword, wherein for any token comprising a family member role keyword and a status keyword (according to a dictionary of status keywords), the group including the family member role keyword includes the status keyword.

Family Medical History (FMH) Trainer 24

Continuing the description of this running example (of training the FMH model) the FMH trainer 24 trains the FMH model using the annotated unstructured training data output from the label annotator engine 22. That is, the FMH trainer 24 is in charge of implementing a deep learning process, for example through state-of-the-art techniques and third-party tools, using the annotated dataset created with the Label Annotator Engine 22. For example, the external libraries of Tensorflow (https://github.com/tensorflow/tensorflow) and Keras (https://keras.io/) for Python may be used to train the FMH model. For example, techniques described in https://keras.io/examples/nlp/ner_transformers/#build-the-ner-model-class-as-a-kerasmodel-subclass may be used.

The inputs to the FMH trainer 24 are the annotated dataset from the label annotator engine 22 and e.g. the pre-trained language models mBERT and BioBERT. The FMH trainer 24 implements techniques for fine-tuning NER and Relation Extraction in Family Medial History data (unstructured (medical history) data) using pre-trained language models. The operations of the FMH trainer 24 include methods of pre-processing and preparation of the input annotated training dataset, conversion of texts to numerical representations, design of the neural network architecture and execution of the deep learning activity. The output of the FMH trainer 24 is the trained Family Medical History (FMH) model which is capable of extracting family medical information from new unstructured clinical documents.

The above running example is not essential.

Returning to the description of Figures 1 and 2, the extractor module 110 is capable of supporting multi-lingual capabilities for the extraction of information from clinical reports in different languages. The resultant information is outputted in JSON format, containing for example for each family member of the patient the following data:

1. Family member role: The role of the family member in relation with the current patient (mother, father, sister, uncle, grandmother, etc.).

2. Family member status: Current status of the family member (healthy, alive, dead, etc.).

3. Family member side: Side of the family member (maternal or paternal).

4. Family member age: Age of the family member (current age or age at death or age at disease diagnosis, or death by such disease).

5. Family member observations: A list containing details of all observations (diseases, conditions, etc.) of the family member and optionally whether or not they died from the condition.

6. Family member observation modality: Tagging the observation as positive (occurrence confirmation of the condition), or, negative (absence of condition).

This is an example of the output provided by the FMH Extractor:
{'info': [{'family_role': {'name': 'Father', 'mod': 'pos'}, 'family_status': {'name': 'died', 'mod': 'pos'}, 'family_observation': [{'name': 'a brain tumor', 'mod': 'pos'}]}, {'family_role': {'name': 'Mother', 'mod': 'pos'}, 'family_status': {'name': 'died', 'mod': 'pos'}, 'family_observation': [{'name': 'diabetes complications', 'mod': 'pos'}]}], 'text': 'Father died of a brain tumor. Mother died of diabetes complications.'}

Family History Data Transformer 120 (Step S30)

This module may be considered to carry out step S30. This module is in charge of analysing the information in JSON format provided by the previous module and transforming such information in appropriate structured data to be used in the subsequent calculations.

Inputs: JSON file with family information from previous module; the gender of patient (0=female, 1=male, "=undefined); the age of patient; and an observation relating to the condition under consideration (e.g., breast cancer).

In step S30 rule-based methods are used to iterate over the JSON data and get the relevant features from relatives needed for the risk score calculations. Such features may include any of ['ID', 'Gender', 'Age', 'Status, 'FatherID', 'MotherID', 'Affected']:

- ID: Identifier of the member (alphanumeric value)
- Gender: The gender of the member (0=female, 1=male, "=undefined)
- Age: The age of the member
- Status: The status of the member (0=dead, 1=alive)
- FatherID: Identifier of the member's father (alphanumeric value corresponding with ID of other member in the list of relatives. In case that member's father is not in the list, the value is 0 by default)
- MotherID: Identifier of the member's mother (alphanumeric value corresponding with ID of other member in the list of relatives. In case that member's mother is not in the list, the value is 0 by default)
- Affected: This feature indicates if the member is affected by the condition for which we want to calculate the health risks of the patient (1=True, i.e., member is affected; 0=False, i.e., member is not affected)

For including an ID to patient and relatives, values from 1 to n [n=number of members] may be assigned, following the order of appearance in the JSON file. The patient ID may be 1, being the first row of the matrix. For the Gender, the role name of family members may be compared with dictionary categories. For instance, role names such as mother or sister would be in groups belonging to female category. The Age value is extracted directly from JSON data, e.g. from a feature 'family_age' of the family member. The Status value is extracted directly from JSON data, e.g. from a feature 'family_status' of the family member. Depending on the status label, the value will be represented with 0=dead, or 1=alive. The FatherID and MotherID will be extracted through iteration over the members and based on the role names and the 'family_side' (maternal, paternal) to establish the correct IDs. The Affected value will be extracted taking the feature 'family_observation' in positive modality and comparing by string similarity such observation with the one introduced in the input. If the result of comparison outputs a score bigger than 0.9 threshold, it may be assumed that relative is affected (=1). Otherwise, it may be assumed that relative is not affected (=0).

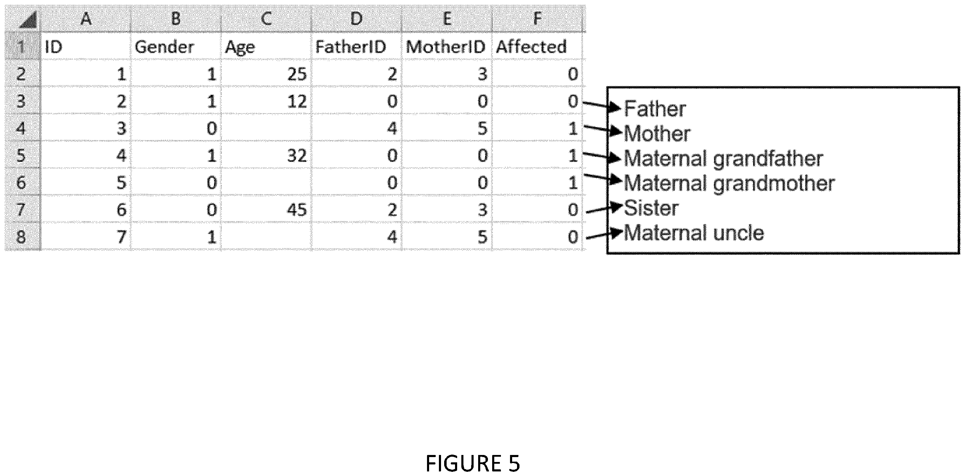

A matrix is created where the columns may be ['ID', 'Gender', 'Age', 'Status, 'FatherID', 'MotherID', 'Affected'], and the rows may be the values for each relative and the patient (in first row). This data will be included in a CSV file that will be sent to Health Risk Calculation module. Figure 5 illustrates an example of such a CSV file (without Status column). The first row is the patient's data [male patient of 25 years old].

Health Risk Calculation module 140

This module may be considered to carry out steps S40-S80. It is in charge of the main algorithms and mathematical models to predict potential future health risks and/or diagnoses of a patient based on the information of the CSV file provided in step S30.

Pedigree Type Classifier 142 (step S40)

This module may be considered to carry out step S40. This module applies known rules from literature to automatically classify the type of inheritance pattern exhibited by the condition under consideration as shown in the extracted information. From literature, the different inheritance patterns that conditions (e.g. mendelian disorders) follow are widely known, and the intrinsic characteristics of each pattern are also known (Chial, H. (2008), "Mendelian genetics: patterns of inheritance and single-gene disorders", Nature Education, 1(1), 63). The plurality of possible inheritance modes (or inheritance pattern or pedigree type) include:

1. Autosomal dominant
2. Autosomal recessive
3. X-linked recessive
4. X-linked dominant
5. Y-linked

The rules defining each inheritance mode are shown below. The rules with * next to them may be the rules that are applied to the extracted information to determine the inheritance mode in step S40.

1 - Autosomal dominant pedigree/inheritance mode

- Affected children usually have an affected parent.
- Heterozygotes (Aa) are affected.
- Two affected parents can produce an unaffected child.*
- Two unaffected parents will not have affected children.*
- Both males and females are affected with equal frequency.
- Key: AA = affected; Aa = affected; aa= normal.

2 - Autosomal recessive pedigree/inheritance mode

- Most affected children have normal parents.
- Heterozygotes (Aa) have a normal phenotype.
- Two affected parents will ALWAYS have affected children.*
- Affected individuals with homozygous normal mates will have normal children.
- Close relatives who reproduce are more likely to have affected children.
- Both male and females are affected with equal frequency.
- Key: aa = affected; Aa = carrier (appears normal); AA = normal; A? = appears normal (one allele unknown).

3 - X-linked recessive pedigree/inheritance mode

- Trait is rare in pedigree.
- Trait skips generations.*
- Affected fathers DO NOT pass to their sons.*
- Males are more often affected than females .
- Usually, it is transmitted from mother to son.*
- Never from father to son.*
- It could be from father to daughter, but more unusual.

4 - X-linked dominant pedigree/inheritance mode

- Trait is common in pedigree.
- Affected fathers pass to ALL of their daughters.*
- Males and females are equally likely to be affected.

5 - Y-linked pedigree/inheritance mode

- Only males affected.*

• Transmitted from father to son only.*

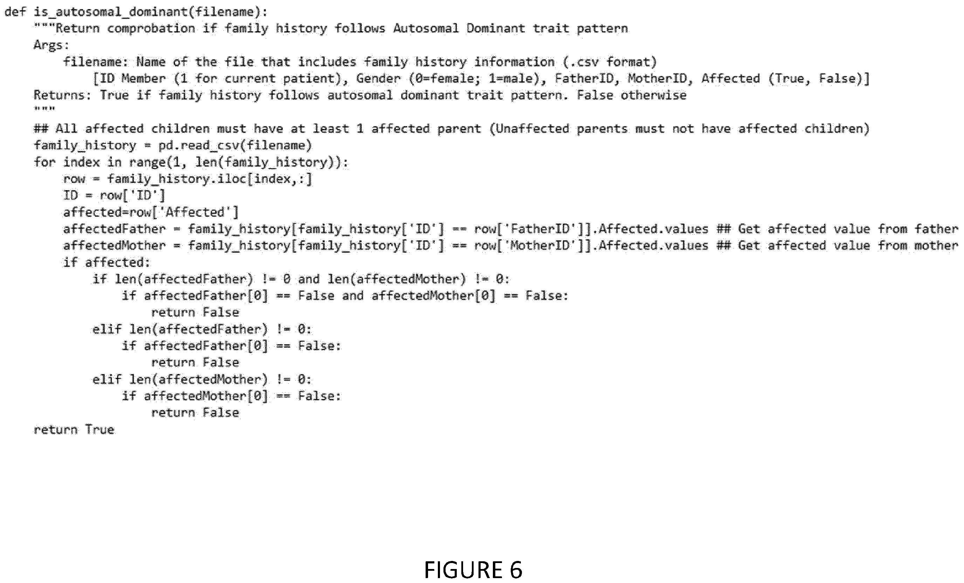

Rules are applied to the information about family members to determine the most likely inheritance mode(s) (i.e. this step comprises checking if the pattern shown in the information fits each inheritance mode). From the CSV file the data of the ID, Gender, FatherID, MotherID and Affected for each family member is used to determine the inheritance mode. It may be that the pattern shown in the information fits in more than one inheritance mode. In this case both/all the inheritance modes that fit the information may be output. An excerpt of example code for determining if some information about family members fits the autosomal dominant inheritance mode is shown in Figure 6.

As mentioned above, some conditions only have one inheritance mode so that for some conditions the inheritance mode does not need to be determined as described above and the system 11 may not comprise the inheritance mode classifier 142.

Family Genotypes Estimator 144 (Step S50)

This module may be considered to carry out step S50. This module applies known rules from literature (which may be programmed into the module) to automatically estimate the genotypes of each family member based on the determined inheritance mode [AUTOSOMAL_DOMINANT, AUTOSOMAL_RECESSIVE, X_LINKED_DOMINANT, X_LINKED_RECESSIVE, Y_LINKED]. Valid genotype possibilities are assigned to each family member based on the associated observation (affected by the condition or not), using the pedigree inheritance type.

Whether the family member is affected by the condition or not may be considered to mean whether the family member is known to have (or have had) the condition or not, or may be considered to mean whether the family member has been diagnosed with the condition or not. There is a possibility that a family member is not known to have or have had the condition even though they do/did have it. Therefore the information concerning whether the family member is affected or not is an approximation based on the family medical history information.

Inputs to the family genotypes estimator (step S50) include: The pedigree inheritance type (autosomal dominant/recessive, x-linked dominant/recessive, y-linked); and from the CSV file, for each family member, the gender (useful in x-linked and y-linked cases), the affected value, the list of affected values of parents and the list of affected values of children. Knowing the pedigree inheritance type, the Gender and Affected value of each member, the scheme below is followed to assign the genotypes.

• Autosomal dominant: AA = affected; Aa = affected; aa= normal
• Autosomal recessive: aa = affected; Aa = carrier (appears normal); AA = normal; A? = appears normal (one allele unknown)
• X-linked dominant: XX= affected; Xx= affected; Xy= affected; xx= normal; xy=normal
• X-linked recessive: xx=affected; Xx= carrier (appears normal); XX=normal; xy=affected; Xy=normal
• Y-linked: xY= affected; xy=normal

It should be noted that the assigned genotype is considered to represent a genotype which codes for the presence or absence of the condition under consideration.

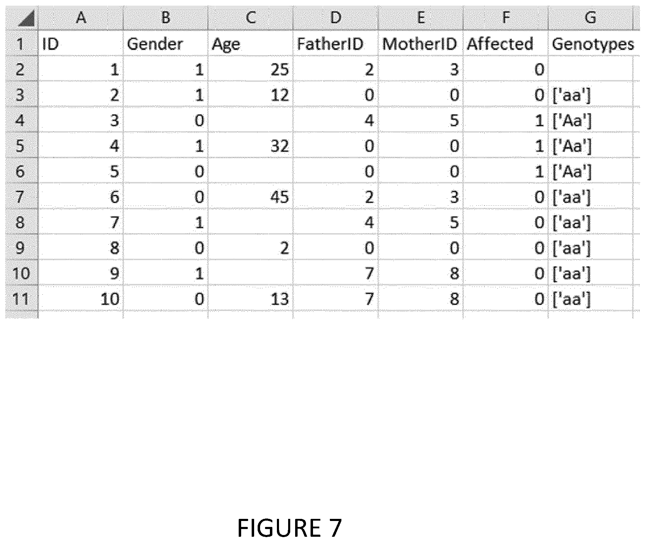

Genotypes are thus assigned for family members but not the patient. The CSV file may be updated to include a new column 'Genotypes' with this new information. Figure 7 illustrates an example of the CSV file with the Genotypes column.

Mendelian/Bayes Risk Scoring module 146 (steps S60-S80)

This module may be considered to carry out steps S60-S80. This module uses known statistical models to determine a genetic risk score and calculates a new measure factor called the 'Danger Factor' weight or the family history risk score.

Inputs to the score module 146 may include: Data from CSV file (in this step is the following are relevant: the age, FatherID, MotherID, Status and the genotypes); the pedigree inheritance type; age window to estimate the risk; and penetrance values of the disease.

Step S50 may comprise determining a genetic risk score using Mendelian analysis or using Bayesian analysis or using both and generating two outputs, or using both and computing an average between the resulting outputs.

In the Mendelian analysis (the Mendelian basic probability risk prediction), Punnett Squares (https://biologydictionary.net/punnett-square/; Davis, L. C. (1993), Origin of the Punnett square, The American Biology Teacher, 55(4), 209-212; Punnett, R. C. (1919), Mendelism, Macmillan and Company, Limited) analysis is used to combine the assigned genotypes of the patient's father and mother and obtain risk probabilities of being affected. A genetic risk score of the patient being a carrier in recessive cases (i.e., the situation when the person is not affected but is carrier of the condition) may also be determined.

In the Bayesian analysis, (the Bayesian probability risk prediction), the Mendelian approach is further refined by additional steps which take into account the assigned genotypes of the patient's child or children and the other parent(s) of those children. This approach is a known process for computing a probability of an individual having a particular genotype based on the genotypes of their children and the other parent. For example, it is described in Ogino, S., & Wilson, R. B. (2004), "Bayesian analysis and risk assessment in genetic counseling and testing", The Journal of Molecular Diagnostics, 6(1), 1-9 (https://www.ncbi.nlm.nih.gov/pmc/articles/PMC1867463/). A Punnett Squares analysis is carried out using the assigned genotypes of the patient's father and mother to obtain prior probabilities. This corresponds to the Mendelian analysis. Next, the genotypes of the patient's partner(s) and children are taken into account and a Bayes analysis is carried out. That is, a Punnett Squares approach is applied over the genotypes of the partner and possible genotypes of the patient to find the genotype possibilities of the children depending on the pedigree inheritance type. Conditional probabilities are obtained for the assigned genotype of each child conditional on the different possible genotypes of the patient. A multiplicative sum is applied for each genotype possibility of the patient based on the conditional probabilities and the number of children:

$$\prod_1^c cond[gp]$$

where,

c = number of children
gp = genotype possibility of the patient depending on inheritance type (e.g. AA, Aa, AA in Autosomal cases)

Then, the cross product is calculated over the combinative matrix of prior probabilities and conditional probabilities, obtaining joint probabilities. Finally, over the joint probabilities Bayes algorithm (https://statswithr.github.io/book/the-basics-of-bayesian-statistics.html) is applied for every genotype possibility of the patient, obtaining the probability of the patient possessing a genotype which causes the condition. The probability of the patient possessing a genotype which causes them to be a carrier in recessive cases can also be determined. Bayes' theorem/algorithm formula:

$$P\left(B_J \mid A\right) = \frac{P\left(A \mid B_J\right) P\left(B_J\right)}{\sum_{i=1}^{n} P\left(A \mid B_i\right) P\left(B_i\right)}$$

where P(A/B) represents a conditional probability so that P(A/B)*P(B) represents a joint probability. This formula can be considered as finding the probability ($P(B_j/A)$) that the patient has genotype $B_j$ given that the child/children have genotype(s) A (the genotype(s) assigned to them) by using:

- $P(A/B_j)$: the probability of the child/children having genotype(s) A given that the patient has genotype $B_j$;
- $P(B_j)$: the probability of the patient having genotype $B_j$;
- $P(A/B_i)$: the probability of the child/children having genotype(s) A given that the patient has genotype $B_i$;
- $P(B_i)$: the probability of the patient having genotype $B_i$ (as determined by the Punnett Squares approach - prior probability).

The probabilities $P(A/B_{i/j})$ are determined using the Punnett Squares approach to predict possible genotypes of the children using the assigned genotype of the other parent and genotype $B_{i/j}$ of the patient. The probabilities $P(B_j)$ are determined using the Punnett Squares approach to predict possible genotypes of the patient based on the assigned genotypes of the patient's parents.

For both Mendelian basic probability risk and Bayes probability risk prediction, depending on the inheritance type, probabilities of genotypes that could cause the patient to have/be affected by the condition are summed to estimate an initial risk of being affected. Probabilities of genotypes that could cause the patient to be a carrier may also be summed to estimate a Carrier risk. The above example assumes that there is only one other parent of the patient's children. If this is not the case then a separate analysis will be carried out for each child/set of children from a particular other parent and a multiplicative sum will be carried out over the resulting probabilities.

The output of step S60 is a genetic risk score and may comprise the final probability of the patient having the disease as computed above by using either the Mendelian or the Bayesian analysis. The step S60 may output two genetic risk scores, one based on the Mendelian approach and the other on the Bayesian approach. The genetic risk score may instead be an average of a score computed based on the Mendelian approach and a score computed based on the Bayesian approach.

Step S70 comprises determining a family history risk score. The family history risk score may be referred to as the Danger Factor measure. The genetic risk score is based on the genotypes information from the closest relatives such as parents, partner and children. But other relevant information like age or status from these relatives and that of other relatives such as aunts, uncles, grandparents, etc. is not considered in the genetic risk score. The danger factor formula considers information from relatives like the number of relatives still alive with the disease (or dead but not because of the disease), the number of relatives dead by the disease (or dead and who had the disease), the relative's current age or age when diagnosed with the disease or at death by such disease.

In an example, the danger factor is determined by determining first to third contributions as follows. Family members are classified by degree levels 1st, 2nd and 3rd (1st degree level: parents, sisters, brothers, children; 2nd degree level: aunts, uncles, grandparents, grandchildren, nieces, nephews, half-siblings; 3rd degree level: first cousins, great-grandparents, great-uncles, great-aunts, great-nieces, great-nephews, great-grandchildren, half-aunts, half-uncles).

First contribution: score for number of relatives that have or had the disease (are known to have or have had the disease) but have not died from the disease (are not known to have died from the disease). That is, the first contribution is based on family members who are known to have or have had the disease but who are still alive or are dead but not because of the disease (according to extracted information):

$$f1 = w'1 * n_{1st} + w'2 * n_{2nd} + w'3 * n_{3rd}$$

where,

n = Number of relatives that are known to have or have had the disease but are not known to have died from the disease
1st, 2nd, 3rd = First, second, and third degree level
w'1, w'2, w'3 = weights of relevance depending on the degree level, may be referred to as first to third primary weighting factors.

The first contribution may be calculated by dividing f1 by the total number of family members of the patient.

Second contribution: Score for number of relatives who are known to have died from the disease (because of the disease):

$$f2 = w''1 * m_{1st} + w''2 * m_{2nd} + w''3 * m_{3rd}$$

where,

m = Number of relatives who are known to have died from the disease
1st, 2nd, 3rd = First, second, and third degree level
w''1, w''2, w''3 = weights of relevance depending on the degree level, may be referred to as first to third secondary weighting factors

The second contribution may be calculated by dividing f2 by the total number of family members of the patient.

Third contribution: Score based on age of relatives:

$$f3 = wn * cond1 + wm * cond2$$

$$cond1 = w1 \sum_{i=1}^{n1st} \left(1 - \frac{age_i}{100}\right) + w2 \sum_{j=1}^{n2nd} \left(1 - \frac{age_j}{100}\right) + w3 \sum_{k=1}^{n3rd} \left(1 - \frac{age_k}{100}\right)$$

$$cond2 = w1 \sum_{i=1}^{m1st} \left(1 - \frac{age_i}{100}\right) + w2 \sum_{j=1}^{m2nd} \left(1 - \frac{age_j}{100}\right) + w3 \sum_{k=1}^{m3rd} \left(1 - \frac{age_k}{100}\right)$$

where

n = Number of relatives that are known to have or have had the disease but are not known to have died from the disease
m = Number of relatives who are known to have died from the disease
1st, 2nd, 3rd = First, second, and third degree level
wn, wm = weights of relevance of cond1 and cond2, respectively, may be referred to as fourth and fifth weighting factors
w1, w2, w3 = weights of relevance depending on the degree level, may be referred to as sixth to eighth weighting factors
For cond1, "age" indicates the family member's age when they were diagnosed with the condition
For cond2, "age" indicates the family member's age when they died

The third contribution may be calculated by dividing f3 by the total number of family members of the patient or by the number of family members of the patient considered in the calculation of cond1 and cond2 if not all of the family members are considered - for example if the relevant age is not known for a family member.

Final Danger Factor score:

$$DF = \alpha * f1 + \beta * f2 + \gamma * f3$$

where $\alpha$, $\beta$, $\gamma$ may be referred to as ninth to eleventh weighting factors, respectively.

The weighting factors described above may be determined through trial and error or through training or may be set by an expert. The factors may depend on any of the considerations below. The following considerations are relevant for the danger score:

- Increase of danger with increasing the age of the patient: This factor is already covered by the penetrance value (described below)
- Increase of danger with multiple deaths in the family by the condition. Therefore, a higher weight may be applied to the number of relatives dead by the disease than with the disease but still alive.
- Information from the closest relatives is more important, so relatives may be categorised by the degree levels (1st degree level: parents, sisters, brothers, children; 2nd degree level: aunts, uncles, grandparents, grandchildren, nieces, nephews, half-siblings; 3rd degree level: first cousins, great-grandparents, great-uncles, great-aunts, great-nieces, great-nephews, great-grandchildren, half-aunts, half-uncles) and different weights of relevance may be applied depending on the degree level, i.e. a higher weight in the cases of 1st degree level relatives, and a lower weight in the category of 3rd degree level.
- The age of the relatives is also relevant as follows:

  - More danger with relatives having disease at younger age.
  - More danger with relatives dead by disease at younger age. May be incorporated into the danger factor with a higher weight than previous statement
  - Degree level of relatives may be taken into account here.

The family history risk score may be calculated by incorporating an additional, final step of dividing by the total number of family members of the patient (for example if the first to third contributions were not calculated in this way). The primary and secondary weighting factors may be the same, for example because of the ninth and tenth weighting factors which define the relative weights of the first and second contributions.

A penetrance value may be determined. The penetrance value is for example the percentage of individuals in a group with a given genotype who exhibit the phenotype associated with that genotype. The individuals in a group may be of a particular age range. The penetrance value may be determined based on literature or may be provided to the module/processor. The penetrance value may depend on an age (a predictive age). The age of the patient and a projection age or age window may be summed to calculate the age on which the penetrance value depends. The projection age or age window is the number of years in future for which the risk is to be estimated, e.g., in 5 years/7 years/10 years/etc.). For instance, if age of patient is 28 and the age window is 5 years in the future, the resultant sum value (the predictive age) will be 33 years old. The projection age may be zero - i.e. the predictive age may be the current age of the patient. The penetrance value will also depend on the condition under consideration.

The genetic family history risk score or final risk of being affected by disease is:

$$\text{Genetic family history risk score} = \text{Genetic } (Basic \text{ or } Bayes) \text{ } Risk \text{ score} * Penetrance * DF$$

The genetic family history risk score could also be calculated by summing the family history risk score and the genetic risk score multiplied by the penetrance value. That is, an alternative version of the above formula may be:

$$\text{Genetic family history risk score} = \text{Genetic } (Basic \text{ or } Bayes) \text{ } Risk \text{ score} * Penetrance + DF$$

The genetic risk score may be determined using the Mendelian approach (in which case the score may be referred to as "basic") or using the Bayesian approach (in which case the score may be referred to as "Bayes"). The genetic risk score may be a combination of the two approaches (i.e. average). Two genetic family history risk scores may be output: one with a basic genetic risk score (which may be referred to as a basic genetic family history risk score) and the other with a Bayes genetic risk score (which may be referred to as a Bayes genetic family history risk score).

In the above it is assumed that one genotype is assigned to each family member in step S50. It may be that multiple genotypes are assigned to one or some or all family members. For example, the combination of the rules of the determined inheritance mode and the extracted information may not lead to a definitive genotype for every family member. In case a family member has more than one genotype any process dependent on that genotype may be carried out once per assigned genotype. This may for example give rise to multiple genotypes for another family member in step S50. Any calculation dependent on a family members genotype who has multiple assigned genotypes may be carried out once per assigned genotype and an average of the resulting values may be obtained. Where a calculation/process is dependent on multiple family members who each has multiple assigned genotypes the calculation/process may be carried out once per each combination of genotypes and an average taken as the final result of that calculation.

Some aspects disclosed herein may include determining a diagnosis. For example, steps of the method in Figure 2 may be iterated for different conditions as described above and at least one condition with the highest genetic family history risk score or with a genetic family history risk score above a threshold genetic family history risk score may be determined as a diagnosis. A diagnosis determined in this way may be referred to as a genetic family history diagnosis.

Some aspects disclosed herein include outputting a diagnosis for the patient based on the genetic family history risk score. Embodiments may comprise prediction and monitoring of potential diagnosis and future risks for patients by the automated analysis of the joint features of symptoms, genetic family history risk score and physiological measurements e.g. collected through wearable devices. That is, embodiments may include outputting a diagnosis based on any of a genetic family history risk score (e.g. as determined above), at least one symptom of the patient, and at least one physiological measurement of the patient. A wearable device may be used to collect any of the at least one symptom and the at least one physiological measurement. The at least one symptom and/or the at least one physiological measurement may be collected through a sensor and/or may be extracted from medical history information (e.g. an EHR) or may be obtained in other ways.

The physiological measurements (e.g. vital signs) and the current symptoms may be considered together and an estimation decision of current diagnoses provided following clinical rule-based algorithms. Such algorithms involve a set of premises (the vital signs and symptoms), analyse a set of logical rules with such premises, and return a conclusion based on the output of the logical rules. The set of logical rules may be defined by physicians and healthcare professionals. A simple example is: *if Symptom(nasal congestion) and Symptom(headache) and Thermometer(value > 38°C) -> Diagnosis(Flu, cold)*.

The outputted potential diagnoses are corroborated with the at least one genetic family history diagnosis. The at least one diagnosis resulting from the analysis of the symptoms and physiological measurements is compared with the at least one genetic family history diagnosis. Below, three main cases are considered:

1. If at least one diagnosis appears in the results from both analyses, it is marked as a primary diagnosis, and the rest of the diagnoses are extracted as secondary or less relevant.

2. If there is no diagnosis appearing in the results from both analyses, all diagnoses extracted will be considered of the same importance.

3. If there is no at least one genetic family history diagnosis, only the diagnoses output by the analysis of vital signs and symptoms will be taken into consideration.

In an example, a method of outputting the at least one diagnosis for the patient (which may be a part of the method in Figure 2) comprises

A. predicting at least one at least one genetic family history diagnosis as described above;
B. predicting at least one symptom diagnosis, the at least one symptom diagnosis being at least one diagnosis of the patient based on the at least one symptom of the patient;
C. predicting at least one measurement diagnosis, the at least one measurement diagnosis being at least one diagnosis of the patient based on the at least one measurement of the patient, and
D. comparing the at least one at least one genetic family history diagnosis, the at least one symptom diagnosis, and the at least one measurement diagnosis and, when at least one diagnosis among the at least one genetic family history diagnosis is the same as a diagnosis among the at least one symptom diagnosis and at least one diagnosis among the at least one measurement diagnosis, outputting the at least one diagnosis as at least one final diagnosis.

The feature described above of corroboration may be considered accomplished by feature D.

Predicting the at least one symptom diagnosis may comprise: maintaining a symptom diagnosis score for each of a plurality of possible diagnoses; adjusting at least one of the symptom diagnosis scores based on a set of symptom diagnosis rules and based on the at least one symptom of the target patient; and determining at least one of the possible diagnoses having the highest symptom diagnosis score as the at least one symptom diagnosis.

Predicting the at least one physiological diagnosis may comprise: maintaining a physiological diagnosis score for each of a plurality of possible diagnoses; adjusting at least one of the physiological diagnosis scores based on a set of physiological diagnosis rules and based on the at least one physiological measurement of the target patient; and determining at least one of the possible diagnoses having the highest physiological diagnosis score as the at least one physiological diagnosis.

Determining the at least one at least one genetic family history diagnosis may comprise disregarding a diagnosis if its genetic family history score is below a genetic family history score threshold. Determining the at least one symptom diagnosis may comprise disregarding a diagnosis if its symptom diagnosis score is below a symptom diagnosis score threshold. Determining the at least one physiological diagnosis may comprise disregarding a diagnosis if its physiological diagnosis score is below a physiological diagnosis score threshold.

The diagnosis scores for the physiological and symptom diagnoses may be calculated following a rule-based approach defining an amount to be added or subtracted to a score in view of one or more particular symptoms or physiological measurements within particular ranges.

Example - diagnosing 'heart disease'

Described below is an example following embodiments to diagnose heart disease (in this specific case a diagnosis of Heart Failure is output).

Collection of information

Implicit data: Physiological measurements (i.e., vital signs) may be collected e.g. in real-time from the data extracted with the sensors of a wearable device. In this example the following physiological measurement values are collected (these measurements are relevant in the context of Heart Disease):

- ECG sensor: Time-series values of the last 15 minutes. Very irregular values and abnormalities present in the electrocardiogram waveforms. The irregularities and abnormalities may be identified/detected by comparing, using a computer, the measurements with other ECG measurements of healthy and of unhealthy hearts.
- Thermometer: Temperature of 38.6° measured
- EMG sensor: Time-series values of the last 15 minutes. Abnormal values and low electromyographic activity measured. The abnormalities may be identified/detected and the electromyographic activity classified (e.g. as low) by comparing, using a computer, the measurements with other EMG measurements of healthy and of unhealthy hearts.

- Pacemaker pulse detection: Low breathing rate and high and enlarged heart rate measured. The breathing and heart rate may be classified (e.g. as low/high and enlarged) by comparing, using a computer, the measurements with other pacemaker pulse detection measurements of healthy and of unhealthy hearts.
- GSR sensors: Increased skin conductivity with low GSR activity values. The skin conductivity and GSR activity values may be classified (e.g. as increased and low) by comparing, using a computer, the measurements with other GSR measurements of people with healthy and unhealthy hearts
- Other measurements from Optical sensors:

- High blood pressure
- pH analysis: acid-base balance
- Skin light intensity: Values in normal range
- Blood-glucose levels: values in the normal range

Physiological measurements may be classified e.g. as "normal", "low", "healthy", "abnormal", etc., by comparing (using a computer) those measurements with corresponding measurements of other people (e.g. with healthy and unhealthy hearts, in this case).

The symptoms may be collected in a similar way to the FMH extractor from the unstructured medical data and/or may be reported by the patient.

Receive/Transformation of information

Some useful definitions are below:

- P-wave axis: a measure calculated from an ECG of the net direction of atrial depolarization. It is determined by measuring net positive or negative P-wave deflections on all six limb leads and calculating the net direction of electrical activity using the hexaxial reference system. Abnormal P-wave axis may be defined as any value outside 0-75°. The P wave is an integral part of an ECG. It represents the electrical depolarization of the atria of the heart. It is typically a small positive deflection from the isoelectric baseline that occurs just before the QRS complex.

- QRS duration: A combination of the Q wave, R wave and S wave, the "QRS complex" represents ventricular depolarization. The normal duration (interval) of the QRS complex may be considered to be between 0.08 and 0.10 seconds - that is, 80 and 100 milliseconds. When the duration is between 0.10 and 0.12 seconds, it may be considered intermediate or slightly prolonged. A QRS duration of greater than 0.12 seconds may be considered abnormal.

- QRS axis: The QRS axis represents the major vector of ventricular activation, which is the overall direction of electrical activity.

- General definition of axis in the context of the heart: When all electrical signals from the heart are averaged, the direction of the average electrical depolarization can be indicated with an arrow (vector). This is the heart axis. A change of the heart axis or an extreme deviation can be an indication of pathology. To determine the heart axis the extremity leads only (not V1-V6) are analysed. If focus is especially given to leads I, II, and AVF a good estimate of the heart axis can be made. An important concept in determining the heart axis is the fact that electricity going towards a lead yields a positive deflection in the electric recording of that lead. It can be useful to imagine the leads as cameras looking at the heart. Lead I looks horizontally from the left side. Lead II looks from the left leg. Lead III from the right leg and lead AVF from below towards the heart. A positive deflection here is defined as the QRS having a larger 'area under the curve' above the baseline than below the baseline.

Following the physiological measurements and their values described above for this example, the following information is extracted in this example:

- ECG sensor. Transform the (15 minutes worth of) time-series to relevant categorical and numerical values. Specific values in this example include (with associated rules indicated inside the parentheses {}): P-wave axis 99° (enlarged) {normal range between 0°-75°}, QRS duration = 0.17 (prolonged duration) {normal duration between 0.08 and 0.10}, Time to ID=62ms (abnormal) {values > 50ms are considered abnormal}, QRS axis=-45° (left axis deviation) {left axis deviation if QRS axis between -30° and -90°}. Therefore the categorical information extracted from the ECG sensor measurement includes: P-wave axis is 'enlarged'; QRS duration is 'prolonged'; time to ID is abnormal; left axis deviation=True.
- Numerical value of temperature=38.6°.
- EMG sensor. Transform the (15 minutes worth of) time-series to relevant categorical and numerical values. Specific values in this example include (with associated rules indicated inside the parentheses {}): Cross-Sectional Area (CSA) muscle - 134 $cm^2$ (reduction) {normal values 165.2 $\pm$ 7.4 $cm^2$, P=0.002}, muscle strength ~ 227 N (reduction) {normal values 286.9 $\pm$ 17.1 N, P<0.05}, muscular fatigability ~ -2.10 (high) {normal values - 0.54 $\pm$ 0.20 N/s, P<0.01}, submaximal contraction ~ 68 % (low) {normal values 114 $\pm$ 36%; P<0.05}. Therefore the categorical information extracted from the ECG sensor measurement includes: CSA muscle as 'reduction', muscle strength as 'reduction', muscular fatigability as 'high', submaximal contraction as 'low'. In the above measurements, P is the p-value. In statistics, the p-value is the probability of obtaining results at least as extreme as the observed results of a statistical hypothesis test, assuming that the null hypothesis is correct. A statistically significant test result (P ≤ 0.05) means that the test hypothesis is false or should be rejected. A P value greater than 0.05 means that no effect was observed.

- Pacemaker pulse detection. The categorical information extracted from the pacemaker pulse detection measurement includes: breathing rate='low', heart rate='high', heart rate='enlarged'.
- GSR sensors (skin conductivity measurement): Categorical information extracted: Values around less than 15kohms (low values indicating "negative" emotional states such as "sad", "fear", "angry", etc.)
- Other measurements from Optical sensors. Categorical values:

  • Blood pressure=high
  • pH analysis: acid-base balance
  • Skin light intensity: normal
  • Blood-glucose levels: normal

In the above example measurements, CSA muscle is the area of the cross section of a muscle perpendicular to its fibres, generally at its largest point (it is typically used to describe the contraction properties of pennate muscles); muscle strength may be defined as the ability to exert force on an external resistance; muscular fatigability may be is defined as a decrease in maximal force or power production in response to contractile activity; submaximal contraction may be defined as e.g. a number of all contractions without maximal effort. Further information on EMGs and particular quantities that may be measured may be found at https://www.ncbi.nlm.nih.gov/pmc/articles/PMC3821366/.

An example of the symptom information in this example, for example recorded by the target patient using a microphone and converted to textual data or extracted from medical history data is: *"I feel tired and weak, and I have chest pain and difficulty breathing. Sometimes I experience tachycardia, nausea and accelerated heartbeat. I have had a persistent cough for several days"*.

Processing information of symptoms

Real-time symptoms: NER techniques are used to extract the following entities from the textual data relating to symptoms: [tired, weak, chest pain, difficulty breathing, tachycardia, nausea, accelerated heartbeat, persistent cough]

Determining Diagnoses

A rule-based approach is followed to determine physiological diagnosis scores for a number of heart diseases (and other diseases/conditions) based on the physiological measurements above in this example. Initially, the physiological diagnosis scores start at 0. A change in a physiological diagnosis score is indicated as follows: a physiological measurement resulting in adding 1 to a diagnosis score is shown below by "Diagnosis([condition], +1)".

If P-wave=='enlarged':
Diagnosis(atrial enlargement, +1), Diagnosis(chronic respiratory disease, +1), Diagnosis(heart failure, +1), Diagnosis(congestive heart failure, +1), Diagnosis(cardiomyopathies, +1), Diagnosis(congenital heart defects, +1), Diagnosis(valvular heart disease, +1);

If *QRS_duration=='prolonged'*:
Diagnosis(heart failure, +1), Diagnosis(congestive heart failure, +1), Diagnosis(atrial fibrillation, +1), Diagnosis(coronary disease, +1), Diagnosis(arrhythmia, +1), Diagnosis(ischemic heart disease, +1), Diagnosis(valvular heart disease, +1);

If *left_axis_deviation*==True:
Diagnosis(ischemic heart disease, +1), Diagnosis(heart failure, +1), Diagnosis(congestive heart failure, +1), Diagnosis(congenital heart defects, +1);

If *time_to_ID* > 50:
Diagnosis(heart failure, +1), Diagnosis(coronary heart disease, +1);

If *CSA_muscle*=='reduction':
Diagnosis(heart failure, +1), Diagnosis(congestive heart failure, +1), Diagnosis(skeletal muscle atrophy, +1), etc.

If *muscle_strength*=='reduction':

Diagnosis(heart failure, +1), Diagnosis(congestive heart failure, +1), Diagnosis(skeletal muscle atrophy, +1), Diagnosis(muscular dystrophy, +1), etc.

If *muscle_ fa tigability=='h\gh':*
Diagnosis(heart failure, +1), Diagnosis(congestive heart failure, +1), Diagnosis(skeletal muscle atrophy, +1), Diagnosis(muscular dystrophy, +1), etc.

If *submaximal_contraction=='low':*
Diagnosis(heart failure, +1), Diagnosis(congestive heart failure, +1), Diagnosis(skeletal muscle atrophy, +1), Diagnosis(muscular dystrophy, +1), Diagnosis(neurological disorders, +1), etc.

If *breathing_rate=='low':*
Diagnosis(heart failure, +1), Diagnosis(congestive heart failure, +1), Diagnosis(bradypnea, +1), Diagnosis(lung disorders, +1), Diagnosis(chronic bronchitis, +1), Diagnosis(pneumonia, +1), etc.

If *heart* rate=='high':
Diagnosis(heart failure, +1), Diagnosis(congestive heart failure, +1), Diagnosis(valvular heart disease, +1), Diagnosis(coronary heart disease, +1), Diagnosis(cardiomyopathies, +1), etc.

If *heart* rate=='enlarged':
Diagnosis(heart failure, +1), Diagnosis(congestive heart failure, +1), Diagnosis(stroke, +1), Diagnosis(cardiomyopathies, +1);

If *GSR* < 15: //low values -> negative -> associated to emotional state of sad, anxiety or anger.

Diagnosis(depression, +1), Diagnosis(anxiety, +1), etc.;

If *blood_pressure_fluid=='high':*
Diagnosis(heart failure, +1), Diagnosis(congestive heart failure, +1), Diagnosis(heart attack, +1), Diagnosis(stroke, +1), Diagnosis(arterial disease, +1), Diagnosis(aortic aneurysm, +1), etc.

There may of course be other diagnosis scores not considered above and in places these are indicated by "etc.". In another example focused on heart disease, only diagnosis scores relating to heart diseases may be included and diagnosis scores related to e.g. depression and anxiety may not be included/considered.

After summing each diagnosis score and selecting the highest four, the physiological diagnoses are:

- Heart failure with score=12
- Congestive heart failure with score=11
- Cardiomyopathies with score=3
- Valvular heart disease with score=3

A rule-based approach is followed to determine symptom diagnosis scores for a number of heart diseases based on the symptoms above in this example. Initially, the symptom diagnosis scores start at 0. Some comments about the rules are given inside "// //" below.

If *tired_weak*==True:
//This symptom may imply many diseases besides 'heart diseases'. Therefore, addition to score may be less than 1 in other examples//.
Diagnosis(heart failure, +1), Diagnosis(congestive heart failure, +1), etc.

If *chest_pain*==True:
//This symptom could be caused by many other diseases, but, if physiological measures and family history support case of 'heart disease', it would be determinant.// Diagnosis(heart failure, +1), Diagnosis(congestive heart failure, +1), Diagnosis(muscle strain, +1), Diagnosis(GERD, +1), Diagnosis(asthma, +1), Diagnosis(costochondritis, +1), Diagnosis(valvular heart disease, +1), etc.

If *bad_breath*==True:
//Same comment as previous rule//

Diagnosis(heart failure, +1), Diagnosis(congestive heart failure, +1), Diagnosis(pneumonia, +1), Diagnosis(coronavirus, +1), Diagnosis(asthma, +1), Diagnosis(emphysema, +1), Diagnosis(valvular heart disease, +1), etc.

If *tachycardia*==True:
//Relevant for heart diseases//

Diagnosis(heart failure, +1), Diagnosis(congestive heart failure, +1), Diagnosis(coronary artery disease, +1), Diagnosis(heart attack, +1), Diagnosis(congenital heart defects, +1), Diagnosis(valvular heart disease, +1), Diagnosis(hypertension, +1), Diagnosis(cardiomyopathies, +1), etc.

If *nausea*==True:
//This symptom may imply many diseases besides 'heart diseases'. Therefore, addition to score may be less than 1 in other examples//Diagnosis(heart failure, +1), Diagnosis(congestive heart failure, +1), etc.

If *heartbeat_accelerated*==True:
//My be considered synonym to *'tachycardia'*. Relevant to heart diseases// Diagnosis(heart failure, +1), Diagnosis(congestive heart failure, +1), Diagnosis(coronary artery disease, +1), Diagnosis(heart attack, +1), Diagnosis(congenital heart defects, +1), Diagnosis(valvular heart disease, +1), Diagnosis(hypertension, +1), Diagnosis(cardiomyopathies, +1), etc.

If *persistent_cough*==True:
//This symptom could be caused by many other diseases, but, if physiological measures and family history support case of 'heart disease', it would be determinant//. Diagnosis(heart failure, +1), Diagnosis(congestive heart failure, +1), Diagnosis(COPD, +1), Diagnosis(GERD, +1), Diagnosis(asthma, +1), Diagnosis(coronavirus, +1), etc.

After summing each diagnosis score and selecting the highest four the symptom diagnoses are:

- Heart failure with score=7
- Congestive heart failure with score=7
- Valvular heart disease with score=4
- Asthma with score=3

In this example, any diagnoses appearing in the top four symptom diagnoses as well as the top four physiological diagnoses are preliminary selected. Therefore, the following diagnoses are preliminarily selected:

- Heart failure
- Congestive heart failure
- Valvular heart disease

The preliminarily selected diagnoses are then corroborated by comparing them with the at least one at least one genetic family history diagnosis.
In this example, the conditions and their associated genetic family history risk scores are assumed as follows (an example of determining genetic family risk scores is described in another section below):

- Heart failure = 0.45
- Valvular heart disease = 0.35
- Heart attack = 0.4
- Carotid artery disease = 0.2
- Diabetes = 0.2
- Hypertension = 0.2

In this example, a threshold of 0.3 is applied to the diagnoses. Then, the genetic family history diagnoses output are (ranked according to their scores):

- Heart failure
- Heart attack
- Valvular heart disease

# EP 4 451 274 A1

In this example, the preliminarily selected diagnoses (based on the physiological measurements and the symptoms) are compared with the at least one at least one genetic family history diagnosis based on the following heuristic rules:

1. If a diagnosis appears in the at least one at least one genetic family history diagnosis and in the preliminarily selected diagnoses, the diagnosis will be marked as a primary diagnosis, and the other at least one at least one genetic family history diagnosis marked as secondary or less relevant.
2. If there is no diagnosis which appears in the at least one at least one genetic family history diagnosis and in the preliminarily selected diagnoses, all diagnoses extracted will be considered at the same importance.
3. If there is no at least one at least one genetic family history diagnosis, the preliminarily selected diagnoses are output as at least one final diagnosis.

In this example, the at least one final diagnosis output is:

- Primary diagnoses = Heart failure and Valvular heart disease
- Secondary diagnoses = Heart attack

Example for diagnosing 'mental illness'

Described below is an example following disclosed aspects to diagnose mental illness (in this specific case a diagnosis of Depression is output).

Collection of information

Implicit data: Physiological measurements (i.e., vital signs) may be collected e.g. in real-time from the data extracted with sensors of a wearable device. In this example, the following physiological measurements (values) are collected. Most of them are not relevant to the diagnosis of mental health conditions such as depression. The relevance of some of the physiological measurements is indicated below in brackets.

- ECG sensor: Time-series values of the last 15 minutes. Irregular heart rate variability present (https://www.sciencedaily.com/releases/2017/11/171121095403.html) in the electrocardiogram waveforms. (Relevant to the diagnosis of mental health conditions despite the fact it could indicate other diseases)
- Thermometer: Temperature of 36.5°
- EMG sensor: Time-series values of the last 15 minutes. Normal values shown. [May not be relevant in this example]
- Pacemaker pulse detection: High heart rate. (Relevant to the diagnosis of mental health conditions despite the fact it could indicate other diseases)
- GSR sensors: Abnormal variability measurements. Alternance of increased and decreased skin conductivity and GSR activity values. (Relevant to the diagnosis of mental health conditions)Other measurements from Optical sensors:

- High blood pressure
- pH analysis: low pH levels (relevant)
- Skin light intensity: No values out of the normal range
- Blood-glucose levels: big fluctuations in blood sugar levels (relevant)

Physiological measurements may be classified e.g. as "normal", "low", "healthy", "abnormal", etc., by comparing (using a computer) those measurements with corresponding measurements of other people (e.g. with healthy and unhealthy hearts, in this case).

Receive/Transformation of information

Following the physiological measurements and their values described above for this example, the following information is extracted in this example. The relevance of some of the extracted and processed physiological measurements is indicated below in brackets

- ECG sensor: Transform the (15 minutes worth of) time-series to relevant categorical and numerical values. Specific values in this example include irregular heart rate variability=True (not determinant in the diagnosis of mental health conditions)
- Numerical value of temperature=36.5° - within normal range

33

- EMG: Normal values (not relevant in the diagnosis of mental health conditions)
- Pacemaker pulse detection: heart rate=high (Not determinant in the diagnosis of mental health conditions as this measurement could indicate multiple other diseases).
- GSR sensors: large variability of high and low values. The categorical information extracted is Values_variability=high (Relevant in the diagnosis of mental health conditions).
- Other measures from Optical sensors. Categorical values:

  • Blood pressure=high
  • pH analysis: low-levels
  • Skin light intensity: normal
  • Blood-glucose levels: big fluctuations

An example of the symptom information in this example, for example recorded by the target patient using a microphone and converted to textual data or extracted from medical history data is: *"I feel tired, apathetic and lack of enthusiasm. I usually have episodes of deep sadness and stress. Also I have no appetite and I feel anxious".*

Processing information of symptoms

Real-time symptoms: NER techniques are used to extract the following entities from the textual data relating to symptoms: [tired, apathetic, lack of enthusiasm, sadness, stress, no appetite, anxious]

Determining Diagnoses

A rule-based approach is followed to determine physiological diagnosis scores for a number of mental health conditions (and other diseases/conditions) based on the physiological measurements above in this example. Initially, the physiological diagnosis scores start at 0. A change in a physiological diagnosis score is indicated as follows: a physiological measurement resulting in adding 1 to a diagnosis score is shown below by "Diagnosis([condition], +1)".

If *heart rate variability*=='irregular':
Diagnosis(heart failure, +1), Diagnosis(congestive heart failure, +1), Diagnosis(stroke, +1), Diagnosis(depression, +1), Diagnosis(anxiety, +1), etc.;

If *GSR*_fluctuation = 'high':
Diagnosis(depression, +1), Diagnosis(anxiety, +1), etc.;

If *blood_pressure_fluid*=='high':
Diagnosis(heart failure, +1), Diagnosis(congestive heart failure, +1), Diagnosis(heart attack, +1), Diagnosis(stroke, +1), Diagnosis(arterial disease, +1), Diagnosis(aortic aneurysm, +1), Diagnosis(depression, +1), Diagnosis(anxiety, +1), etc.

If *pH-levels* = 'low':
Diagnosis(depression, +1), Diagnosis(anxiety, +1), Diagnosis(congestive heart failure, +1), etc.;

If *glucose_fluctuation* = 'large':
Diagnosis(depression, +1), Diagnosis(anxiety, +1), Diagnosis(diabetes, +1), etc.;

There may of course be other diagnosis scores not considered above and in places these are indicated by "etc.". In another example focused on heart disease, only diagnosis scores relating to heart diseases may be included and diagnosis scores related to e.g. depression and anxiety may not be included/considered.

After summing each diagnosis score and selecting the highest four, the physiological diagnoses are:

- Depression with score=5
- Anxiety with score=5
- Congestive heart failure with score=3
- Heart failure=2

A rule-based approach is followed to determine symptom diagnosis scores for a number of heart diseases based on the symptoms above in this example. Initially, the symptom diagnosis scores start at 0. Some comments about the rules are given inside "// //" below.

```
If tired_weak==True:
// This symptom may imply many diseases besides 'heart diseases'. Therefore, addition to score may be less than
1 in other examples //
Diagnosis(heart failure, +1), Diagnosis(congestive heart failure, +1), Diagnosis(depression, +1), Diagnosis(anxiety,
+1), etc.

If apathetic==True:
//Relevant in the diagnosis of mental health conditions//
Diagnosis(depression, +1), Diagnosis(major depressive disorder, +1), etc.

If lack of enthusiasm==True:
//Relevant in the diagnosis of mental health conditions//
Diagnosis(depression, +1), Diagnosis(major depressive disorder, +1), Diagnosis(suicidal ideation, +1), etc.

If sadness==True:
//Relevant in the diagnosis of mental health conditions//
Diagnosis(depression, +1), Diagnosis(major depressive disorder, +1), Diagnosis(suicidal ideation, +1), etc.

If stress_anxious==True:
Diagnosis(anxiety, +1)

If no_appetite==True:
Diagnosis(depression, +1), Diagnosis(major depressive disorder, +1, Diagnosis(anorexia, +1), Diagnosis(nutrition
disorders, +1), etc.
```

After summing each diagnosis score and selecting the highest four the symptom diagnoses are:

- Depression with score=5
- Major depressive disorder with score=4
- Anxiety with score=2
- Suicidal ideation with score=2

In this example, any diagnoses appearing in the top four symptom diagnoses as well as the top four physiological diagnoses are preliminary selected. Therefore, the following diagnoses are preliminarily selected:

- Depression
- Anxiety

In this example, the preliminarily selected diagnoses are then corroborated with the at least one at least one genetic family history diagnosis.
In this example the conditions and their associated genetic family history risk scores are assumed as follows (an example of determining genetic family risk scores is described in another section below):

- Depression = 0.9
- Anxiety = 0.9
- Bipolar disease = 0.35
- Suicide = 0.2
- Mental illness = 0.2
- Hypertension = 0.2

In this example, a threshold of 0.3 is applied to the diagnoses. Then, the genetic family history diagnoses output are (ranked according to their scores):

- Depression
- Anxiety
- Bipolar disease

In this example, the preliminarily selected diagnoses (based on the physiological measurements and the symptoms) are compared with the genetic family history diagnoses based on the following heuristic rules:

1. If a diagnosis appears in the at least one at least one genetic family history diagnosis and in the preliminarily selected diagnoses, the diagnosis will be marked as a primary diagnosis, and the other at least one at least one genetic family history diagnosis marked as secondary or less relevant.

2. If there is no diagnosis which appears in the at least one at least one genetic family history diagnosis and in the preliminarily selected diagnoses, all diagnoses extracted will be considered at the same importance.

3. If there is no at least one at least one genetic family history diagnosis, the preliminarily selected diagnoses are output as at least one final diagnosis.

In this example, the at least one final diagnosis output is:

- Primary diagnoses = Depression and Anxiety
- Secondary diagnoses = Bipolar disease

In this example all three factors are considered in generating at least one diagnosis. However in other examples the physiological measurements may not be considered. In fact, the at least one diagnosis may be based on any of the physiological measurements, symptoms, and family medical history data.

The at least one final diagnosis output may therefore be an at least one genetic family history diagnosis or may be at least one final diagnosis determined as described above (optionally including primary and secondary diagnoses).

Risk Score & Alert System Integrator 160

This component is in charge of transmitting information to the device 200 and/or adapting the information to be visualized in the device 200. The information output from the method in Figure 2 and e.g. performed by the estimation module 100 may comprise at least one condition and the associated genetic family history risk scores or at least one final diagnosis as described above.

The EHR updater 180 (or updater module 180) updates the patient's EHR with the information. This component may connect directly with the patient's profile of a healthcare system's database to integrate the information in such database in relation with patient's health risks, calculated by the system. The EHR updater 180 may also monitor the EHR (and/or family members' EHRs) for new medical history information and may cause the system 11 to repeat the generation of the information (genetic family history risk score(s) and/or at least one final diagnosis) with the new medical history information included.

The integrator module 160 also provides explainable reasons for the information output. Such explanations may be included in the patient EHR so that the doctor can check the patient's profile and understand why the patient has got those specific health risks. These explanations may be constructed by heuristic rule-based techniques, splitting each step of the workflow to show the intermediate results and the process of our calculations.

Below is an example of these explanations.

*You are at 8.5% risk of running the condition in the future, and, at 75.0% risk of being carrier of the condition. The result of 8.5% risk is coming from next considerations:*

- *The family pedigree follows AUTOSOMAL_RECESSIVE mendelian pattern. From such pattern, we can estimate the genotypes ['AA', 'Aa'] for the mother and the genotypes ['aa'] for the father.*
- *Then, we combine the genotypes from mother and father following Punnett Squares approach and we obtain the above table with the probabilities of each genotype for the patient.*
- *Since the mendelian pattern is AUTOSOMAL_RECESSIVE, only the genotype with two lower letters (e.g., 'aa') will be affected by condition. Therefore, aggregating probabilities of genotypes that could be affected by the condition, we obtain an initial risk of 25.0.*
- *But, as the patient age is 38 years old, and we apply an age window of 7, the risk estimation would be for 45 years old, with a penetrance of 0.34.*
- *The inclusion of penetrance (25.0 * 0.34) returns the outputted risk of 8.5%.*

*The result of 75.0% carrier risk is because since the mendelian pattern is AUTOSOMAL_RECESSIVE, all genotypes with at least one capital letter (e.g., Aa) could be carriers of condition. Aggregating the probabilities of those genotypes in above table, we obtain the value of 75.0% for carrier risk.*

To adapt the patient's health risks information for output by the magnet IoT device 200, the following may be sent to the device: the list of relevant diseases of the patient based on the family pedigree, and, for each disease the correspondent probabilities of Basic Risk and Bayes Risk of being affected or the genetic family history risk scores. The information described above (genetic family history scores and/or at least one final diagnosis) may be sent alternatively or additionally. An Alert Colour System may define colours shown on the device 200 corresponding to the level of risk (genetic family history risk score). The probability risk to be linked in the system of colours may be the average between Basic and Bayes risk. For instance, with a Basic risk (via Mendelian analysis) of 12.5% and a Bayes risk of 9.37%, the probability risk will be: $\frac{(12.5+9,37)}{2} = \mathbf{10.935\%}$ .

In an example implementation six colours for the Alert Colour System are defined, a kind of warning semaphore. Ranges of probabilities for making the link between the risk value and the correspondent colour among the six options are defined, e.g. the following:

• Strong Green    Probabilities from 0% to 9.99%
• Soft Green    Probabilities from 10% to 19.99%
• Soft Yellow    Probabilities from 20% to 34.99%
• Strong Yellow    Probabilities from 35% to 49.99%
• Soft Red    Probabilities from 50% to 64.99%
• Strong Red     Probabilities from 65% to 100%

Above, probabilities may mean the probability risk defined above or the genetic family history risk scores.
Following accepted standardization and collective assumption, for example, near to Strong Green may mean less risk and danger, while increasing the warm colours in the semaphore may indicate greater risk until the Strong Red is reached which may indicate the most risk and danger of being affected by the disease. In previous example with a probability risk of **10.935%,** the alert colour for the patient would be Soft Green. Once the associated colour is determined for a given condition, such colour flag may be sent by the integrator module 160 for the corresponding condition to the device 200 in order to turn on such colour light in the device 200. If the risk/condition changes due to updates the colour will be turned off and e.g. a new colour may be turned on.

Device 200 Interface and Architecture

The interface and the architecture of the device 200 will be described. Inputs from the integrator module 160 may include any of: List of diseases relevant for the patient depending on the family history; the probability of basic risk score for each disease; the probability of the Bayes risk score for each disease; the flag colour associated to each disease; conditions determined as relevant (having a genetic family history risk score above a threshold or the condition having the highest genetic family history risk score); at least one final diagnosis as described above; the genetic family history risk score(s) for the condition(s) or diagnosis(es) (and optionally information indicating the associated colour).

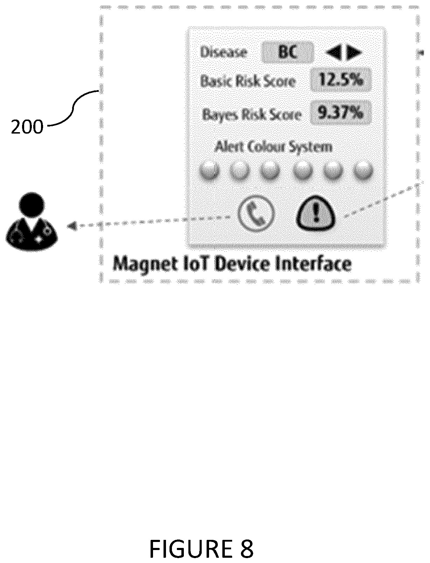

Figure 8 illustrates an example of the interface of the device 200. The device 200 may be a magnet device for example that is to stay at home and may be stuck on any magnetized surface, such as a fridge for instance, to be easily visible by the patient in any moment. In this example the device 200 comprises:

• an output screen to show the name of the disease of interest (e.g., BC for Breast Cancer).
• Interactive directional buttons so that the patient can push such buttons to go forward/backward over the list of diseases
• Output screens to show the risk probabilities relating to the disease selected (in the example BC=Breast Cancer) for two models (Basic Mendelian model and Bayes algorithm model). Instead the device 200 may display the genetic family history risk score(s).
• Light elements (e.g. lights) to show in an understandable way the risk following a kind of semaphore signal system to warn the patient of the risk/danger in real-time.
• Call Interactive button. When the patient pushes this button a direct call with her/his primary care doctor may be established and/or in the EHR system may appear automatically for the doctor the summary information of the patient for the disease selected (the health risks probabilities/genetic family history risk score(s), the flag light associated to patient's risk, and, the explainability reasons of the risk values only accessible for the doctor). In this way, if the patient wants to call the doctor because of concerns about the health risk is increasing or concerns about their diagnosis, the doctor will see directly the relevant records of the patient related to such call without the need of doing specific queries or searches in the system
• Warning Interactive button (the button with an exclamation mark). When the patient pushes this button a warning message will be forwarded to her/his EHRs, updating the relevant information related to this warning in the records.

The system will send alert signal to her/his primary care doctor. A priority may be indicated depending on the risk/genetic family history risk score/flag colour and this may be used to order warnings relating to different patients in the doctor's system. In this way, the doctor will see in the first positions of their list those patients with higher risk/danger.

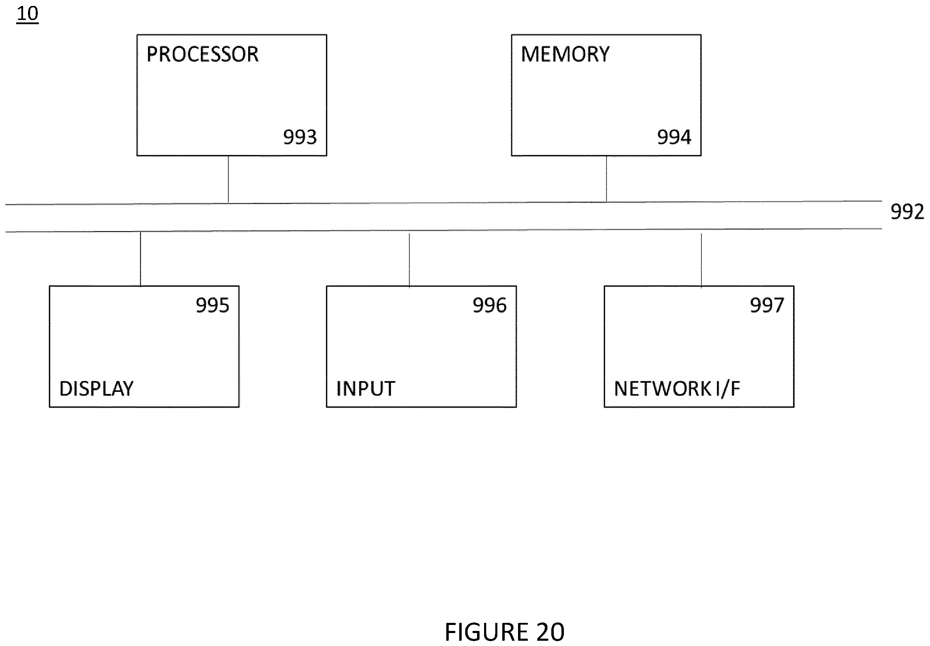

In an example, one side of the device 200 is the interface as described above and the other side is a magnetized surface. The device 200 comprises hardware which may be required for the appropriate working of the device: CPU, RAM and hard drive. The CPU will contain the basic logic to process data and visualize it in the corresponding output screens and alert light, and to process the orders connected from the interactive buttons with the data associated. RAM and hard drive are used to access and store the information needed/provided by this external device. Appropriate drivers/buses may be included to output information in screens, to handle the lights and to receive the input from the patients when buttons are pushed. And, network/WIFI drivers may be included to connect the IoT device to the internet and access to the patients' EHRs in the database of the hospital to receive/send the data. The apparatus illustrated in Figure 20 and described below may be considered to be an implementation of the device 200.

A device e.g. the device 200 may implement some or all of the method steps in Figure 2. A device e.g. the device 200 may implement some or all of the functions of the modules illustrated in Figure 1. Any information to be output as a result of methods described herein may be output to a different display means, e.g. a screen or a speaker, rather than or as well as to the device 200. The system 11 may not comprise the device 200. The system 11 may comprise at least one sensor for obtaining physiological measurements from the patient. The system 11 may comprise at least one sensor/user input device for obtaining other information from the patient such as symptom information and/or (family) medical history information, for example a microphone and/or a keyboard.

All of the functions of the system 11 may be carried out by a single device which is not necessarily the same as the device 200.

Example: genetic family history risk score determination

Family Medical History (FMH) Extractor 110

The FMH extractor 110 receives an input of a text paragraph about family antecedents. For this example, the family antecedents are the following:

*Her mother suffered breast cancer at age 31. Her father died of breast cancer at 81 age. Maternal grandmother died of breast cancer around 60 age. Maternal grandfather died at 75 of heart problems. Her maternal aunt had breast cancer at age 19. A female cousin was diagnosed of breast cancer at 21 age. Now she is healthy. Sister died of breast cancer at 27. Brother died from HIV/AIDS. The husband is alive and healthy. A child is healthy. Other son has autism. A daughter is healthy at 21 age.*

Using the approach described above, for example (step S20), the data extracted is:

1. Family member roles: mother, father, grandmother, grandfather, aunt, female cousin, sister, brother, husband, child, son, daughter.
2. Family member status: father->died, grandmother->died, grandfather->died, female cousin->healthy, sister->died, brother->died, husband->alive, healthy, child->healthy, daughter->healthy.
3. Family member side: grandmother->maternal, grandfather->maternal, aunt->maternal.
4. Family member age: mother->31, father->81, grandmother->60, grandfather->75, aunt->19, female cousin->21, sister->27, daughter->21.
5. Family member observations: mother->breast cancer, father->breast cancer, grandmother->breast cancer, grandfather->heart problems, aunt->breast cancer, female cousin->breast cancer, sister->breast cancer, brother->HIV/AIDS, son->autism.
6. Family member observation modality: mother->breast cancer->positive, father->breast cancer->positive, grandmother->breast cancer->positive, grandfather->heart problems->positive, aunt->breast cancer->positive, female cousin->breast cancer->positive, sister->breast cancer->positive, brother->HIV/AIDS->positive, son->autism->positive.

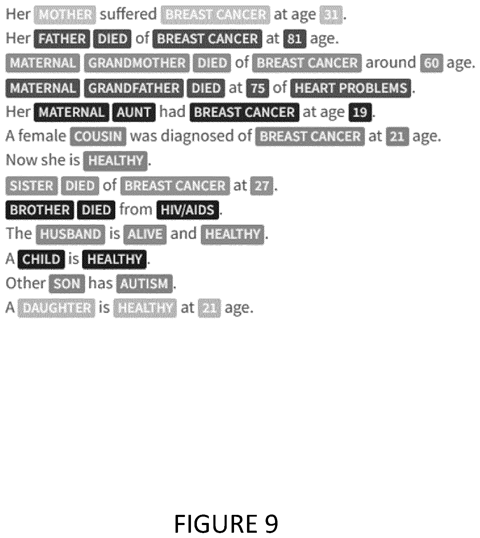

Figure 9 illustrates highlighted information to be extracted from the medical history information. The resultant information is output in JSON format:

NFO:root:{'info': [{'family_role': {'name': 'mother', 'mod': 'pos'}, 'family_age': ['31'], 'family_observation': [{'name': 'breast cancer', 'mod': 'pos'}]}, {'family_role': {'name': 'father', 'mod': 'pos'}, 'family_status': {'name': 'died', 'mod': 'pos'}, 'family_age': ['81'], 'family_observation': [{'name': 'breast cancer', 'mod': 'pos'}]}, {'family_role': {'name': 'grandmother', 'mod': 'pos'}, 'family_side': {'name': 'Maternal', 'mod': 'pos'}, 'family_status': {'name': 'died', 'mod': 'pos'}, 'family_age':

['60'], 'family_observation': [{'name': 'breast cancer', 'mod': 'pos'}]}, {'family_role': {'name': 'grandfather', 'mod': 'pos'}, 'family_side': {'name': 'Maternal', 'mod': 'pos'}, 'family_status': {'name': 'died', 'mod': 'pos'}, 'family_age': ['75'], 'family_observation': [{'name': 'heart problems', 'mod': 'pos'}]}, {'family_role': {'name': 'aunt', 'mod': 'pos'}, 'family_side': {'name': 'maternal', 'mod': 'pos'}, 'family_age': ['19'], 'family_observation': [{'name': 'breast cancer', 'mod': 'pos'}]}, {'family_role': {'name': 'cousin', 'mod': 'pos'}, 'family_status': {'name': 'healthy', 'mod': 'pos'}, 'family_age': ['21'], 'family_observation': [{'name': 'breast cancer', 'mod': 'pos'}]}, {'family_role': {'name': 'Sister', 'mod': 'pos'}, 'family_status': {'name': 'died', 'mod': 'pos'}, 'family_age': ['27'], 'family_observation': [{'name': 'breast cancer', 'mod': 'pos'}]}, {'family_role': {'name': 'Brother', 'mod': 'pos'}, 'family_status': {'name': 'died', 'mod': 'pos'}, 'family_observation': [{'name': 'HIV/AIDS', 'mod': 'pos'}]}, {'family_role': {'name': 'husband', 'mod': 'pos'}, 'family_status': {'name': 'alive', 'mod': 'pos'}}, {'family_role': {'name': 'child', 'mod': 'pos'}, 'family_status': {'name': 'healthy', 'mod': 'pos'}}, {'family_role': {'name': 'son', 'mod': 'pos'}, 'family_observation': [{'name': 'autism', 'mod': 'pos'}]}, {'family_role': {'name': 'daughter', 'mod': 'pos'}, 'family_status': {'name': 'healthy', 'mod': 'pos'}, 'family_age': ['21']}], 'text': 'Her mother suffered breast cancer at age 31.\nHer father died of breast cancer at 81 age.\nMaternal grandmother died of breast cancer around 60 age.\nMaternal grandfather died at 75 of heart problems.\nHer maternal aunt had breast cancer at age 19.\nA female cousin was diagnosed of breast cancer at 21 age.\nNow she is healthy.\nSister died of breast cancer at 27.\nBrother died from HIV/AIDS.\nThe husband is alive and healthy.\nA child is healthy.\nOther son has autism.\nA daughter is healthy at 21 age.'}

Family History Data Transformer 120

Inputs: JSON data above; the gender of patient (0=female); the age of patient (e.g., 26); and one observation/condition under consideration (breast cancer).

Rule-based methods are applied to iterate over the JSON data and get the relevant features from relatives needed for the risk calculation, as described above. Such features will be ['ID', 'Gender', 'Age', 'Status, 'FatherID', 'MotherID', 'Affected']:

- ID: Identifier of the member (alphanumeric value)
- Gender: The gender of the member (0=female, 1=male, "=undefined)
- Age: The age of the member
- Status: The status of the member (0=dead, 1=alive)
- FatherID: Identifier of the member's father (alphanumeric value corresponding with ID of other member in the list of relatives. In case that member's father is not in the list, the value is 0 by default)
- MotherID: Identifier of the member's mother (alphanumeric value corresponding with ID of other member in the list of relatives. In case that member's mother is not in the list, the value is 0 by default)
- Affected: This feature indicates if the member is affected by the condition under consideration (1=True, i.e., member is affected; 0=False, i.e., member is not affected)

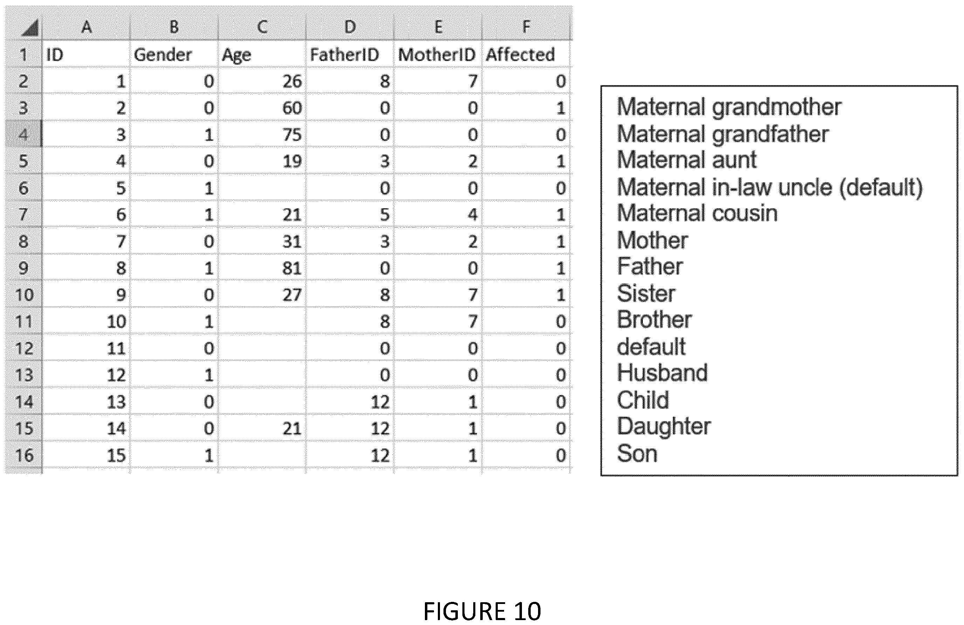

Figure 10 is an example of CSV file for these family antecedents (without Status column). The first row is patient's data [female patient of 26 years old].

Health Risk Calculation Module 140 - Input: CSV file.

• Pedigree Type Classifier 142

From the CSV file the following information is used: data of the ID, Gender, FatherID, MotherID and Affected. Applying rules defining inheritance modes, the inheritance mode is filtered in a discriminative way as described above:

- Y-linked is discarded because there are females affected in the family.
- X-linked recessive is discarded because two affected parents (like is the case of the father and the mother) would always have their children affected, and this is not the case in the family.
- Autosomal recessive is discarded for the same reason as X-linked recessive.
- X-linked dominant is discarded because affected fathers pass to all the daughters, and this is not the case with our patient.

In this example, therefore, the only possible inheritance pattern/mode in this family for breast cancer condition is the mode of AUTOSOMAL_DOMINANT.

• Family Genotypes Estimator 144

Genotypes are assigned for the family members based on the pedigree inheritance type (**AUTOSOMAL_DOMINANT**) and based on whether or not the family member is known to have or have had the condition (affected or not).

Inputs: The pedigree inheritance type (**AUTOSOMAL_DOMINANT**); from the CSV file, for each family member: the affected value, the list of affected values of parents and the list of affected values of children. Knowing the pedigree inheritance type, the Gender and Affected value of each member, the rules defining the inheritance mode are used to assign a genotype to each family member. The possible genotypes that may be assigned in the case of each inheritance mode are shown below.

- Autosomal dominant: AA = affected; Aa = affected; aA= affected; aa= normal
- Autosomal recessive: aa = affected; Aa = carrier (appears normal); AA = normal; A? = appears normal (one allele unknown)
- X-linked dominant: XX= affected; Xx= affected; Xy= affected; xx= normal; xy=normal
- X-linked recessive: xx=affected; Xx= carrier (appears normal); XX=normal; xy=affected; Xy=normal

• Y-linked: xY= affected; xy=normal

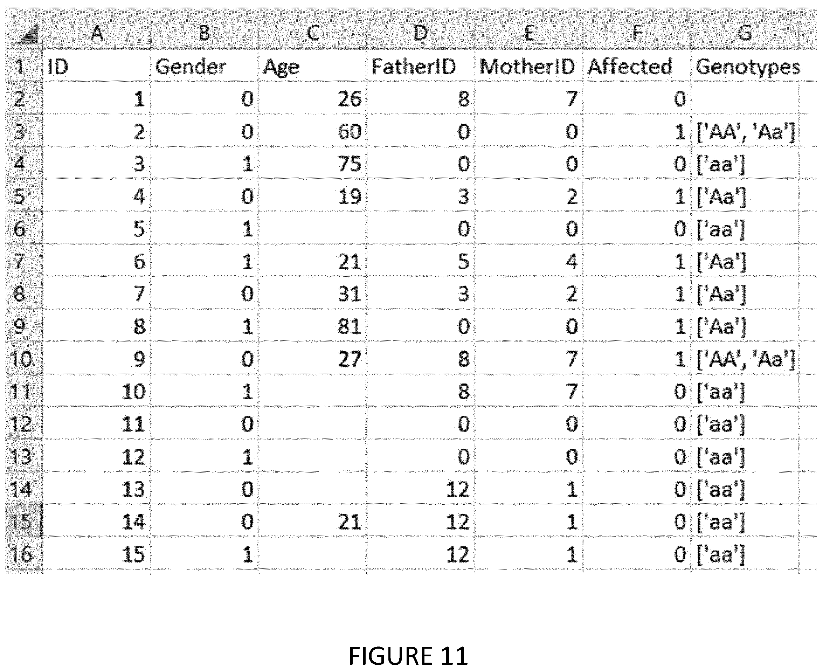

A list of genotypes for each family member is returned. The CSV file is updated to include a new column 'Genotypes' with this new information. The CSV file with the Genotypes column for the current example is shown in Figure 11.

• Mendelian/Bayes Risk Scoring Module 146

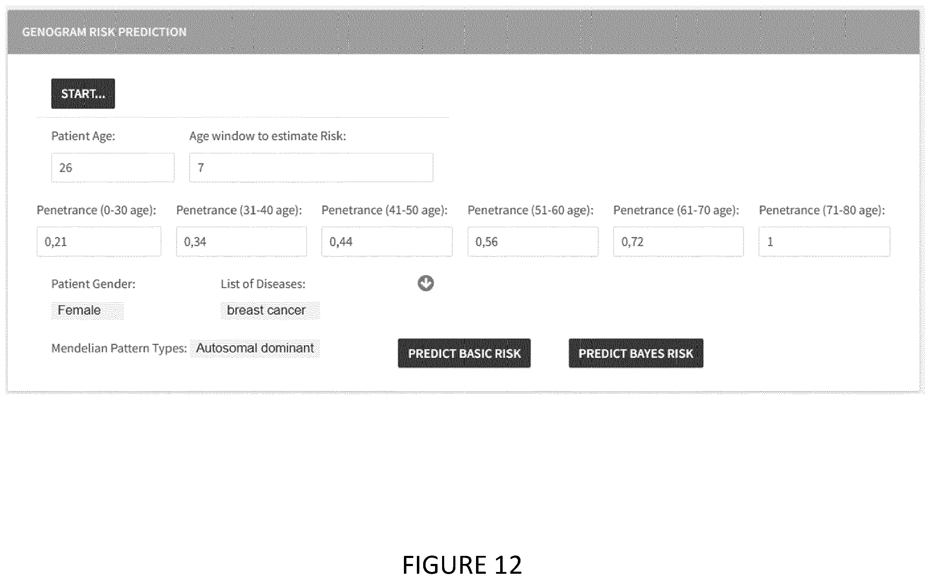

Inputs: Data from the CSV file (in this step the following is relevant for each family member: the age, FatherID, MotherID and the genotype(s)); the pedigree inheritance type/inheritance mode (**AUTOSOMAL_DOMINANT**); age window to estimate the risk (e.g., 7 years); and penetrance values of the disease depending on age ranges. Figure 12 illustrates an example interface for a user to cause a computer to calculate a genetic family history risk score and shows some example penetrance values for different age ranges for breast cancer.

In the calculation of the Mendelian/basic genetic risk score, a Punnett Squares approach is applied, combining the genotypes from the patient's father and mother and obtaining probabilities of being affected considering the inheritance mode of AUTOSOMAL_DOMINANT.

- Mother's genotypes: ID=7, ['Aa']
- Father's genotypes: ID=8, ['Aa']

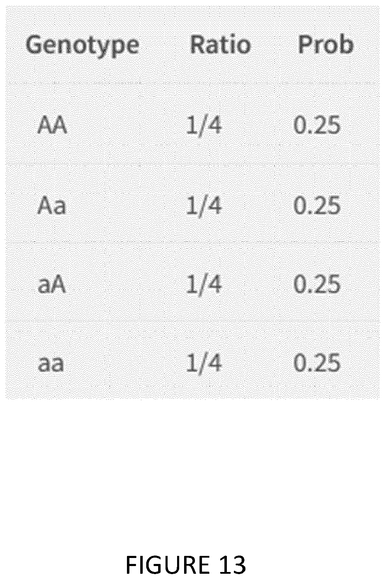

Figure 13 illustrates the result of applying the Punnett Squares approach in this example. In the AUTOSOMAL_DOMINANT inheritance mode, all genotypes with at least one capital letter [AA, Aa, aA] are considered to cause the patient to be affected by the condition, therefore in this example the sum of the probabilities of such cases (cases in which the patient is considered to be affected) gives the following basic genetic risk score: 75% (0.25 + 0.25 + 0.25) * 100).

In the calculation of the Bayes genetic risk score, the same Punnett Squares approach is applied over the genotypes of the patient's father and mother in the same way as for the basic genetic risk score calculation. The probability for the patient to have each genotype is summed to obtain prior probabilities: Prior probabilities of each genotype = {'AA': 0.25, 'Aa': 0.50, 'aa': 0.25}

Next, the genotypes of the patient's children and the other parent are taken into account. The Punnett Squares approach is applied over the genotypes of the patient and the other parent to determine genotypes possibilities for the children. Conditional probabilities are obtained for the assigned genotype of each child conditional on the different possible genotypes of the patient.

- Partner's genotypes: ID=12, ['aa']

Example code for applying Punnett Squares approach:

```
child_probs = general_punnet_squares(partner_genotypes, ['Aa'])
child_probs_2 = general_punnet_squares(partner_genotypes, ['AA'])
child_probs_3 = general_punnet_squares(partner_genotypes, ['aa'])
```

A multiplicative sum is applied for each genotype possibility of the patient based on the conditional probabilities and the number of children (3 children in this example). For instance, for genotype Aa (analogous in the other cases) some example code is:

```
conditional['Aa'] = child_probs['Aa']
  for n in range(1, number_children):
    conditional['Aa'] = conditional['Aa'] * child_probs['Aa']
```

After these calculations, in this running example the conditional probabilities of obtaining the assigned genotypes of the children conditional on the patient's possible genotypes are:

patient genotype = 'AA': 1.0
patient genotype = 'Aa': 0.125
patient genotype = 'aa': 1.0

Then, the cross product is calculated over the combinative matrix of prior probabilities and conditional probabilities, obtaining joint probabilities. An example of the code for this step is:

for (p_genotype, p_probability), (c_genotype, c_probability) in zip(prior.items(), conditional.items()):

$$joint[p\_genotype] = p\_probability * c\_probability$$

In this running example, the joint probabilities are: {'AA': 0.25, 'Aa': 0.0625, 'aa': 0.25}

Finally, Bayes algorithm/theorem is applied for every genotype possibility, obtaining the probability of the patient possessing a genotype assumed to cause them to be affected by the condition for the AUTOSOMAL_DOMINANT type. An example of the code used for this step is:

```
denominator = Fraction()
   for genotype, probability in joint.items():
     denominator = denominator + probability
   for genotype, probability in joint.items():
     final_prob[genotype] = probability / denominator
```

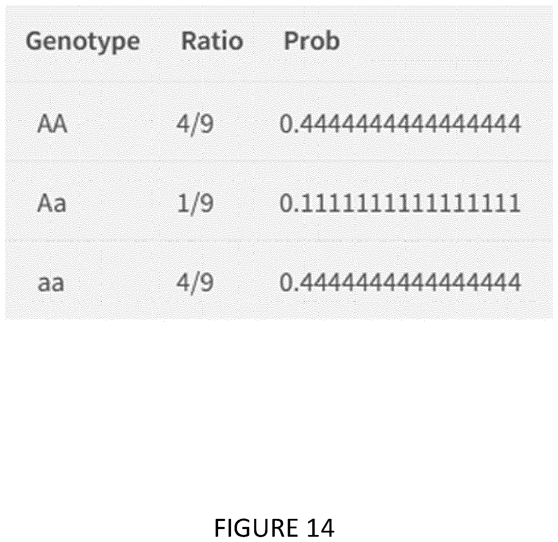

Figure 14 shows the results of the application of Bayes theorem in this running example, i.e. the probability of the patient having each possible genotype based on the genotypes of their parents, their children, and the children's other parent. For AUTOSOMAL_DOMINANT type, the Bayes genetic risk score is determined by summing the probabilities of genotypes with at least one capital letter [AA, Aa, aA] (the ones that are considered to cause the patient to be affected), and the following Bayes genetic risk score is determined in this running example: 55.56% (0.44444... + 0.11......) * 100).

Since in AUTOSOMAL_DOMINANT type there is not possibility of an individual being a carrier without being considered to be affected by the condition it is not possible to determine a carrier risk in this case.

The age of the patient and the age window or projection age (i.e., number of years in future when we want to estimate the risk) are summed to obtain the predictive age.

$$26 \text{ years old} + 7 \text{ years of age window} = 33 \text{ years old}$$

The penetrance value associated to that age range that is obtained and in this example is 0.34 (referring to Figure 12). This penetrance value will be used in the determination of the genetic family history risk score.

The danger factor or family history risk score is determined. The first contribution which considers relatives that are known to have or have had the disease and are not known to have died from the disease:

$$f1 = w'1 * n_{1st} + w'2 * n_{2nd} + w'3 * n_{3rd}$$

We define empirical weight values: w'1 = 0.55; w'2 = 0.30; w'3 = 0.15, considering that first degree level relatives are the most important for these calculations. Therefore in this running example, using the information in the CSV file, the first contribution is:

$$f1 = 0.55 * 1 \text{ (mother)} + 0.30 * 1 \text{ (aunt)} + 0.15 * 1 \text{ (cousin)} = 1$$

The second contribution which considers relatives who are known to have died from the disease:

$$f2 = w1 * m_{1st} + w2 * m_{2nd} + w3 * m_{3rd}$$

In this example the secondary weighting factors have the same values as the primary weighting factors and therefore f2 = 0.55 * 2 (father,sister) + 0.30 * 1 (grandmother) + 0.15 * 0 = 1.4

The third contribution based on age of relatives:

$$cond1 = w1 \sum_{i=1}^{n1st} \left(1 - \frac{age_i}{100}\right) + w2 \sum_{j=1}^{n2nd} \left(1 - \frac{age_j}{100}\right) + w3 \sum_{k=1}^{n3rd} \left(1 - \frac{age_k}{100}\right)$$

$$cond1 = 0.55 \sum_{i=1}^{1(mother)} \left(1 - \frac{31}{100}\right) + 0.30 \sum_{j=1}^{1(aunt)} \left(1 - \frac{19}{100}\right) + 0.15 \sum_{k=1}^{1(cousin)} \left(1 - \frac{21}{100}\right)$$

$$= 0.741$$

$$cond2 = w1 \sum_{i=1}^{m1st} \left(1 - \frac{age_i}{100}\right) + w2 \sum_{j=1}^{m2nd} \left(1 - \frac{age_j}{100}\right) + w3 \sum_{k=1}^{m3rd} \left(1 - \frac{age_k}{100}\right)$$

$$cond2 = 0.55 * \left(\left(1 - \frac{81}{100}\right) + \left(1 - \frac{27}{100}\right)\right) + 0.30 * \left(\left(1 - \frac{60}{100}\right)\right) + 0.15 * 0 = 0.626$$

$$f3 = wn * cond1 + wm * cond2$$

In this running example empirical weight values are defined: wn=0.40, wm=0.60, considering that cases resulting in death may be more dangerous for the risk of the patient. Therefore in this running example:

$$f3 = 0.40 * 0.741 + 0.60 * 0.626 = 0.672$$

Final Danger Factor or family history risk score is:

$$DF = \alpha * f1 + \beta * f2 + \gamma * f3$$

In this example empirical coefficients of α=0.15, β=0.30, γ=0.55 are set, giving more relevance to the third contribution. Therefore in this example:

$$DF = 0.15 * 1 + 0.30 * 1.4 + 0.55 * 0.672 = 0.9396$$

Therefore, in this example the genetic family history risk score (the basic genetic family history risk score and the Bayes genetic family history risk score) is:

$$Genetic\ family\ history\ risk\ score = Initial\ (Basic\ or\ Bayes)\ Risk * Penetrance * DF$$

$$Basic\ genetic\ family\ history\ risk\ score = 0.75 * 0.34 * 0.9396 = 0.24 = 24\%$$

$$Bayes\ genetic\ family\ history\ risk\ score = 0.56 * 0.34 * 0.9396 = 0.18 = 18\%$$

In the running example, multiplication has been used in the calculation of the genetic family history risk score. In other examples, summing may be used (i.e. genetic risk score * penetrance + DF).

Integrator module 160 and updater module 180

Continuing the running example, the updater module 180 connects to the patient's EHR in the hospital's database to upload and integrate the determined risk information in the records of the patient. The updater module 180 runs in the background and monitors if new information about patient's family antecedents appear (i.e. new family members' information e.g. in family members' EHRs). If there is new family member information the module will cause the above risk score determination processes to be repeated taking account of the new information. The updater module 180 may also include reasons for the genetic family history risk score determination in the updates sent to the patient EHR. In this running example, the explanations/reasons are:

Basic genetic family history risk score:

*You are (or the patient is) at 24% risk of running the condition in the future.*
*The result of 24% risk is based on the following considerations:*

- *The family pedigree follows AUTOSOMAL_DOMINANT mendelian pattern. From such pattern, we can estimate the genotypes ['Aa'] for the mother and the genotypes ['Aa'] for the father.*
- *Then, we combine the genotypes from mother and father following Punnett Squares approach and we obtain the above table with the probabilities of each genotype for the patient.*
- *Since the mendelian pattern is AUTOSOMAL_DOMINANT, all genotypes with at least one capital letter (e.g. 'Aa') will be affected by condition. Therefore, aggregating probabilities of genotypes that could be affected by the condition, we obtain an initial risk of 75.0.*
- *But, as the patient age is 26 years old, and we apply an age window of 7, the risk estimation would be for 33 years old, with a penetrance of 0.34.*
- *The inclusion of penetrance and Danger Factor (75.0 * 0.34 * 0.9396) returns the outputted risk of 24%.*

Bayes Risk Prediction:

*You are (or the patient is) at 18% risk of running the condition in the future.*
*The result of 18% risk is coming from next considerations:*

- *The family pedigree follows AUTOSOMAL_DOMINANT mendelian pattern. From such pattern, we can estimate the genotypes ['Aa'] for the mother and the genotypes ['Aa'] for the father*
- *Then, we combine the genotypes from mother and father following Punnett Squares approach and we obtain the prior probabilities of each genotype, {AA': Fraction(1, 4), 'Aa': Fraction(1, 2), 'aa': Fraction(1, 4)}.*
- *Since the children are healthy, we apply Punnett Squares approach between all genotypes possibilities and the genotypes of the other progenitor (['aa']), obtaining conditional probabilities based on the 3 children, {AA': Fraction(1, 1), 'Aa': Fraction(1, 8), 'aa': Fraction(1, 1)}.*
- *Next, we combine prior and conditional probabilities obtaining {AA': Fraction(1, 4), 'Aa': Fraction(1, 16), 'aa': Fraction(1, 4)}, and we execute Bayes approach over such combination taking into account each genotype possibility, what returns the above table of probabilities.*
- *Since the mendelian pattern is AUTOSOMAL_DOMINANT, all genotypes with at least one capital letter (e.g. 'Aa') will be affected by condition. Therefore, aggregating probabilities of genotypes that could be affected by the condition, we obtain an initial risk of 55.56.*
- *But, as the patient age is 26 years old, and we apply an age window of 7, the risk estimation would be for 33 years old, with a penetrance of 0.34.*

- *The inclusion of penetrance and Danger Factor (55.56 \* 0.34 \* 0.9396) returns the outputted risk of 18%.*

The reasons may be determined in the integrator module 160 or in the updater module 180.

The integrator module transmits the results obtained to the external device associated to the patient. The information to send in this example is:

- The list of diseases relevant in the family: breast cancer
- For each disease, the basic genetic family history risk score: breast cancer -> 24%
- For each disease, the Bayes genetic family history risk score : breast cancer -> 18%
- For each disease, the flag risk colour associated to patient's risk

For the flag risk colour assignation, the average between the basic and the Bayes genetic family history risk score is determined.

$$\frac{(24 + 18)}{2} = 21\%$$

The colour of the corresponding category is assigned, following the Alert Colour System definition described above. In this case, for the 21% risk the colour assigned is Soft Yellow.

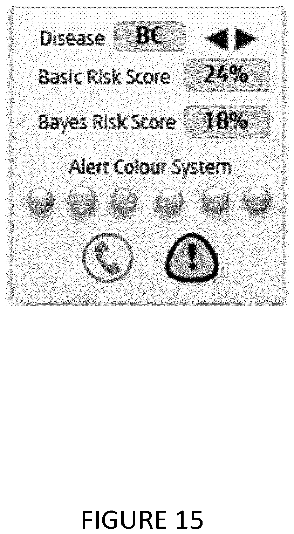

The device 200 receives this information and connects the data of the flag colour with the appropriate light driver and shows/displays in the small output screens the rest of the outputted information. Figure 15 illustrates an example interface of the device 200 for this running example (the light colour is not shown in Figure 15).

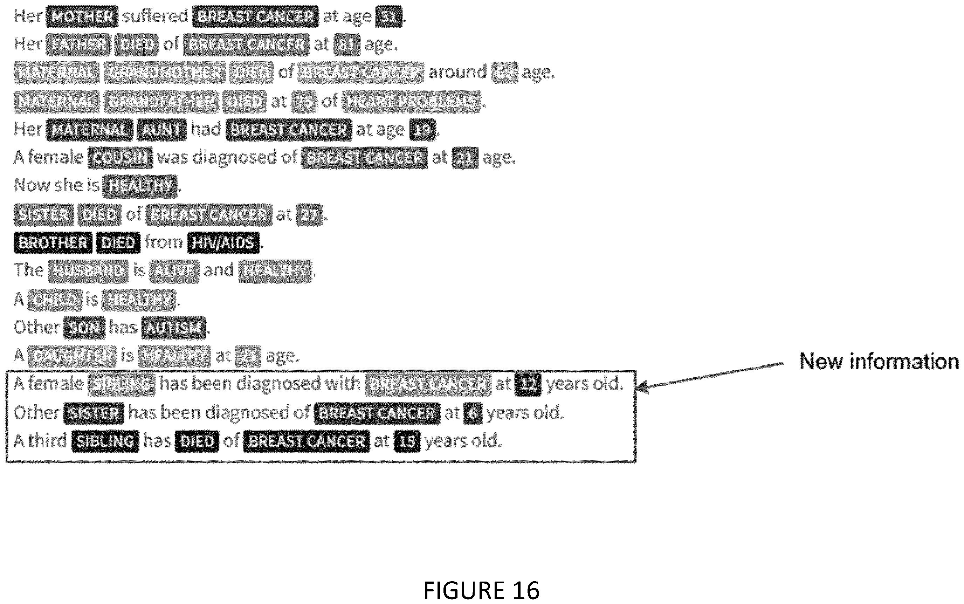

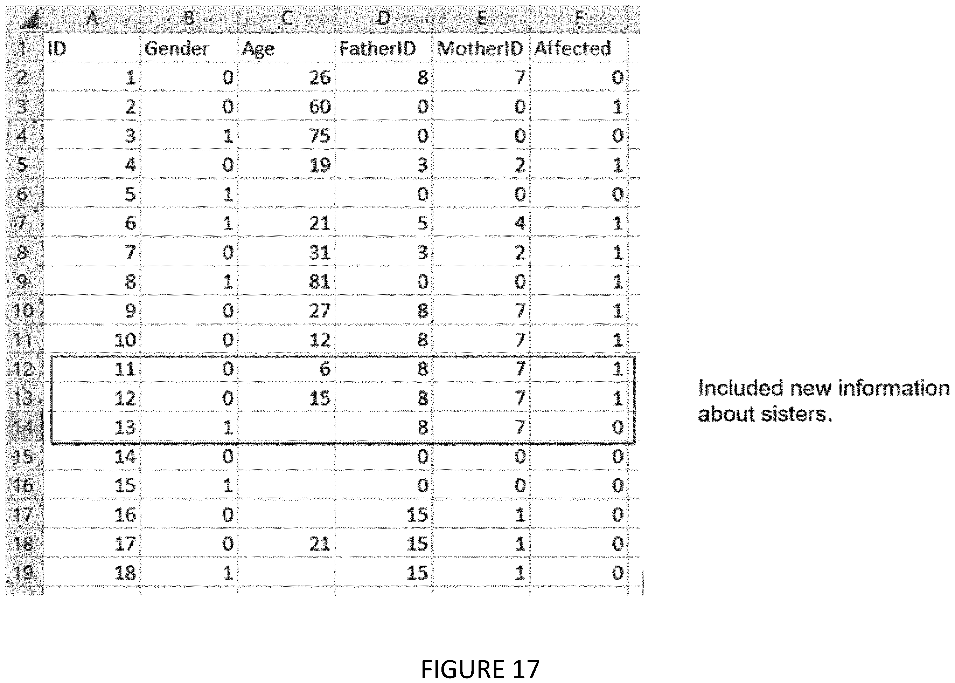

Next, a continuation of the above running example is described in which new information from patient's relatives is received. In this example two patient's sisters have been diagnosed with breast cancer and a third sister has died of breast cancer. The updater 180 detects this new information and causes the above processes to be repeated. The new information to be extracted is illustrated in Figure 16. Figure 17 illustrates the new CSV file including the new extracted information.

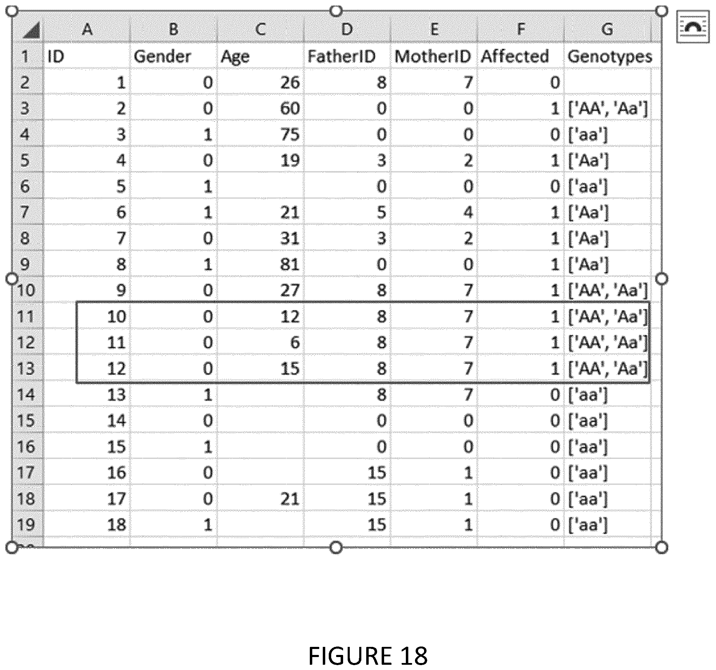

The inheritance mode is determined again to be AUTOSOMAL_DOMINANT. When new information is detected, in some implementations when the processes are repeated the inheritance mode may not be re-determined and may be assumed the same as before. The genotypes of the family members are determined and assigned again and the results are illustrated in Figure 18 in this running example. The Basic and Bayes genetic risk score are not affected by the new information in this example and are still:

Basic Risk model: 75%
Bayes Risk model: 55.56%

The method may include determining whether or not the new information has resulted in different assigned genotypes for the patient, their children, or the children's other parent(s) and if so the genetic family history risk score may be recalculated and otherwise the genetic family history risk score may not be recalculated.

In this example the Danger Factor is calculated again taking account of the new information.

First contribution (using the same primary weighting factors as before):

f1 = 0.55 \* 3 (mother,sister,sister) + 0.30 \* 1 (aunt) + 0.15 \* 1 (cousin) = 2.1

Second contribution (using the same secondary weighting factors as before the new information):

f2 = 0.55 \* 3 (father,sister,sister) + 0.30 \* 1 (grandmother) + 0.15 \* 0 = 1.95

Third contribution ((using the same weighting factors as before the new information):

$$cond1 = 0.55\left(\left(1 - \frac{31}{100}\right)_{mother} + \left(1 - \frac{12}{100}\right)_{sister} + +\left(1 - \frac{6}{100}\right)_{sister}\right)$$

$$+ 0.30 \sum_{j=1}^{1(aunt)} \left(1 - \frac{19}{100}\right) + 0.15 \sum_{k=1}^{1(cousin)} \left(1 - \frac{21}{100}\right) = 1.742$$

$$cond2 = 0.55 * \left(\left(1 - \frac{81}{100}\right) + \left(1 - \frac{27}{100}\right) + \left(1 - \frac{15}{100}\right)\right) + 0.30 * \left(\left(1 - \frac{60}{100}\right)\right) + 0.15$$

$$* 0 = 1.0935$$

$$f3 = 0.40*1.742 + 0.60*1.0935 = 1.3529$$

Family history risk score (using the same weighting factors as before the new information):

$$DF = 0.15 * 2.1 + 0.30 * 1.95 + 0.55 * 1.3529 = 1.644095$$

Therefore, the genetic family history risk scores (Basic and Bayes) using the new information are:

Basic genetic family history risk score = 0.75 * 0.34 * 1.644095 = 0.42 = 42%

Bayes genetic family history risk score = 0.56 * 0.34 * 1.644095 = 0.31 = 31%

Due to the new information there is a notable increase for this patient in the determined risk of being affected by breast cancer disease (genetic family history risk score) in both models (Basic: from 24% to 42%; Bayes: from 18% to 31%). This is reasonable considering the new information of two sisters diagnosed by breast cancer at younger ages and a third one dead by breast cancer at younger age.

Something to highlight in this example is that initial statistical models based on genetics (i.e. the genetic risk scores) did not reflect the increased risk to the patient due to this new information and only the danger factor or family history risk score was determinant in the increasing of patient's risk.

For the flag risk colour assignation, the average between the basic genetic family history risk score and the Bayes genetic family history risk score is calculated:

$$((42+31))/2 = 36.5\%$$

The colour of the corresponding category is assigned, following the Alert Colour System described above. In this case, for the 36.5% risk the colour assigned is Strong Yellow. This new information about the patient's risk will be sent to the device 200 for showing the updates.

Magnet IoT Device Interaction

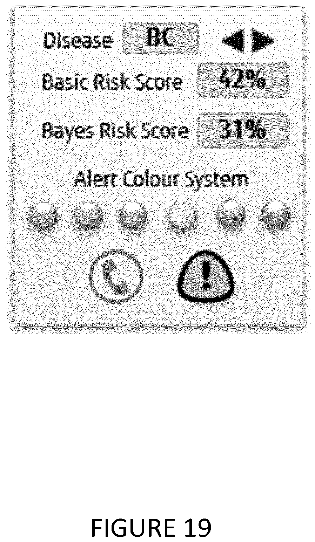

Figure 19 illustrates an example of the device 200 showing updated risk information based on the new information that was used in the repeated genetic family history risk score determination.

After the latest updates in patient's relatives' information, the light of the Alert Colour System for the Breast Cancer disease changed from Soft Yellow to Strong Yellow. This could increase the concerns of the patient. They may wish to contact and call their primary care doctor. If this is the case, the patient only needs to push interactive button with a picture of a phone. After pushing, a direct call may be connected to the doctor. Summary information of this patient will appear instantly in the hospital's application on the doctor's computer, for example (and may appear only if the doctor takes the call). For this example, the application will show for breast cancer disease the genetic family history risk scores of the two models, the flag alert colour associated, and the explainability information included previously about the workflow and consecution of results (this may be accessible only to the doctor or may also/alternatively be accessible to the patient). From this call and the patient's summary information, the doctor may take the appropriate actions.

In case the doctor is not able to take the call, the patient could send a warning message by pushing the button with the exclamation mark. If this is done, a warning alert may be forwarded to the patient's EHR and this change may be visible in the hospital's application on doctor's computer. In such an application, the doctor's list of consultation may be modified to assign higher priority to this patient since their flag alert is in Strong Yellow and was increased recently. When the doctor opens this application, the updates on the consultation list will be highlighted and summary information of patients relating e,g, to risk may be visualized in pop-up windows to get the doctor's attention. All these mechanisms may support the doctor in the analysis of patient's situation to make decisions and take appropriate actions.

As mentioned above, the information shown by the device 200 and/or used to update the patient EHR and/or sent to the doctor may comprise (optionally or additionally) diagnosis information. The diagnosis information may comprise a final diagnosis and the associated genetic family history risk score(s) as described above.

The aspects disclosed herein may be integrated as a plugin inside existing current frameworks or integrated within a bigger clinical decision support system for healthcare applications.

Some advantages of the aspects disclosed herein include:

- Real-time updates of patients' health risks
- IoT device with direct communication between patient, EHR and physician
- Integration of alert system of health risks in a device, easily understandable to reach all patients
- Multilingual capabilities
- Improved diagnosis
- More accurate diagnosis
- Diagnosis that takes into account wide variety of information including any of predicted genetic information of patient and family members, medical history of patient and family members, physiological measurements of patient, and symptoms of patient. The wide variety of information improves the reliability and/or accuracy of the diagnosis and/or results in a more robust diagnosis. Furthermore the genetic information is predicted so that there is no need to obtain such information from tests.

Some aspects disclosed herein include the following:

• An ad-hoc external magnet IoT device design that provides alert information of patient's health risks based on family antecedents in an easy and understandable way, showing such information in accepted common way to reach all the patients. This may include:

a. Interface that shows relevant information to patients, capable of processing and adapting data from central databases and generated by the disclosed system to be visualized in the external device. Such interface is also capable of processing interactive inputs from the patients to modify information and forward these changes to patient's profile in the EHRs of the hospital's database. Such interactions may include (i) direct calls to the doctor, sending in that moment relevant information to appear directly in the application that doctor is running without the need of specific queries or searches by the doctor; (ii) send of warning signs by patient that will modify the patient's EHR and the consultation application of the doctor to provide prioritization and special alert emphasis.

b. Magnetized surface to allow users to stick the device in a visible place of the house, such as fridge, to maintain use at home the device in all moments.

c. Hardware architecture that handles and process all the data, interactions, and connection to share information between this external device and central databases such as patients' EHRs.

• An automated patient's health risk prediction system that processes the information of family medical history automatically from unstructured clinical reports in textual format with deep learning techniques in a multilingual environment and provides risk probabilities for the patient of being affected by certain disease or being carrier of such disease. The architecture system comprising:

- a pedigree type classifier to estimate the inheritance pattern of the family.
- a family genotype estimator engine to estimate the potential genotypes of patient's relatives based on the inheritance pattern.
- a mendelian/bayes risk scoring engine that is capable of calculating the patient's health risks for 2 models based on statistical models, patient and relatives' information, penetrance values on age ranges for the disease and the Danger Factor. The Danger Factor score will be based on features such as number of relatives alive that suffered the condition, number of relatives dead by the condition and equations considering relatives' ages when suffering the condition or dead by the condition, including discrimination by degree level of relatives for all the features.

• A risk score and alert system integrator module that is capable of connecting to patient's EHR in the hospital's database and provides real-time updates in risk scoring through the monitoring of modifications in patient's family information. This module is also capable of linking and adapting the risk scoring with light semaphore in the external device to represent an alert colour system in danger categories for the patients depending on their health risk probabilities.

• In the automated patient's health risk prediction system the extraction of information from patient's family may follow multilingual capabilities for any unstructured textual document.

• In the risk score and alert system integrator module the alert colour warning system for patient's health risk may follow a categorization of 6 colours (strong green, soft green, soft yellow, strong yellow, soft red, strong red) simulating the common understanding of a semaphore to make it understandable to all patients.

• In the alert colour warning system the design for linking patient's health risk probabilities with the 6 colours' categories may follow an average calculation of basic and Bayes genetic family history risk scores and a definition of six probability ranges for each colour:

a) Strong Green    Probabilities from 0% to 9.99%
b) Soft Green    Probabilities from 10% to 19.99%
c) Soft Yellow    Probabilities from 20% to 34.99%
d) Strong Yellow    Probabilities from 35% to 49.99%
e) Soft Red    Probabilities from 50% to 64.99%
f) Strong Red    Probabilities from 65% to 100%,

stipulating that closest to Strong Green is less risky and dangerous for patient, while increasing towards Strong Red increases the health risk and danger for patient of suffering certain disease.

• In the risk score and alert system integrator module explainability descriptions may be included in the patient's EHR to provide coarse-grained understanding of the workflow in the risk calculation methodology and intermediate results to the doctor.

• The external magnet IoT device may include any of the features:

a) (Small) output screen to show the name of the disease of interest.
b) Interactive directional buttons so that the patient can push such buttons to go forward/backward over their list of diseases.
c) (Small) output screens to show the risk probabilities in percentage of being affected by the disease (genetic family history risk score) selected for the two models (Basic Mendelian model and Bayes algorithm model).
d) Lights to show in understandable way the risk of the patient following a kind of semaphore signals to warn of the risk in real-time to the patient.
e) Interactive call button: When the patient pushes this button a direct call with their primary care doctor will be established, and, in the EHR system will appear automatically for the doctor the summary information of the patient for the disease selected in the external device (the health risks probabilities, the flag light associated to patient's risk, and, the explainability reasons of the risk values only accessible for the doctor). In this way, if the patient wants to call the doctor because of concerns if the health risk is increasing, the doctor will see directly the relevant records of the patient related to such call without the need of doing specific queries or searches in the system.
f) Interactive warning button: When the patient pushes this button a warning message will be forwarded to their EHRs, updating the relevant information related to this warning in the records. The system will send alert signal to their primary care doctor in the doctor's application, updating the doctor's list of consultation by assigning higher priority depending on the risk flag light of the patient. In this way, the doctor will see in the first positions of the list those patients with higher risk/danger prioritized by the system. Therefore, the doctor may use such prioritized list and patients' health risks summary information with explainability as decision support for taking the appropriate actions as soon as possible.

Figure 20 is a block diagram of an information processing apparatus 10 or a computing device 10, such as a data storage server, which embodies the present invention, and which may be used to implement some or all of the operations of a method embodying the present invention, and perform some or all of the tasks of apparatus of an embodiment. The computing device 10 may be used to implement any of the method steps described above, e.g. any of S20-S90 in Figure 2. The apparatus 10 may serve as the system 11 excluding the device 200 or may serve as the device 200. The apparatus 10 may serve as the system which is configured to display information without use of the device 200 as described above.

The computing device 10 comprises a processor 993 and memory 994. Optionally, the computing device also includes a network interface 997 for communication with other such computing devices, for example with other computing devices of invention embodiments. Optionally, the computing device also includes one or more input mechanisms such as keyboard and mouse 996, and a display unit such as one or more monitors 995. These elements may facilitate user interaction. The components are connectable to one another via a bus 992.

The memory 994 may include a computer readable medium, which term may refer to a single medium or multiple media (e.g., a centralized or distributed database and/or associated caches and servers) configured to carry computer-executable instructions. Computer-executable instructions may include, for example, instructions and data accessible by and causing a general purpose computer, special purpose computer, or special purpose processing device (e.g., one or more processors) to perform one or more functions or operations. For example, the computer-executable instructions may include those instructions for implementing a method disclosed herein, or any method steps disclosed herein, for example any of S20-S90 in Figure 2. Thus, the term "computer-readable storage medium" may also include any medium that is capable of storing, encoding or carrying a set of instructions for execution by the machine and that cause the machine to perform any one or more of the method steps of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing computer program code stored in the memory 994 to implement any of the method steps described herein. The memory 994 stores data being read and written by the processor 993 and may store unstructured (medical history) data and/or unstructured (medical history) training data and/or any of the extracted family history information and/or extracted medical (history) information and/or input data and/or any rules described above and/or any of the determined information above such as values and genotypes and inheritance modes, as described above, and/or programs for executing any of the method steps described above, e.g. any of S20-S90 in Figure 2, and/or any of the rule-based algorithms/processes described above and/or any calculations described above. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and operations discussed herein. The processor 993 may be considered to comprise/implement any of the modules described above. Any operation(s) described as being implemented by one or more modules may be implemented as a method by a computer and e.g. by the processor 993.

The display unit 995 may display a representation of data stored by the computing device, such as any output described above, for example any of at least one final diagnosis, genetic family history risk score(s), conditions/diseases, a light indicating a genetic family history risk score(s) or a genetic risk score(s), and may also display a cursor and dialog boxes and screens enabling interaction between a user and the programs and data stored on the computing device. The input mechanisms 996 may enable a user to input data and instructions to the computing device, such as enabling a user to input any user input described above.

The network interface (network I/F) 997 may be connected to a network, such as the Internet, and is connectable to other such computing devices via the network. The network I/F 997 may control data input/output from/to other apparatus via the network. Other peripheral devices such as microphone, speakers, printer, power supply unit, fan, case, scanner, trackerball etc may be included in the computing device.

Methods embodying the present invention may be carried out on a computing device/apparatus 10 such as that illustrated in Figure 20. Such a computing device need not have every component illustrated in Figure 20, and may be composed of a subset of those components. For example, the apparatus 10 may comprise the processor 993 and the memory 994 connected to the processor 993. Or the apparatus 10 may comprise the processor 993, the memory 994 connected to the processor 993, and the display 995. A method embodying the present invention may be carried out by a single computing device in communication with one or more data storage servers via a network. The computing device may be a data storage itself storing at least a portion of the data.

A method embodying the present invention may be carried out by a plurality of computing devices operating in cooperation with one another. One or more of the plurality of computing devices may be a data storage server storing at least a portion of the data. For example a computing device may implement the device 200 and another computing device may implement the remaining components of the system 11.

The invention may be implemented in digital electronic circuitry, or in computer hardware, firmware, software, or in combinations of them. The invention may be implemented as a computer program or computer program product, i.e., a computer program tangibly embodied in a non-transitory information carrier, e.g., in a machine-readable storage device, or in a propagated signal, for execution by, or to control the operation of, one or more hardware modules.

A computer program may be in the form of a stand-alone program, a computer program portion or more than one computer program and may be written in any form of programming language, including compiled or interpreted languages, and it may be deployed in any form, including as a stand-alone program or as a module, component, subroutine, or other unit suitable for use in a data processing environment. A computer program may be deployed to be executed on one module or on multiple modules at one site or distributed across multiple sites and interconnected by a communication network.

Method steps of the invention may be performed by one or more programmable processors executing a computer program to perform functions of the invention by operating on input data and generating output. Apparatus of the invention may be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

The above-described embodiments of the present invention may advantageously be used independently of any other of the embodiments or in any feasible combination with one or more others of the embodiments.

**Claims**

1. A computer-implemented method comprising:

   assigning a plurality of family members of a patient each at least one genotype based on rules defining an inheritance mode of a condition and based on information indicating whether or not each of the plurality of family members of the patient is known to have or have had the condition;
   determining a genetic risk score indicating a likelihood of the patient having the condition using Mendelian and/or Bayesian analysis based on the assigned genotypes of family members of the patient;
   determining a family history risk score indicating a likelihood of the patient having the condition based on:

      how many family members of the patient are known to have or have had the condition and who are not known to have died from the condition and at least one said family member's age when they were diagnosed with the condition, and
      how many family members of the patient are known to have died from the condition and at least one said family member's age when they died; and

   determining a genetic family history risk score of the patient having the condition based on the genetic risk score and the family history risk score.

2. The computer-implemented method according to claim 1, further comprising determining the inheritance mode of the condition by applying rules defining a plurality of inheritance modes to the information indicating whether or not each of the plurality of family members of the patient is known to have or have had the condition.

3. The computer-implemented method according to claim 1 or 2, wherein determining the family history risk score comprises:

   determining a first contribution to the family history risk score based on how many family members of the patient are known to have or have had the condition and who are not known to have died from the condition;
   determining a second contribution to the family history risk score based on how many family members of the patient are known to have died from the condition;
   determining a third contribution to the family history risk score based on:

      the age of at least one family member who is known to have or have had the condition and who is not known to have died from the condition when they were diagnosed with the condition, and
      the age of at least one family member who is known to have died from the condition when they died; and

summing the first to third contributions with corresponding first to third weighting scores.

4. The computer-implemented method according to any of claims 1 to 3, wherein:

the first contribution is determined such that it increases as the number of family members of the patient who are known to have or have had the condition and who are not known to have died from the condition increases; and/or

the second contribution is determined such that it increases as the number of family members of the patient who are known to have died from the condition increases; and/or

the third contribution is determined such that it increases as the age or ages of the family member or members who are known to have or have had the condition and who are not known to have died from the condition decreases and as the age or ages of the family member or members who are known to have died from the condition decreases.

5. The computer-implemented method according to any of the preceding claims, wherein determining the first contribution comprises:

determining the number of first degree family members of the patient who are known to have or have had the condition and who are not known to have died from the condition;

determining the number of second degree family members of the patient who are known to have or have had the condition and who are not known to have died from the condition;

determining the number of third degree family members of the patient who are known to have or have had the condition and who are not known to have died from the condition; and

summing the determined numbers of family members with corresponding first degree, second degree, and third degree primary weighting factors,

wherein first degree family members include any of parents, siblings, and children,

wherein second degree family members include any of aunts, uncles, grandparents, grandchildren, nieces, nephews, and half-siblings,

and wherein third degree family members include any of first cousins, great-grandparents, great-uncles, great-aunts, great-nieces, great-nephews, great-grandchildren, half-aunts, and half-uncles.

6. The computer-implemented method according to any of the preceding claims, wherein determining the second contribution comprises:

determining the number of first degree family members of the patient who are known to have died from the condition;

determining the number of second degree family members of the patient who are known to have died from the condition;

determining the number of third degree family members of the patient who are known to have died from the condition; and

summing the determined numbers of family members with corresponding first degree, second degree, and third degree secondary weighting factors,

wherein first degree family members include any of parents, siblings, and children,

wherein second degree family members include any of aunts, uncles, grandparents, grandchildren, nieces, nephews, and half-siblings,

and wherein third degree family members include any of first cousins, great-grandparents, great-uncles, great-aunts, great-nieces, great-nephews, great-grandchildren, half-aunts, and half-uncles.

7. The computer-implemented method according to any of the preceding claims, wherein determining the third contribution comprises computing the formula:

$$f3 = wn * cond1 + wm * cond2,$$

wherein:

$$cond1 = w1 \sum_{i=1}^{n1st} \left(1 - \frac{age_i}{100}\right) + w2 \sum_{j=1}^{n2nd} \left(1 - \frac{age_j}{100}\right) + w3 \sum_{k=1}^{n3rd} \left(1 - \frac{age_k}{100}\right)$$

wherein:

$$cond2 = w1 \sum_{i=1}^{m1st} \left(1 - \frac{age_i}{100}\right) + w2 \sum_{j=1}^{m2nd} \left(1 - \frac{age_j}{100}\right) + w3 \sum_{k=1}^{m3rd} \left(1 - \frac{age_k}{100}\right)$$

and wherein:

n1st, n2nd, and n3rd are the numbers of first to third degree family members of the patient who are known to have or have had the condition and who are not known to have died from the condition, respectively;
mist, m2nd, and m3rd are the numbers of first to third degree family members of the patient who are known to have died from the condition, respectively;
wn and wm are fourth and fifth weighting factors, respectively;
w1, w2, and w3 are sixth to eighth weighting factors, respectively;
in cond1, age indicates the age of the respective family member who is known to have or have had the condition and who is not known to have died from the condition when they were diagnosed with the condition; and
in cond2, age indicates the age of the respective family member who is known to have died from the condition when they died.

8. The computer-implemented method according to any of the preceding claims, wherein the method comprises carrying out, for a plurality of conditions: the assigning of genotypes, the determining of a genetic risk score, the determining of a family history risk score, and the determining of a genetic family history risk score.

9. The computer-implemented method according to any of the preceding claims, further comprising predicting a diagnosis for the patient based on at least one said genetic family history risk score and outputting the diagnosis.

10. The computer-implemented method according to any of the preceding claims, further comprising displaying, using a device, information indicating output information, the output information comprising any of:

at least one determined genetic family history risk score and the associated condition; and
a determined diagnosis and optionally the associated genetic family history risk score.

11. A computer program which, when run on a computer, causes the computer to carry out a method comprising:

assigning a plurality of family members of a patient each at least one genotype based on rules defining an inheritance mode of a condition and based on information indicating whether or not each of the plurality of family members of the patient is known to have or have had the condition;
determining a genetic risk score indicating a likelihood of the patient having the condition using Mendelian and/or Bayesian analysis based on the assigned genotypes of family members of the patient;
determining a family history risk score indicating a likelihood of the patient having the condition based on:

how many family members of the patient are known to have or have had the condition and who are not known to have died from the condition and at least one said family member's age when they were diagnosed with the condition, and
how many family members of the patient are known to have died from the condition and at least one said family member's age when they died; and

determining a genetic family history risk score of the patient having the condition based on the genetic risk score and the family history risk score.

12. An information processing apparatus comprising a memory and a processor connected to the memory, wherein the processor is configured to:

assign a plurality of family members of a patient each at least one genotype based on rules defining an inheritance mode of a condition and based on information indicating whether or not each of the plurality of family members of the patient is known to have or have had the condition;

determine a genetic risk score indicating a likelihood of the patient having the condition using Mendelian and/or Bayesian analysis based on the assigned genotypes of family members of the patient;

determine a family history risk score indicating a likelihood of the patient having the condition based on:

how many family members of the patient are known to have or have had the condition and who are not known to have died from the condition and at least one said family member's age when they were diagnosed with the condition, and

how many family members of the patient are known to have died from the condition and at least one said family member's age when they died; and

determine a genetic family history risk score of the patient having the condition based on the genetic risk score and the family history risk score.

13. A system comprising a device and a server, wherein the server comprises the information processing apparatus according to claim 12, wherein the server is configured to transmit output information to the device and the device is configured to display information indicating the output information, the output information comprising any of:

at least one determined genetic family history risk score and the associated condition; and

a determined diagnosis and optionally the associated genetic family history risk score.

14. The system according to claim 13, wherein the device is a magnetic device and/or an internet of things, IOT, device.

15. The system according to claim 13 or 14, wherein the device comprises at least one light element and is configured to display a colour using a said light element to indicate a range in which the determined genetic family history risk score falls.

**Amended claims in accordance with Rule 137(2) EPC.**

1. A computer-implemented method comprising:

assigning (S50) a plurality of family members of a patient each at least one genotype based on rules defining an inheritance mode of a condition and based on information indicating whether or not each of the plurality of family members of the patient is known to have or have had the condition;

determining (S60) a genetic risk score indicating a likelihood of the patient having the condition using Mendelian and/or Bayesian analysis based on the assigned genotypes of family members of the patient;

determining (S70) a family history risk score indicating a likelihood of the patient having the condition based on:

how many family members of the patient are known to have or have had the condition and who are not known to have died from the condition and at least one said family member's age when they were diagnosed with the condition, and

how many family members of the patient are known to have died from the condition and at least one said family member's age when they died; and

determining (S80) a genetic family history risk score of the patient having the condition based on the genetic risk score and the family history risk score,

wherein determining (S70) the family history risk score comprises:

determining a first contribution to the family history risk score based on how many family members of the patient are known to have or have had the condition and who are not known to have died from the condition;

determining a second contribution to the family history risk score based on how many family members of the patient are known to have died from the condition;

determining a third contribution to the family history risk score based on:

the age of at least one family member who is known to have or have had the condition and who is not known to have died from the condition when they were diagnosed with the condition, and
the age of at least one family member who is known to have died from the condition when they died; and

summing the first to third contributions with corresponding first to third weighting scores,

wherein determining the third contribution comprises computing the formula:

$$f3 = wn * cond1 + wm * cond2,$$

wherein:

$$cond1 = w1 \sum_{i=1}^{n1st} \left(1 - \frac{age_i}{100}\right) + w2 \sum_{j=1}^{n2nd} \left(1 - \frac{age_j}{100}\right) + w3 \sum_{k=1}^{n3rd} \left(1 - \frac{age_k}{100}\right)$$

wherein:

$$cond2 = w1 \sum_{i=1}^{m1st} \left(1 - \frac{age_i}{100}\right) + w2 \sum_{j=1}^{m2nd} \left(1 - \frac{age_j}{100}\right) + w3 \sum_{k=1}^{m3rd} \left(1 - \frac{age_k}{100}\right)$$

and wherein:

n1st, n2nd, and n3rd are the numbers of first to third degree family members of the patient who are known to have or have had the condition and who are not known to have died from the condition, respectively;
mist, m2nd, and m3rd are the numbers of first to third degree family members of the patient who are known to have died from the condition, respectively;
wn and wm are fourth and fifth weighting factors, respectively;
w1, w2, and w3 are sixth to eighth weighting factors, respectively;
in cond1, age indicates the age of the respective family member who is known to have or have had the condition and who is not known to have died from the condition when they were diagnosed with the condition; and
in cond2, age indicates the age of the respective family member who is known to have died from the condition when they died,

wherein the method comprises carrying out, for a plurality of conditions: the assigning of genotypes, the determining of a genetic risk score, the determining of a family history risk score, and the determining of a genetic family history risk score,
wherein the method further comprises predicting (S90) a diagnosis of the patient by selecting the condition with the highest genetic family history risk score when the highest genetic family history risk score is above a genetic family history risk score threshold.

2. The computer-implemented method according to claim 1, further comprising determining the inheritance mode of the condition by applying rules defining a plurality of inheritance modes to the information indicating whether or not each of the plurality of family members of the patient is known to have or have had the condition.

3. The computer-implemented method according to claim 1 or 2, wherein:

the first contribution is determined such that it increases as the number of family members of the patient who are known to have or have had the condition and who are not known to have died from the condition increases; and/or
the second contribution is determined such that it increases as the number of family members of the patient who are known to have died from the condition increases; and/or
the third contribution is determined such that it increases as the age or ages of the family member or members who are known to have or have had the condition and who are not known to have died from the condition decreases and as the age or ages of the family member or members who are known to have died from the condition decreases.

4. The computer-implemented method according to any of the preceding claims, wherein determining the first contribution comprises:

    determining the number of first degree family members of the patient who are known to have or have had the condition and who are not known to have died from the condition;
    determining the number of second degree family members of the patient who are known to have or have had the condition and who are not known to have died from the condition;
    determining the number of third degree family members of the patient who are known to have or have had the condition and who are not known to have died from the condition; and
    summing the determined numbers of family members with corresponding first degree, second degree, and third degree primary weighting factors,
    wherein first degree family members include any of parents, siblings, and children,
    wherein second degree family members include any of aunts, uncles, grandparents, grandchildren, nieces, nephews, and half-siblings,
    and wherein third degree family members include any of first cousins, great-grandparents, great-uncles, great-aunts, great-nieces, great-nephews, great-grandchildren, half-aunts, and half-uncles.

5. The computer-implemented method according to any of the preceding claims, wherein determining the second contribution comprises:

    determining the number of first degree family members of the patient who are known to have died from the condition;
    determining the number of second degree family members of the patient who are known to have died from the condition;
    determining the number of third degree family members of the patient who are known to have died from the condition; and
    summing the determined numbers of family members with corresponding first degree, second degree, and third degree secondary weighting factors,
    wherein first degree family members include any of parents, siblings, and children,
    wherein second degree family members include any of aunts, uncles, grandparents, grandchildren, nieces, nephews, and half-siblings,
    and wherein third degree family members include any of first cousins, great-grandparents, great-uncles, great-aunts, great-nieces, great-nephews, great-grandchildren, half-aunts, and half-uncles.

6. The computer-implemented method according to any of the preceding claims, further comprising displaying, using a device, information indicating output information, the output information comprising any of:

    at least one determined genetic family history risk score and the associated condition; and
    a determined diagnosis and optionally the associated genetic family history risk score.

7. A computer program which, when run on a computer, causes the computer to carry out a method comprising:

    assigning (S50) a plurality of family members of a patient each at least one genotype based on rules defining an inheritance mode of a condition and based on information indicating whether or not each of the plurality of family members of the patient is known to have or have had the condition;
    determining (S60) a genetic risk score indicating a likelihood of the patient having the condition using Mendelian and/or Bayesian analysis based on the assigned genotypes of family members of the patient;
    determining (S70) a family history risk score indicating a likelihood of the patient having the condition based on:

        how many family members of the patient are known to have or have had the condition and who are not known to have died from the condition and at least one said family member's age when they were diagnosed with the condition, and
        how many family members of the patient are known to have died from the condition and at least one said family member's age when they died; and

determining (S80) a genetic family history risk score of the patient having the condition based on the genetic risk score and the family history risk score,

wherein determining (S70) the family history risk score comprises:

determining a first contribution to the family history risk score based on how many family members of the patient are known to have or have had the condition and who are not known to have died from the condition;

determining a second contribution to the family history risk score based on how many family members of the patient are known to have died from the condition;

determining a third contribution to the family history risk score based on:

the age of at least one family member who is known to have or have had the condition and who is not known to have died from the condition when they were diagnosed with the condition, and

the age of at least one family member who is known to have died from the condition when they died; and

summing the first to third contributions with corresponding first to third weighting scores,

wherein determining the third contribution comprises computing the formula:

$$f3 = wn * cond1 + wm * cond2,$$

wherein:

$$cond1 = w1 \sum_{i=1}^{n1st} \left(1 - \frac{age_i}{100}\right) + w2 \sum_{j=1}^{n2nd} \left(1 - \frac{age_j}{100}\right) + w3 \sum_{k=1}^{n3rd} \left(1 - \frac{age_k}{100}\right)$$

wherein:

$$cond2 = w1 \sum_{i=1}^{m1st} \left(1 - \frac{age_i}{100}\right) + w2 \sum_{j=1}^{n2nd} \left(1 - \frac{age_j}{100}\right) + w3 \sum_{k=1}^{m3rd} \left(1 - \frac{age_k}{100}\right)$$

and wherein:

n1st, n2nd, and n3rd are the numbers of first to third degree family members of the patient who are known to have or have had the condition and who are not known to have died from the condition, respectively;

mist, m2nd, and m3rd are the numbers of first to third degree family members of the patient who are known to have died from the condition, respectively;

wn and wm are fourth and fifth weighting factors, respectively;

w1, w2, and w3 are sixth to eighth weighting factors, respectively;

in cond1, age indicates the age of the respective family member who is known to have or have had the condition and who is not known to have died from the condition when they were diagnosed with the condition; and

in cond2, age indicates the age of the respective family member who is known to have died from the condition when they died,

wherein the method comprises carrying out, for a plurality of conditions: the assigning of genotypes, the determining of a genetic risk score, the determining of a family history risk score, and the determining of a genetic family history risk score,

wherein the method further comprises predicting (S90) a diagnosis of the patient by selecting the condition with the highest genetic family history risk score when the highest genetic family history risk score is above a genetic family history risk score threshold.

8.  An information processing apparatus (10) comprising a memory (994) and a processor (993) connected to the memory (994), wherein the processor (993) is configured to:

assign a plurality of family members of a patient each at least one genotype based on rules defining an inheritance mode of a condition and based on information indicating whether or not each of the plurality of family members of the patient is known to have or have had the condition;

determine a genetic risk score indicating a likelihood of the patient having the condition using Mendelian and/or Bayesian analysis based on the assigned genotypes of family members of the patient;

determine a family history risk score indicating a likelihood of the patient having the condition based on:

how many family members of the patient are known to have or have had the condition and who are not known to have died from the condition and at least one said family member's age when they were diagnosed with the condition, and

how many family members of the patient are known to have died from the condition and at least one said family member's age when they died; and

determine a genetic family history risk score of the patient having the condition based on the genetic risk score and the family history risk score,

wherein determining the family history risk score comprises:

determining a first contribution to the family history risk score based on how many family members of the patient are known to have or have had the condition and who are not known to have died from the condition;

determining a second contribution to the family history risk score based on how many family members of the patient are known to have died from the condition;

determining a third contribution to the family history risk score based on:

the age of at least one family member who is known to have or have had the condition and who is not known to have died from the condition when they were diagnosed with the condition, and

the age of at least one family member who is known to have died from the condition when they died; and

summing the first to third contributions with corresponding first to third weighting scores,

wherein determining the third contribution comprises computing the formula:

$$f3 = wn * cond1 + wm * cond2,$$

wherein:

$$cond1 = w1 \sum_{i=1}^{n1st} \left(1 - \frac{age_i}{100}\right) + w2 \sum_{j=1}^{n2nd} \left(1 - \frac{age_j}{100}\right) + w3 \sum_{k=1}^{n3rd} \left(1 - \frac{age_k}{100}\right)$$

wherein:

$$cond2 = w1 \sum_{i=1}^{m1st} \left(1 - \frac{age_i}{100}\right) + w2 \sum_{j=1}^{m2nd} \left(1 - \frac{age_j}{100}\right) + w3 \sum_{k=1}^{m3rd} \left(1 - \frac{age_k}{100}\right)$$

and wherein:

$n1st$, $n2nd$, and $n3rd$ are the numbers of first to third degree family members of the patient who are known to have or have had the condition and who are not known to have died from the condition, respectively;

mist, $m2nd$, and $m3rd$ are the numbers of first to third degree family members of the patient who are known to have died from the condition, respectively;

wn and wm are fourth and fifth weighting factors, respectively;

w1, w2, and w3 are sixth to eighth weighting factors, respectively;

in cond1, age indicates the age of the respective family member who is known to have or have had the condition and who is not known to have died from the condition when they were diagnosed with the condition;

and

in cond2, age indicates the age of the respective family member who is known to have died from the condition when they died,

wherein the processor (993) is configured to carry out, for a plurality of conditions: the assigning of genotypes, the determining of a genetic risk score, the determining of a family history risk score, and the determining of a genetic family history risk score,

wherein the processor (993) is further configured to predict a diagnosis of the patient by selecting the condition with the highest genetic family history risk score when the highest genetic family history risk score is above a genetic family history risk score threshold.

9. A system comprising a device (200) and a server, wherein the server comprises the information processing apparatus (10) according to claim 8, wherein the server is configured to transmit output information to the device (200) and the device (200) is configured to display information indicating the output information, the output information comprising any of:

at least one determined genetic family history risk score and the associated condition; and
a determined diagnosis and optionally the associated genetic family history risk score.

10. The system according to claim 9, wherein the device (200) is a magnetic device and/or an internet of things, IOT, device.

11. The system according to claim 9 or 10, wherein the device (200) comprises at least one light element and is configured to display a colour using a said light element to indicate a range in which the determined genetic family history risk score falls.

FIGURE 1

EP 4 451 274 A1

Iterate (different condition)

| S20 | Extract information |
| S30 | Transform information |
| S40 | Determine inheritance mode |
| S50 | Assign genotypes to family members |
| S60 | Determine genetic risk score |
| S70 | Determine family history risk score |
| S80 | Determine genetic family history risk score |

S90
Determine final diagnosis. Output final diagnosis and score(s)

FIGURE 2

EP 4 451 274 A1

| S21 | Process Tokenization, Noun Detection, POS tagging and Syntax Tree Dependencies |
|---|---|

| S22 | Extract family member roles |
|---|---|

| S23 | Extract family side associated to each family role |
|---|---|

| S24 | Extract status associated to each family role |
|---|---|

| S25 | Extract observations associated to each family role |
|---|---|

| S26 | Extract modality of observations (pos, neg) associated to each family role |
|---|---|

| S27 | Build annotated dataset in JSON from extracted entities and relations |
|---|---|

| S28 | Transform annotated dataset to BIO format |
|---|---|

FIGURE 3

FIGURE 4

| | A | B | C | D | E | F |
|---|---|---|---|---|---|---|
| 1 | ID | Gender | Age | FatherID | MotherID | Affected |
| 2 | 1 | 1 | 25 | 2 | 3 | 0 |
| 3 | 2 | 1 | 12 | 0 | 0 | 0 | → Father |
| 4 | 3 | 0 | | 4 | 5 | 1 | → Mother |
| 5 | 4 | 1 | 32 | 0 | 0 | 1 | → Maternal grandfather |
| 6 | 5 | 0 | | 0 | 0 | 1 | → Maternal grandmother |
| 7 | 6 | 0 | 45 | 2 | 3 | 0 | → Sister |
| 8 | 7 | 1 | | 4 | 5 | 0 | → Maternal uncle |

FIGURE 5

EP 4 451 274 A1

```python
def is_autosomal_dominant(filename):
    """Return comprobation if family history follows Autosomal Dominant trait pattern
    Args:
        filename: Name of the file that includes family history information (.csv format)
            [ID Member (1 for current patient), Gender (0=female; 1=male), FatherID, MotherID, Affected (True, False)]
    Returns: True if family history follows autosomal dominant trait pattern. False otherwise
    """
    ## All affected children must have at least 1 affected parent (Unaffected parents must not have affected children)
    family_history = pd.read_csv(filename)
    for index in range(1, len(family_history)):
        row = family_history.iloc[index,:]
        ID = row['ID']
        affected=row['Affected']
        affectedFather = family_history[family_history['ID'] == row['FatherID']].Affected.values ## Get affected value from father
        affectedMother = family_history[family_history['ID'] == row['MotherID']].Affected.values ## Get affected value from mother
        if affected:
            if len(affectedFather) != 0 and len(affectedMother) != 0:
                if affectedFather[0] == False and affectedMother[0] == False:
                    return False
            elif len(affectedFather) != 0:
                if affectedFather[0] == False:
                    return False
            elif len(affectedMother) != 0:
                if affectedMother[0] == False:
                    return False
    return True
```

FIGURE 6

| | A | B | C | D | E | F | G |
|---|---|---|---|---|---|---|---|
| 1 | ID | Gender | Age | FatherID | MotherID | Affected | Genotypes |
| 2 | 1 | 1 | 25 | 2 | 3 | 0 | |
| 3 | 2 | 1 | 12 | 0 | 0 | 0 | ['aa'] |
| 4 | 3 | 0 | | 4 | 5 | 1 | ['Aa'] |
| 5 | 4 | 1 | 32 | 0 | 0 | 1 | ['Aa'] |
| 6 | 5 | 0 | | 0 | 0 | 1 | ['Aa'] |
| 7 | 6 | 0 | 45 | 2 | 3 | 0 | ['aa'] |
| 8 | 7 | 1 | | 4 | 5 | 0 | ['aa'] |
| 9 | 8 | 0 | 2 | 0 | 0 | 0 | ['aa'] |
| 10 | 9 | 1 | | 7 | 8 | 0 | ['aa'] |
| 11 | 10 | 0 | 13 | 7 | 8 | 0 | ['aa'] |

FIGURE 7

EP 4 451 274 A1

FIGURE 8

Her MOTHER suffered BREAST CANCER at age 31.
Her FATHER DIED of BREAST CANCER at 81 age.
MATERNAL GRANDMOTHER DIED of BREAST CANCER around 60 age.
MATERNAL GRANDFATHER DIED at 75 of HEART PROBLEMS.
Her MATERNAL AUNT had BREAST CANCER at age 19.
A female COUSIN was diagnosed of BREAST CANCER at 21 age.
Now she is HEALTHY.
SISTER DIED of BREAST CANCER at 27.
BROTHER DIED from HIV/AIDS.
The HUSBAND is ALIVE and HEALTHY.
A CHILD is HEALTHY.
Other SON has AUTISM.
A DAUGHTER is HEALTHY at 21 age.

FIGURE 9

| | A | B | C | D | E | F |
|---|---|---|---|---|---|---|
| 1 | ID | Gender | Age | FatherID | MotherID | Affected |
| 2 | 1 | 0 | 26 | 8 | 7 | 0 |
| 3 | 2 | 0 | 60 | 0 | 0 | 1 |
| 4 | 3 | 1 | 75 | 0 | 0 | 0 |
| 5 | 4 | 0 | 19 | 3 | 2 | 1 |
| 6 | 5 | 1 | | 0 | 0 | 0 |
| 7 | 6 | 1 | 21 | 5 | 4 | 1 |
| 8 | 7 | 0 | 31 | 3 | 2 | 1 |
| 9 | 8 | 1 | 81 | 0 | 0 | 1 |
| 10 | 9 | 0 | 27 | 8 | 7 | 1 |
| 11 | 10 | 1 | | 8 | 7 | 0 |
| 12 | 11 | 0 | | 0 | 0 | 0 |
| 13 | 12 | 1 | | 0 | 0 | 0 |
| 14 | 13 | 0 | | 12 | 1 | 0 |
| 15 | 14 | 0 | 21 | 12 | 1 | 0 |
| 16 | 15 | 1 | | 12 | 1 | 0 |

Maternal grandmother
Maternal grandfather
Maternal aunt
Maternal in-law uncle (default)
Maternal cousin
Mother
Father
Sister
Brother
default
Husband
Child
Daughter
Son

FIGURE 10

EP 4 451 274 A1

EP 4 451 274 A1

|   | A | B | C | D | E | F | G |
|---|---|---|---|---|---|---|---|
| 1 | ID | Gender | Age | FatherID | MotherID | Affected | Genotypes |
| 2 | 1 | 0 | 26 | 8 | 7 | 0 | |
| 3 | 2 | 0 | 60 | 0 | 0 | 1 | ['AA', 'Aa'] |
| 4 | 3 | 1 | 75 | 0 | 0 | 0 | ['aa'] |
| 5 | 4 | 0 | 19 | 3 | 2 | 1 | ['Aa'] |
| 6 | 5 | 1 | | 0 | 0 | 0 | ['aa'] |
| 7 | 6 | 1 | 21 | 5 | 4 | 1 | ['Aa'] |
| 8 | 7 | 0 | 31 | 3 | 2 | 1 | ['Aa'] |
| 9 | 8 | 1 | 81 | 0 | 0 | 1 | ['Aa'] |
| 10 | 9 | 0 | 27 | 8 | 7 | 1 | ['AA', 'Aa'] |
| 11 | 10 | 1 | | 8 | 7 | 0 | ['aa'] |
| 12 | 11 | 0 | | 0 | 0 | 0 | ['aa'] |
| 13 | 12 | 1 | | 0 | 0 | 0 | ['aa'] |
| 14 | 13 | 0 | | 12 | 1 | 0 | ['aa'] |
| 15 | 14 | 0 | 21 | 12 | 1 | 0 | ['aa'] |
| 16 | 15 | 1 | | 12 | 1 | 0 | ['aa'] |

FIGURE 11

**FIGURE 12**

| Genotype | Ratio | Prob |
|----------|-------|------|
| AA | 1/4 | 0.25 |
| Aa | 1/4 | 0.25 |
| aA | 1/4 | 0.25 |
| aa | 1/4 | 0.25 |

FIGURE 13

| Genotype | Ratio | Prob |
|---|---|---|
| AA | 4/9 | 0.444444444444444 |
| Aa | 1/9 | 0.111111111111111 |
| aa | 4/9 | 0.444444444444444 |

FIGURE 14

FIGURE 15

EP 4 451 274 A1

Her MOTHER suffered BREAST CANCER at age 31 .

Her FATHER DIED of BREAST CANCER at 81 age.

MATERNAL GRANDMOTHER DIED of BREAST CANCER around 60 age.

MATERNAL GRANDFATHER DIED at 75 of HEART PROBLEMS .

Her MATERNAL AUNT had BREAST CANCER at age 19 .

A female COUSIN was diagnosed of BREAST CANCER at 21 age.

Now she is HEALTHY .

SISTER DIED of BREAST CANCER at 27 .

BROTHER DIED from HIV/AIDS .

The HUSBAND is ALIVE and HEALTHY .

A CHILD is HEALTHY .

Other SON has AUTISM .

A DAUGHTER is HEALTHY at 21 age.

A female SIBLING has been diagnosed with BREAST CANCER at 12 years old.

Other SISTER has been diagnosed of BREAST CANCER at 6 years old.

A third SIBLING has DIED of BREAST CANCER at 15 years old.

New information

FIGURE 16

| | A | B | C | D | E | F |
|---|---|---|---|---|---|---|
| 1 | ID | Gender | Age | FatherID | MotherID | Affected |
| 2 | 1 | 0 | 26 | 8 | 7 | 0 |
| 3 | 2 | 0 | 60 | 0 | 0 | 1 |
| 4 | 3 | 1 | 75 | 0 | 0 | 0 |
| 5 | 4 | 0 | 19 | 3 | 2 | 1 |
| 6 | 5 | 1 | | 0 | 0 | 0 |
| 7 | 6 | 1 | 21 | 5 | 4 | 1 |
| 8 | 7 | 0 | 31 | 3 | 2 | 1 |
| 9 | 8 | 1 | 81 | 0 | 0 | 1 |
| 10 | 9 | 0 | 27 | 8 | 7 | 1 |
| 11 | 10 | 0 | 12 | 8 | 7 | 1 |
| 12 | 11 | 0 | 6 | 8 | 7 | 1 |
| 13 | 12 | 0 | 15 | 8 | 7 | 1 |
| 14 | 13 | 1 | | 8 | 7 | 0 |
| 15 | 14 | 0 | | 0 | 0 | 0 |
| 16 | 15 | 1 | | 0 | 0 | 0 |
| 17 | 16 | 0 | | 15 | 1 | 0 |
| 18 | 17 | 0 | 21 | 15 | 1 | 0 |
| 19 | 18 | 1 | | 15 | 1 | 0 |

Included new information about sisters.

FIGURE 17

| | A | B | C | D | E | F | G |
|---|---|---|---|---|---|---|---|
| 1 | ID | Gender | Age | FatherID | MotherID | Affected | Genotypes |
| 2 | 1 | 0 | 26 | 8 | 7 | 0 | |
| 3 | 2 | 0 | 60 | 0 | 0 | 1 | ['AA', 'Aa'] |
| 4 | 3 | 1 | 75 | 0 | 0 | 0 | ['aa'] |
| 5 | 4 | 0 | 19 | 3 | 2 | 1 | ['Aa'] |
| 6 | 5 | 1 | | 0 | 0 | 0 | ['aa'] |
| 7 | 6 | 1 | 21 | 5 | 4 | 1 | ['Aa'] |
| 8 | 7 | 0 | 31 | 3 | 2 | 1 | ['Aa'] |
| 9 | 8 | 1 | 81 | 0 | 0 | 1 | ['Aa'] |
| 10 | 9 | 0 | 27 | 8 | 7 | 1 | ['AA', 'Aa'] |
| 11 | 10 | 0 | 12 | 8 | 7 | 1 | ['AA', 'Aa'] |
| 12 | 11 | 0 | 6 | 8 | 7 | 1 | ['AA', 'Aa'] |
| 13 | 12 | 0 | 15 | 8 | 7 | 1 | ['AA', 'Aa'] |
| 14 | 13 | 1 | | 8 | 7 | 0 | ['aa'] |
| 15 | 14 | 0 | | 0 | 0 | 0 | ['aa'] |
| 16 | 15 | 1 | | 0 | 0 | 0 | ['aa'] |
| 17 | 16 | 0 | | 15 | 1 | 0 | ['aa'] |
| 18 | 17 | 0 | 21 | 15 | 1 | 0 | ['aa'] |
| 19 | 18 | 1 | | 15 | 1 | 0 | ['aa'] |

FIGURE 18

FIGURE 19

10

PROCESSOR

993

MEMORY

994

992

995

DISPLAY

996

INPUT

997

NETWORK I/F

FIGURE 20

EP 4 451 274 A1

**EUROPEAN SEARCH REPORT**

Europäisches Patentamt
European Patent Office
Office européen des brevets

Application Number

EP 23 38 2349

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JANE W LIANG ET AL: "Statistical methods for Mendelian models with multiple genes and cancers", ARXIV.ORG, CORNELL UNIVERSITY LIBRARY, 201 OLIN LIBRARY CORNELL UNIVERSITY ITHACA, NY 14853, 6 April 2022 (2022-04-06), XP091194209, * the whole document * | 1-15 | INV. G16B20/00 G16B10/00 |
| X | Guan Zoe: "Statistical and Machine Learning Methods for Clinical Risk Prediction Citation", , 1 January 2020 (2020-01-01), XP093085010, Retrieved from the Internet: URL:https://dash.harvard.edu/bitstream/handle/1/37365776/GUAN-DISSERTATION-2020.pdf?sequence=1 [retrieved on 2023-09-22] * the whole document * | 1-15 | |
| A | YANG XI ET AL: "Extracting Family History of Patients From Clinical Narratives: Exploring an End-to-End Solution With Deep Learning Models", JMIR MEDICAL INFORMATICS, vol. 8, no. 12, 1 January 2020 (2020-01-01), page e22982, XP093084385, DOI: 10.2196/22982 Retrieved from the Internet: URL:https://medinform.jmir.org/2020/12/e22982/PDF> * the whole document * | 1-15 | TECHNICAL FIELDS SEARCHED (IPC) G16B |

-/--

The present search report has been drawn up for all claims

| Place of search | Date of completion of the search | Examiner |
|---|---|---|
| Munich | 26 September 2023 | Díaz de Lezana, C |

EPO FORM 1503 03.82 (P04C01)

**EUROPEAN SEARCH REPORT**

Europäisches Patentamt
European Patent Office
Office européen des brevets

Application Number

**EP 23 38 2349**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GARCÍA-SANTA NURIA ET AL: "AGG: An Automated Genogram Generator by Discovering Information in Clinical Texts", 18 March 2023 (2023-03-18), ADVANCES IN VISUAL COMPUTING : 16TH INTERNATIONAL SYMPOSIUM, ISVC 2021, VIRTUAL EVENT, OCTOBER 4-6, 2021, PROCEEDINGS, PART II; [LECTURE NOTES IN COMPUTER SCIENCE], SPRINGER INTERNATIONAL PUBLISHING, CHAM, PAGE(S) 599 - 602, XP047656916, ISSN: 0302-9743 ISBN: 978-3-030-90436-4 [retrieved on 2023-03-18] * the whole document * | 1-15 | |
| A | ERYNN S GORDON ET AL: "The future is now: Technology's impact on the practice of genetic counseling", AMERICAN JOURNAL OF MEDICAL GENETICS PART C: SEMINARS IN MEDICAL GENETICS, WILEY-LISS [ETC.], HOBOKEN, USA, vol. 178, no. 1, 7 March 2018 (2018-03-07) , pages 15-23, XP072330717, ISSN: 1552-4868, DOI: 10.1002/AJMG.C.31599 * the whole document * | 1-15 | **TECHNICAL FIELDS SEARCHED (IPC)** |

The present search report has been drawn up for all claims

| Place of search | Date of completion of the search | Examiner |
|---|---|---|
| Munich | 26 September 2023 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FREZZO, T. M. ; RUBINSTEIN, W. S. ; DUNHAM, D. ; ORMOND, K. E.** The genetic family history as a risk assessment tool in internal medicine. *Genetics in Medicine,* 2003, vol. 5 (2), 84-91
- **YANG, X. ; ZHANG, H. ; HE, X. ; BIAN, J. ; WU, Y.** Extracting family history of patients from clinical narratives: exploring an end-to-end solution with deep learning models. *JMIR Medical Informatics,* 2020, vol. 8 (12), e22982
- **CHIAL, H.** Mendelian genetics: patterns of inheritance and single-gene disorders. *Nature Education,* 2008, vol. 1 (1), 63
- **DAVIS, L. C.** Origin of the Punnett square. *The American Biology Teacher,* 1993, vol. 55 (4), 209-212
- **OGINO, S. ; WILSON, R. B.** Bayesian analysis and risk assessment in genetic counseling and testing. *The Journal of Molecular Diagnostics,* 2004, vol. 6 (1), 1-9, https://www.ncbi.nlm.nih.gov/pmc/articles/PMC1867463